(19) Europäisches Patentamt
European Patent Office
Office européen des brevets

(11) EP 0 967 284 A1

(12) **EUROPEAN PATENT APPLICATION**

(43) Date of publication:
**29.12.1999 Bulletin 1999/52**

(21) Application number: **99303985.8**

(22) Date of filing: **21.05.1999**

(51) Int Cl.[6]: **C12N 15/55**, C12N 9/16, G01N 33/68, C12Q 1/44, C07K 16/40, C12N 15/11, A61K 38/46, A61K 35/00, A61K 48/00

(84) Designated Contracting States:
**AT BE CH CY DE DK ES FI FR GB GR IE IT LI LU MC NL PT SE**
Designated Extension States:
**AL LT LV MK RO SI**

(30) Priority: **28.05.1998 GB 9811500**
**30.10.1998 GB 9823882**
**04.12.1998 GB 9826777**
**09.04.1999 GB 9908247**
**10.05.1999 GB 9910801**

(83) **Declaration under Rule 28(4) EPC (expert solution)**

(71) Applicants:
- **Pfizer Limited**
  **Sandwich Kent CT13 9NJ (GB)**
  Designated Contracting States:
  **GB**
- **PFIZER INC.**
  **Groton, CT 06340 (US)**
  Designated Contracting States:
  **BE CH DE DK ES FI FR GR IE IT LI LU MC PT SE AT CY**

(72) Inventors:
- **Lanfear, Jeremy, c/o Pfizer Central Research**
  **Sandwich, Kent CT13 9NJ (GB)**
- **Robas, Nicola Melanie,**
  **c/o Pfizer Central Research**
  **Sandwich, Kent CT13 9NJ (GB)**

(74) Representative: **Harding, Charles Thomas et al**
**D. Young & Co.**
**21 New Fetter Lane**
**London EC4A 1DA (GB)**

Remarks:
The applicant has subsequently filed a sequence listing and declared, that it includes no new matter.

(54) **Phosphodiesterases**

(57) Amino acid sequences and nucleotide sequences are described. The amino acid sequences comprise the sequence presented as SEQ ID No. 2 or SEQ ID No. 4 or SEQ ID No. 15 or SEQ ID No. 17 or SEQ ID No. 19 or a variant, homologue, fragment or derivative thereof.

EP 0 967 284 A1

## Description

BACKGROUND OF THE PRESENT INVENTION

The present invention relates to an enzyme. The present invention also relates to a nucleotide sequence encoding same.

In particular, the present invention relates to novel nucleic acid sequences encoding novel phosphodiesterase enzymes.

The present invention also relates to the use of the novel nucleic acid and amino acid sequences in the diagnosis and treatment of disease.

The present invention also relates to the use of the novel nucleic acid and amino acid sequences to evaluate and/or to screen for agents that can modulate phosphodiesterase activity.

The present invention further relates to genetically engineered host cells that comprise or express the novel nucleic acid and amino acid sequences to evaluate and/or to screen for agents that can modulate phosphodiesterase activity.

BACKGROUND ART

Cyclic nucleotide phosphodiesterases (PDEs) are a family of enzymes that catalyse the degradation of cyclic nucleotides. Cyclic nucleotides, particularly cAMP (i.e. cyclic adenosine 3',5'-monophosphate), are important intracellular second messengers. PDEs are one cellular component that regulate the concentration of cyclic nucleotides.

In recent years, at least seven PDE enzymes (such as PDEI - PDEVII), as well as many subtypes of these enzymes, have been defined based on substrate affinity and cofactor requirements (Beavo JA and Reifsnyder DH, Trends Pharmacol. Sci. 11:150 [1990]; Beavo J, In: Cyclic Nucleotide Phosphodiesterases: Structure, Regulation and Drug Action., Beavo J and Housley MD (Eds.). Wiley : Chichester, pp. 3-15 [1990]). Examples of PDEs (i.e. cyclic nucleotide phosphodiesterases) include: PDEI which is a $Ca^{2+}$/Calmodulin-dependent PDE; PDEII which is a cGMP stimulated PDE; PDEIII which is a cGMP inhibited PDE; PDEIV which is a high affinity cAMP-specific PDE; and PDEV which is a cGMP specific PDE.

Each PDE family may contain two or more isoforms (i.e. there may be two or more PDE isoenzymes). By way of example, mammalian PDE IV, the homologue of the *Drosophila* Dunce gene (Chen CN *et al.,* Proc. Nat. Acad. Sci. (USA) 83:9313 [1986]), is known to have four isoforms in the rat (Swinnen JV *et al.,* Proc. Nat. Acad. Sci. (USA) 86: 5325 [1989]). Human PDEs are also known to occur as isoforms and have splice variants. For example, the cloning of one human isoform of PDEIV from monocytes was reported in 1990 (Livi GP *et al.,* Mol. Cell. Bio., 10:2678 [1990]). By way of further example, other workers have independently cloned three splice variants of PDEIV, which are now designated hPDEIV-B1, hPDEIV-B2, and hPDEIV-B3.

Teachings on a further cyclic nucleotide phosphodiesterase - namely CN PCDE8 - can be found in WO-A-97/35989. According to WO-A-97/35989, CN PCDE8 has two isozymes - which were designated CN PCDE8A and CN PCDE8B. The term "isozyme" is sometimes referred to in the art as "isoform".

According to WO-A-97/35989, many inhibitors of different PDEs have been identified and some have undergone clinical evaluation. For example, PDEIII inhibitors are being developed as antithrombotic agents, as antihypertensive agents and as cardiotonic agents useful in the treatment of congestive heart failure. Rolipram, a PDEIII inhibitor, has been used in the treatment of depression and other inhibitors of PDEIII are undergoing evaluation as anti-inflammatory agents. Rolipram has also been shown to inhibit lipopolysaccharide (LPS) induced TNF-alpha which has been shown to enhance HIV-1 replication *in vitro.* Therefore, rolipram may inhibit HIV-1 replication (Angel *et al* 1995 AIDS 9:1137-44). Additionally, based on its ability to suppress the production of TNF alpha and beta and interferon gamma, rolipram has been shown to be effective in the treatment of encephalomyelitis, the experimental animal model for multiple sclerosis (Sommer *et al,* 1995 Nat Med 1:244-248) and may be effective in the treatment of tardive dyskinesia (Sasaki *et al,* 1995 Eur J Phamacol 282:71-76).

According to WO-A-97/35989, there are also non-specific PDE inhibitors such as theophylline, used in the treatment of bronchial asthma and other respiratory diseases, and pentoxifylline, used in the treatment of intermittent claudication and diabetes-induced peripheral vascular disease. Theophylline is thought to act on airway smooth muscle function as well as in an anti-inflammatory or immunomodulatory capacity in the treatment of respiratory diseases (Banner *et al* 1995 Respir J 8:996-1000) where it is thought to act by inhibiting both CN PDE cAMP and cGMP hydrolysis (Banner *et al* 1995 Monaldi Arch Chest Dis 50:286-292). Pentoxifylline, also known to block TNF-alpha production, may inhibit HIV-1 replication (Angel *et al supra*). A list of CN PDE inhibitors is given in Beavo 1995 *supra.*

It has been suggested that selective inhibitors of PDEs, in addition to their isozymes and their subtypes, will lead to more effective therapy with fewer side effects. For example, see the teachings in the reviews of Wieshaar RE *et al,* (J. Med. Chem., 28:537 [1985]), Giembycz MA (Biochem. Pharm., 43:2041 [1992]) and Lowe JA and Cheng JB (Drugs of the Future, 17:799-807 [1992]).

Thus, for some applications it is desirable to have a selective inhibition of an individual type of PDE. Hence, the cloning and expression of a novel PDE would greatly aid the discovery of selective inhibitors.

SUMMARY ASPECTS OF THE PRESENT INVENTION

Aspects of the present invention are presented in the claims and in the following commentary.

In brief, some aspects of the present invention relate to:

1. Novel amino acids.
2. Novel nucleotide sequences.
3. Assays using said novel sequences.
4. Compounds/compositions identified by use of said assays.
5. Expression systems comprising or expressing said novel sequences.
6. Methods of treatment based on said novel sequences.
7. Pharmaceutical compositions based on said novel sequences.

Other aspects concerning the amino acid sequence of the present invention and/or the nucleotide sequence of the present invention include: a construct comprising or capable of expressing the sequences of the present invention; a vector comprising or capable of expressing the sequences of the present invention; a plasmid comprising or capable of expressing the sequences of present invention; a tissue comprising or capable of expressing the sequences of the present invention; an organ comprising or capable of expressing the sequences of the present invention; a transformed host comprising or capable of expressing the sequences of the present invention; a transformed organism comprising or capable of expressing the sequences of the present invention. The present invention also encompasses methods of expressing the same, such as expression in a micro-organism; including methods for transferring same.

For ease of reference, aspects of the present invention are now discussed under appropriate section headings. However, the teachings under each section are not necessarily limited to each particular section.

In the following commentary references to "nucleotide sequence of the present invention" and "amino acid sequence of the present invention" refer respectively to any one or more of the nucleotide sequences presented herein and to any one or more of the amino acid sequences present herein. Also, and as used herein, "amino acid sequence" refers to peptide or protein sequences and may refer to portions thereof. In addition, the term "amino acid sequence of the present invention" is synonymous with the phrase "polypeptide sequence of the present invention". Also, the term "nucleotide sequence of the present invention" is synonymous with the phrase "polynucleotide sequence of the present invention".

DETAILED ASPECTS OF THE PRESENT INVENTION

According to a first aspect of the present invention there is provided an amino acid sequence comprising the sequence presented as Formula I or a variant, homologue, fragment or derivative thereof; wherein the amino acid sequence is capable of displaying PDE activity.

**Formula I**

An amino acid sequence comprising any one or more of peptide sequences or amino acids Z1 - Z28, any of which peptide sequence or amino acid Z1 - Z28 may be separated from another of said peptide sequence or amino acid Z1 - Z28 by a suitable peptide sequence or amino acid residue;

wherein Z1 - Z28 are:

Z1 = LTDEKVKAYLSLHPQVLDEFVSESVSAETVEKWLKRK
Z2 = NK
Z3 = DE
Z4 = PKEVSRYQDTNMQGVVYELNSYIEQRLDTGGDN
Z5 = LLLYELSSII
Z6 = IATKADGFALYFLGECNNSLC
Z7 = F
Z8 = PPG
Z9 = KEG
Z10 = PRLIPAGPITQGTT
Z11 =
SAYVAKSRKTLLVEDILGDERFPRGTGLESGTRIQSVLCLPIVTAIGDLIGILELYRHWGK
EAFCLSHQEVATANLAWASVAIHQVQVCRGLAKQTELNDFLLDVSKTYFDNIVAIDSLLEH
IMIYAKNLVNADRCALFQVDHKNKELYSDLFDIGEEKEGKP
Z12 =
FKKTKEIRFSIEKGIAGQVARTGEVLNIPDAYADPRFNREVDLYTGYTTRNILCMPIVSRG
SVIGVVQMVNKISGSAFSKTDENNFKMFAVFCALALHCANMYHRIRHSECIYRVTMEKLSY
HSICTSEEWQGLM
Z13 = F
Z14= LP
Z15= R
Z16= C
Z17= IELFHFDIGPFENMWPGIFVYM
Z18= HRSCGTSCFELEKLCRFIMSVKKNYRRVPYHNWKHAVTVAHCMYAILQNN
Z19= LFTDLERKGLLIACLCHDLDHRGFSNSYLQKFDHPL
Z20 =
ALYSTSTMEQHHFSQTVSILQLEGHNIFSTLSSSEYEQVLEIIRKAIIATDLALYFGNRQL
EEMYQTGSLNL
Z21 = NQSHRDRVIGLMMTACDLCSVTK
Z22 = WPVTKLTANDIYAEFWAEGDEMKKLGIQPIPMMDRDK
Z23 = DEVPQGQLGFYNAVAIPCYTTLTQILPPTEPLLKACRDNL
Z24 = QWEKVIRGEETA
Z25 = WIS
Z26 = P
Z27 = A
Z28 = E

and; wherein the amino acid sequence is made up of more than 159 amino acid residues.

For convenience, we now present a Table indicating the codes used for the amino acids.

| AMINO ACID | THREE LETTER ABBREVIATION | ONE LETTER SYMBOL |
|---|---|---|
| Alanine | Ala | A |
| Arginine | Arg | R |
| Asparagine | Asn | N |
| Aspartic acid | Asp | D |
| Cysteine | Cys | C |
| Glutamine | Gln | Q |
| Glutamic acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| Any residue | Xaa | X |

Preferably, the amino acid sequence of Formula I comprises at least one or more of Z11, Z12, Z18 and Z20, or analogues thereof.

The term "analogue" as used herein means a sequence having a sequence similar to that of Formula I but wherein non-detrimental (i.e. not detrimental to enzymatic activity) amino acid substitutions or deletions have been made.

Preferably, the amino acid sequence of Formula I comprises at least each of Z11, Z12. Z18 and Z20, or analogues thereof.

Preferably, the amino acid sequence of Formula I comprises at least each of Z11, Z12, Z18 and Z20.

Preferably, the amino acid sequence of Formula I comprises at least one or more of Z1, Z4, Z17, Z19, Z21, Z22 and Z33, or analogues thereof.

Preferably, the amino acid sequence of Formula I comprises at least each of of Z1, Z4, Z17, Z19, Z21, Z22 and Z33, or analogues thereof.

Preferably, the amino acid sequence of Formula I comprises at least each of of Z1, Z4, Z17, Z19, Z21, Z22 and Z33.

A preferred example of an amino acid sequence comprising the sequence presented as Formula I is the amino acid sequence shown as Formula II or a variant, homologue, fragment or derivative thereof.

## Formula II

X0-Z1-X1-Z2-X2-X3-Z3-X4-X5-Z4-X6-Z5-X7-Z6-X8-Z7-X9-Z8-X10-Z9-

X11-Z10-X12-Z11-X13-Z12-X14-Z13-X15-Z14-X16-Z15-X17-Z16-X18-

X19-Z17-X20-Z18-X21-X22-Z19-X23-Z20-X24-Z21-X25-Z22-X26-Z23-

X27-Z24-X28-Z25-X29-Z26-X30-X31-Z27-X32-X33-X34-X35-X36-X37-

Z28-X38

wherein

each of Z1 - Z28 is as defined above;
each of X0 - X38 is independently selected from a suitable peptide sequence or amino acid; and

wherein any one or more of: X0, X28, Z25, X29, Z26, X30, X31, Z27, X32, X33, X34, X35, X36, X37, Z28, and X38 is optional.

A more preferred example of an amino acid sequence comprising the sequence presented as Formula I is the amino acid sequence shown as Formula III or a variant, homologue, fragment or derivative thereof.

## Formula III

LTDEKVKAYLSLHPQVLDEFVSESVSAETVEKWLKRK$X_1$NK$X_2$$X_3$DE$X_4$$X_5$PKEVSRYQDTN
MQGVVYELNSYIEQRLDTGGDN$X_6$LLLYELSSII$X_7$IATKADGFALYFLGECNNSLC$X_8$F$X_9$
PPG$X_{10}$KEG$X_{11}$PRLIPAGPITQGTT$X_{12}$SAYVAKSRKTLLVEDILGDERFPRGTGLESGTRI
QSVLCLPIVTAIGDLIGILELYRHWGKEAFCLSHQEVATANLAWASVAIHQVQVCRGLAKQ
TELNDFLLDVSKTYFDNIVAIDSLLEHIMIYAKNLVNADRCALFQVDHKNKELYSDLFDIG
EEKEGKP$X_{13}$FKKTKEIRFSIEKGIAGQVARTGEVLNIPDAYADPRFNREVDLYTGYTTRN
ILCMPIVSRGSVIGVVQMVNKISGSAFSKTDENNFKMFAVFCALALHCANMYHRIRHSECI
YRVTMEKLSYHSICTSEEWQGLM$X_{14}$F$X_{15}$LP$X_{16}$R$X_{17}$C$X_{18}$$X_{19}$IELFHFDIGPFENMWPGIF
VYM$X_{20}$HRSCGTSCFELEKLCRFIMSVKKNYRRVPYHNWKHAVTVAHCMYAILQNN$X_{21}$$X_{22}$L
FTDLERKGLLIACLCHDLDHRGFSNSYLQKFDHPL$X_{23}$ALYSTSTMEQHHFSQTVSILQLE
GHNIFSTLSSSEYEQVLEIIRKAIIATDLALYFGNRKQLEEMYQTGSLNL$X_{24}$NQSHRDRV
IGLMMTACDLCSVTK$X_{25}$WPVTKLTANDIYAEFWAEGDEMKKLGIQPIPMMDRDK$X_{26}$DEVP
QGQLGFYNAVAIPCYTTLTQILPPTEPLLKACRDNL$X_{27}$QWEKVIRGEETA

wherein

$X_1$ = N or T
$X_2$ = S or A
$X_3$ = E or K
$X_4$ = S or P
$X_5$ = A or S
$X_6$ = Q or H

$X_7$ = K or R
$X_8$ = I or V
$X_9$ = T or I
$X_{10}$ = I or M
$X_{11}$ = K or Q
$X_{12}$ = V or I
$X_{13}$ = V or I
$X_{14}$ = Q or R
$X_{15}$ = T or N
$X_{16}$ = V or A
$X_{17}$ = L or I
$X_{18}$ = K or R
$X_{19}$ = E or D
$X_{20}$ = V or I
$X_{21}$ = H or N
$X_{22}$ = T or G
$X_{23}$ = T or A
$X_{24}$ = N or H
$X_{25}$ = P or L
$X_{26}$ = K or R
$X_{27}$ = S or N

A more preferred example of an amino acid sequence comprising the sequence presented as Formula I is the amino acid sequence shown as Formula IV or a variant, homologue, fragment or derivative thereof.

## Formula IV

$X_0$LTDEKVKAYLSLHPQVLDEFVSESVSAETVEKWLKRK$X_1$NK$X_2$$X_3$DE$X_4$$X_5$PKEVSRYQDT
NMQGVVYELNSYIEQRLDTGGDN$X_6$LLLYELSSII$X_7$IATKADGFALYFLGECNNSLC$X_8$F$X$
$_9$PPG$X_{10}$KEG$X_{11}$PRLIPAGPITQGTT$X_{12}$SAYVAKSRKTLLVEDILGDERFPRGTGLESGTR
IQSVLCLPIVTAIGDLIGILELYRHWGKEAFCLSHQEVATANLAWASVAIHQVQVCRGLAK
QTELNDFLLDVSKTYFDNIVAIDSLLEHIMIYAKNLVNADRCALFQVDHKNKELYSDLFDI
GEEKEGKP$X_{13}$FKKTKEIRFSIEKGIAGQVARTGEVLNIPDAYADPRFNREVDLYTGYTTR
NILCMPIVSRGSVIGVVQMVNKISGSAFSKTDENNFKMFAVFCALALHCANMYHRIRHSEC
IYRVTMEKLSYHSICTSEEWQGLM$X_{14}$F$X_{15}$LP$X_{16}$R$X_{17}$C$X_{18}$$X_{19}$IELFHFDIGPFENMWPGI
FVYM$X_{20}$HRSCGTSCFELEKLCRFIMSVKKNYRRVPYHNWKHAVTVAHCMYAILQNN$X_{21}$$X_{22}$
LFTDLERKGLLIACLCHDLDHRGFSNSYLQKFDHPL$X_{23}$ALYSTSTMEQHHFSQTVSILQL
EGHNIFSTLSSSEYEQVLEIIRKAIIATDLALYFGNRKQLEEMYQTGSLNL$X_{24}$NQSHRDR
VIGLMMTACDLCSVTK$X_{25}$WPVTKLTANDIYAEFWAEGDEMKKLGIQPIPMMDRDK$X_{26}$DEV
PQGQLGFYNAVAIPCYTTLTQILPPTEPLLKACRDNL$X_{27}$QWEKVIRGEETA

wherein

$X_0$ = S or G
$X_1$ = N or T
$X_2$ = S or A
$X_3$ = E or K
$X_4$ = S or P
$X_5$ = A or S
$X_6$ = Q or H
$X_7$ = K or R
$X_8$ = I or V
$X_9$ = T or I

$X_{10}$ = I or M
$X_{11}$ = K or Q
$X_{12}$ = V or I
$X_{13}$ = V or I
$X_{14}$ = Q or R
$X_{15}$ = T or N
$X_{16}$ = V or A
$X_{17}$ = L or I
$X_{18}$ = K or R
$X_{19}$ = E or D
$X_{20}$ = V or I
$X_{21}$ = H or N
$X_{22}$ = T or G
$X_{23}$ = T or A
$X_{24}$ = N or H
$X_{25}$ = P or L
$X_{26}$ = K or R
$X_{27}$ = S or N

A preferred example of an amino acid sequence comprising the sequence presented as Formula I is the amino acid sequence shown as Formula V or a variant, homologue, fragment or derivative thereof.

## Formula V

$X_0$LTDEKVKAYLSLHPQVLDEFVSESVSAETVEKWLKRK$X_1$NK$X_2X_3$DE$X_4X_5$PKEVSRYQDT
NMQGVVYELNSYIEQRLDTGGDN$X_6$LLLYELSSII$X_7$IATKADGFALYFLGECNNSLC$X_8$F$X$
$_9$PPG$X_{10}$KEG$X_{11}$PRLIPAGPITQGTT$X_{12}$SAYVAKSRKTLLVEDILGDERFPRGTGLESGTR
IQSVLCLPIVTAIGDLIGILELYRHWGKEAFCLSHQEVATANLAWASVAIHQVQVCRGLAK
QTELNDFLLDVSKTYFDNIVAIDSLLEHIMIYAKNLVNADRCALFQVDHKNKELYSDLFDI
GEEKEGKP$X_{13}$FKKTKEIRFSIEKGIAGQVARTGEVLNIPDAYADPRFNREVDLYTGYTTR
NILCMPIVSRGSVIGVVQMVNKISGSAFSKTDENNFKMFAVFCALALHCANMYHRIRHSEC
IYRVTMEKLSYHSICTSEEWQGLM$X_{14}$F$X_{15}$LP$X_{16}$R$X_{17}$C$X_{18}X_{19}$IELFHFDIGPFENMWPGI
FVYM$X_{20}$HRSCGTSCFELEKLCRFIMSVKKNYRRVPYHNWKHAVTVAHCMYAILQNN$X_{21}X_{22}$
LFTDLERKGLLIACLCHDLDHRGFSNSYLQKFDHPL$X_{23}$ALYSTSTMEQHHFSQTVSILQL
EGHNIFSTLSSSEYEQVLEIIRKAIIATDLALYFGNRKQLEEMYQTGSLNL$X_{24}$NQSHRDR
VIGLMMTACDLCSVTK$X_{25}$WPVTKLTANDIYAEFWAEGDEMKKLGIQPIPMMDRDK$X_{26}$DEV
PQGQLGFYNAVAIPCYTTLTQILPPTEPLLKACRDNL$X_{27}$QWEKVIRGEETA$X_{28}$WIS

wherein

$X_0$ = S or G
$X_1$ = N or T
$X_2$ = S or A
$X_3$ = E or K
$X_4$ = S or P
$X_5$ = A or S
$X_6$ = Q or H
$X_7$ = K or R
$X_8$ = I or V
$X_9$ = T or I
$X_{10}$ = I or M

$X_{11}$ = K or Q
$X_{12}$ = V or I
$X_{13}$ = V or I
$X_{14}$ = Q or R
$X_{15}$ = T or N
$X_{16}$ = V or A
$X_{17}$ = L or I
$X_{18}$ = K or R
$X_{19}$ = E or D
$X_{20}$ = V or I
$X_{21}$ = H or N
$X_{22}$ = T or G
$X_{23}$ = T or A
$X_{24}$ = N or H
$X_{25}$ = P or L
$X_{26}$ = K or R
$X_{27}$ = S or N
$X_{28}$ = T or M

A preferred example of an amino acid sequence comprising the sequence presented as Formula I is the amino acid sequence shown as Formula VI or a variant, homologue, fragment or derivative thereof.

## Formula VI

$X_0$LTDEKVKAYLSLHPQVLDEFVSESVSAETVEKWLKRK$X_1$NK$X_2$$X_3$DE$X_4$$X_5$PKEVSRYQDT
NMQGVVYELNSYIEQRLDTGGDN$X_6$LLLYELSSII$X_7$IATKADGFALYFLGECNNSLC$X_8$FX
$_9$PPG$X_{10}$KEG$X_{11}$PRLIPAGPITQGTT$X_{12}$SAYVAKSRKTLLVEDILGDERFPRGTGLESGTR
IQSVLCLPIVTAIGDLIGILELYRHWGKEAFCLSHQEVATANLAWASVAIHQVQVCRGLAK
QTELNDFLLDVSKTYFDNIVAIDSLLEHIMIYAKNLVNADRCALFQVDHKNKELYSDLFDI
GEEKEGKP$X_{13}$FKKTKEIRFSIEKGIAGQVARTGEVLNIPDAYADPRFNREVDLYTGYTTR
NILCMPIVSRGSVIGVVQMVNKISGSAFSKTDENNFKMFAVFCALALHCANMYHRIRHSEC
IYRVTMEKLSYHSICTSEEWQGLM$X_{14}$F$X_{15}$LP$X_{16}$R$X_{17}$C$X_{18}$$X_{19}$IELFHFDIGPFENMWPGI
FVYM$X_{20}$HRSCGTSCFELEKLCRFIMSVKKNYRRVPYHNWKHAVTVAHCMYAILQNN$X_{21}$$X_{22}$
LFTDLERKGLLIACLCHDLDHRGFSNSYLQKFDHPL$X_{23}$ALYSTSTMEQHHFSQTVSILQL
EGHNIFSTLSSSEYEQVLEIIRKAIIATDLALYFGNRKQLEEMYQTGSLNL$X_{24}$NQSHRDR
VIGLMMTACDLCSVTK$X_{25}$WPVTKLTANDIYAEFWAEGDEMKKLGIQPIPMMDRDK$X_{26}$DEV
PQGQLGFYNAVAIPCYTTLTQILPPTEPLLKACRDNL$X_{27}$QWEKVIRGEETA$X_{28}$WIS$X_{29}$PX
$_{30}$$X_{31}$A

wherein

$X_0$ = S or G
$X_1$ = N or T
$X_2$ = S or A
$X_3$ = E or K
$X_4$ = S or P
$X_5$ = A or S
$X_6$ = Q or H
$X_7$ = K or R
$X_8$ = I or V
$X_9$ = T or I
$X_{10}$ = I or M

$X_{11}$ = K or Q
$X_{12}$ = V or I
$X_{13}$ = V or I
$X_{14}$ = Q or R
$X_{15}$ = T or N
$X_{16}$ = V or A
$X_{17}$ = L or I
$X_{18}$ = K or R
$X_{19}$ = E or D
$X_{20}$ = V or I
$X_{21}$ = H or N
$X_{22}$ = T or G
$X_{23}$ = T or A
$X_{24}$ = N or H
$X_{25}$ = P or L
$X_{26}$ = K or R
$X_{27}$ = S or N
$X_{28}$ = T or M
$X_{29}$ = S or G
$X_{30}$ = S or G
$X_{31}$ = V or P

A more preferred example of an amino acid sequence comprising the sequence presented as Formula I is the amino acid sequence shown as Formula VII or a variant, homologue, fragment or derivative thereof.

## Formula VII

$X_{0}$LTDEKVKAYLSLHPQVLDEFVSESVSAETVEKWLKRKX$_{1}$NKX$_{2}$X$_{3}$DEX$_{4}$X$_{5}$PKEVSRYQDT
NMQGVVYELNSYIEQRLDTGGDNX$_{6}$LLLYELSSIIX$_{7}$IATKADGFALYFLGECNNSLCX$_{8}$FX$_{9}$
PPGX$_{10}$KEGX$_{11}$PRLIPAGPITQGTTX$_{12}$SAYVAKSRKTLLVEDILGDERFPRGTGLESGTR
IQSVLCLPIVTAIGDLIGILELYRHWGKEAFCLSHQEVATANLAWASVAIHQVQVCRGLAK
QTELNDFLLDVSKTYFDNIVAIDSLLEHIMIYAKNLVNADRCALFQVDHKNKELYSDLFDI
GEEKEGKPX$_{13}$FKKTKEIRFSIEKGIAGQVARTGEVLNIPDAYADPRFNREVDLYTGYTTR
NILCMPIVSRGSVIGVVQMVNKISGSAFSKTDENNFKMFAVFCALALHCANMYHRIRHSEC
IYRVTMEKLSYHSICTSEEWQGLMX$_{14}$FX$_{15}$LPX$_{16}$RX$_{17}$CX$_{18}$X$_{19}$IELFHFDIGPFENMWPGI
FVYMX$_{20}$HRSCGTSCFELEKLCRFIMSVKKNYRRVPYHNWKHAVTVAHCMYAILQNNX$_{21}$X$_{22}$
LFTDLERKGLLIACLCHDLDHRGFSNSYLQKFDHPLX$_{23}$ALYSTSTMEQHHFSQTVSILQL
EGHNIFSTLSSSEYEQVLEIIRKAIIATDLALYFGNRKQLEEMYQTGSLNLX$_{24}$NQSHRDR
VIGLMMTACDLCSVTKX$_{25}$WPVTKLTANDIYAEFWAEGDEMKKLGIQPIPMMDRDKX$_{26}$DEV
PQGQLGFYNAVAIPCYTTLTQILPPTEPLLKACRDNLX$_{27}$QWEKVIRGEETAX$_{28}$WISX$_{29}$PX$_{30}$
X$_{31}$AX$_{32}$X$_{33}$X$_{34}$X$_{35}$X$_{36}$X$_{37}$EX$_{38}$

wherein

$X_{0}$ = S or G
$X_{1}$ = N or T
$X_{2}$ = S or A
$X_{3}$ = E or K
$X_{4}$ = S or P
$X_{5}$ = A or S
$X_{6}$ = Q or H
$X_{7}$ = K or R
$X_{8}$ = I or V

$X_9$ = T or I
$X_{10}$ = I or M
$X_{11}$ = K or Q
$X_{12}$ = V or I
$X_{13}$ = V or I
$X_{14}$ = Q or R
$X_{15}$ = T or N
$X_{16}$ = V or A
$X_{17}$ = L or I
$X_{18}$ = K or R
$X_{19}$ = E or D
$X_{20}$ = V or I
$X_{21}$ = H or N
$X_{22}$ = T or G
$X_{23}$ = T or A
$X_{24}$ = N or H
$X_{25}$ = P or L
$X_{26}$ = K or R
$X_{27}$ = S or N
$X_{28}$ = T or M
$X_{29}$ = S or G
$X_{30}$ = S or G
$X_{31}$ = V or P
$X_{32}$ = Q or P
$X_{33}$ = K or S
$X_{34}$ = A or K
$X_{35}$ = A or S
$X_{36}$ = A or T
$X_{37}$ = S or P
$X_{38}$ = D or K

Preferred examples of an amino acid sequence comprising the sequence presented as Formula I include the amino acids shown as: SEQ ID No. 2 or SEQ ID No. 4 or SEQ ID No. 15, or an amino acid comprising SEQ ID No. 17 and/or SEQ ID No. 19, or a variant, homologue, fragment or derivative thereof

It is to be noted that references to Formula I and/or to any one or more of Formula II - Formula VII herein also apply equally to any one or more of: SEQ ID No. 2 or SEQ ID No. 4 or SEQ ID No. 15 or an amino acid comprising SEQ ID No. 17 and/or SEQ ID No. 19.

An example of a variant of any one of Formula II - Formula VII is an amino acid sequence wherein the group .... $KRKX_1NKX_2X_3$.... is substituted with ....$KRKX_1DKX_2X_3$.... .

An example of a variant of any one of Formula II - Formula VII is an amino acid sequence wherein the group .... $LLLYELSSIIX_7$.... is substituted with .... $LLLYELNSSIIX_7$.....

Preferably references to Formula I and/or to any one or more of Formula II - Formula VII herein mean any one or more of: SEQ ID No. 2 or SEQ ID No. 4 or SEQ ID No. 15 or an amino acid comprising SEQ ID No. 17 and/or SEQ ID No. 19.

More preferably references to Formula I and/or to any one or more of Formula II - Formula VII herein mean any one or more of: SEQ ID No. 2 or SEQ ID No. 4.

According to a second aspect of the present invention there is provided an amino acid sequence comprising the sequence presented as Formula I.

According to a third aspect of the present invention there is provided a nucleotide sequence encoding the amino acid sequence of the present invention.

According to a fourth aspect of the present invention there is provided a nucleotide sequence comprising the sequence presented as SEQ ID No. 1 or SEQ ID No. 3 or SEQ ID No. 14 or a nucleotide sequence comprising SEQ ID No. 16 and/or SEQ ID No. 18, or a variant, homologue, fragment or derivative thereof.

According to a fifth aspect of the present invention there is provided a nucleotide sequence comprising the sequence presented as SEQ ID No. 1 or SEQ ID No. 3 or SEQ ID No. 14 or a nucleotide sequence comprising SEQ ID No. 16 and/or SEQ ID No. 18.

According to a sixth aspect of the present invention there is provided a nucleotide sequence that is capable of hybridising to the nucleotide sequence according to the present invention.

According to a seventh aspect of the present invention there is provided a nucleotide sequence that is capable of hybridising to the nucleotide sequence according to sixth aspect of the present invention.

According to an eighth aspect of the present invention there is provided a vector comprising the nucleotide sequence according to the present invention.

According to a ninth aspect of the present invention there is provided a host cell into which has been incorporated the nucleotide sequence according to the present invention.

According to a tenth aspect of the present invention there is provided an assay method for identifying an agent that can affect PDE11 activity or expression, the assay method comprising contacting an agent with an amino acid according to the present invention or a nucleotide sequence according to the present invention; and measuring the activity or expression of PDE11; wherein a difference between a) PDE activity or expression in the absence of the agent and b) PDE activity or expression in the presence of the agent is indicative that the agent can affect PDEII activity or expression. Preferably the assay is to screen for agents useful in the treatment of sexual dysfunction - such as male erectile dysfunction or female sexual dysfunction.

According to an eleventh aspect of the present invention there is provided a process comprising the steps of: (a) performing the assay according to the present invention; (b) identifying one or more agents that do affect PDE11 activity or expression; and (c) preparing a quantity of those one or more identified agents.

According to a twelfth aspect of the present invention there is provided a method of affecting *in vivo* PDE11 activity or expression with an agent; wherein the agent is capable of affecting PDE11 activity or expression in an *in vitro* assay method; wherein the *in vitro* assay method is the assay method of the present invention.

According to a thirteenth aspect of the present invention there is provided the use of an agent in the preparation of a pharmaceutical composition for the treatment of a disease or condition associated with PDE11, the agent is capable of having an effect on the activity or expression of PDE when assayed *in vitro* by the assay method of the present invention.

According to a fourteenth aspect of the present invention there is provided an enzyme capable of having an immunological reaction with an antibody raised against PDE11.

According to a fifteenth aspect of the present invention there is provided a nucleotide sequence coding for a PDE, wherein the nucleotide sequence is obtainable from NCIMB 40925 or NCIMB 40926 or NCIMB 41007.

According to a sixteenth aspect of the present invention there is provided a PDE wherein the PDE is expressable from a nucleotide sequence obtainable from NCIMB 40925 or NCIMB 40926 or NCIMB 41007.

According to a seventeenth aspect of the present invention there is provided the use of an agent which has an effect on the activity of PDE11 or the expression thereof in the preparation of a pharmaceutical composition for the treatment of a disease or condition associated with PDE11.

According to a further aspect of the present invention there is provided:

A nucleotide sequence selected from:

(a) the nucleotide sequence presented as SEQ ID No. 1, 3, 14, or a nucleotide sequence comprising SEQ ID No. 16 and/or SEQ ID No. 18;

(b) a nucleotide sequence that is a variant, homologue, derivative or fragment of the nucleotide sequence presented as SEQ ID No. 1, 3, 14, or a nucleotide sequence comprising SEQ ID No. 16 and/or SEQ ID No. 18;

(c) a nucleotide sequence that is the complement of the nucleotide sequence set out in SEQ ID No. 1, 3, 14, or a nucleotide sequence comprising SEQ ID No. 16 and/or SEQ ID No. 18;

(d) a nucleotide sequence that is the complement of a variant, homologue, derivative or fragment of the nucleotide sequence presented as SEQ ID No. 1, 3, 14, or a nucleotide sequence comprising SEQ ID No. 16 and/or SEQ ID No. 18;

(e) a nucleotide sequence that is capable of hybridising to the nucleotide sequence set out in SEQ ID No. 1, 3, 14, or a nucleotide sequence comprising SEQ ID No. 16 and/or SEQ ID No. 18:

(f) a nucleotide sequence that is capable of hybridising to a variant, homologue, derivative or fragment of the nucleotide sequence presented as SEQ ID No. 1. 3, 14, or a nucleotide sequence comprising SEQ ID No. 16 and/or SEQ ID No. 18;

(g) a nucleotide sequence that is the complement of a nucleotide sequence that is capable of hybridising to the nucleotide sequence set out in SEQ ID No. 1, 3, 14, or a nucleotide sequence comprising SEQ ID No. 16 and/or SEQ ID No. 18:

(h) a nucleotide sequence that is the complement of a nucleotide sequence that is capable of hybridising to a variant, homologue, derivative or fragment of the nucleotide sequence presented as SEQ ID No. 1, 3, 14, or a nucleotide sequence comprising SEQ ID No. 16 and/or SEQ ID No. 18;

(i) a nucleotide sequence that is capable of hybridising to the complement of the nucleotide sequence set out in SEQ ID No. 1, 3, 14, or a nucleotide sequence comprising SEQ ID No. 16 and/or SEQ ID No. 18;

(j) a nucleotide sequence that is capable of hybridising to the complement of a variant, homologue, derivative or fragment of the nucleotide sequence presented as SEQ ID No. 1, 3, 14, or a nucleotide sequence comprising SEQ ID No. 16 and/or SEQ ID No. 18;
(k) a nucleotide sequence which is degenerate as a result of the genetic code to the nucleotides defined in (a), (b), (c), (d), (e), (f), (g), (h), (i), or (j);
(l) a nucleotide sequence comprising any one of (a), (b), (c), (d), (e), (f), (g), (h), (i), (j) and/or (k).

Other aspects of the present invention include:

An isolated nucleotide sequence or protein sequence according to the present invention.
An assay method for identifying an agent that can affect the expression pattern of the nucleotide sequence of the present invention or the activity of the expression product thereof,
the assay method comprising

exposing the nucleotide sequence of the present invention or the expression product ("EP") thereof with an agent;

determining whether the agent modulates (such as affects the expression pattern or activity) the nucleotide sequence of the present invention or the expression product thereof.

An agent identified by the assay method of the present invention.
An agent identified by the assay method of the present invention, which agent has hitherto been unknown to have a PDE modulation effect in accordance with the present invention.
A process comprising the steps of:

(a) performing the assay of the present invention;

(b) identifying one or more agents that affect the expression pattern of the nucleotide sequence of the present invention or the activity of the expression product thereof;

(c) preparing a quantity of those one or more identified agents.

A process comprising the steps of:

(a) performing the assay according to the present invention;

(b) identifying one or more agents that affect the expression pattern of the nucleotide sequence of the present invention or the activity of the expression product thereof;

(c) preparing a pharmaceutical composition comprising one or more identified agents.

A process comprising the steps of:

(a) performing the assay according to the present invention;

(b) identifying one or more agents that affect the expression pattern of the nucleotide sequence of the present invention or the activity of the expression product thereof;

(c) modifying one or more identified agents to cause a different effect on the expression pattern of the nucleotide sequence of the present invention or the activity of the expression product thereof.

Use of an agent identified by an assay according to the present invention in the manufacture of a medicament which affects the expression pattern of the nucleotide sequence of the present invention or the activity of the expression product thereof.
A method of treating a target (which target can be a mammal, preferably a human), which method comprises delivering (such as administering or exposing) to the target an effective amount of an agent capable of modulating the expression pattern of the nucleotide sequence of the present invention or the activity of the expression product thereof.
A method of treating a target (which target can be a mammal, preferably a human), which method comprises delivering (such as administering or exposing) to the target an effective amount of an agent identified by an assay according to the present invention.

A method of inducing an immunological response in a subject, the method comprising administering to the subject the nucleotide sequence of the present invention or the expression product thereof.

PDE11(PDEXI)

Thus, the present invention relates to a novel PDE enzyme - which we have called PDE11 (which may otherwise be expressed as PDEXI) - and to a nucleotide sequence encoding same. The present invention also relates to the use of the novel nucleic acid and amino acid sequences in the diagnosis and treatment of disease. The present invention also relates to the use of the novel nucleic acid and amino acid sequences to evaluate and/or to screen for agents that can modulate phosphodiesterase activity. The present invention further relates to genetically engineered host cells that comprise or express the novel nucleic acid and amino acid sequences to evaluate and/or to screen for agents that can modulate phosphodiesterase activity.

As used herein, the term "PDE11" refers to a novel family of PDEs which, up until now, were uncharacterised. By way of example, we have identified two human isoenzymes of PDE11 - which we have called PDE11A1 and PDE11A2 (sometimes referred to as HSPDE11A1 and HSPDE11A2 respectively). We believe that PDE11A2 is a splice variant of PDE11A1. We have also identified a mouse sequence - which we have called PDE11A3 (sometimes referred to as MmPDE11A3). We have also identified a rat sequence - which we have partially sequenced - which we have PDE11A4 (sometimes referred to as RtPDE11A4).

For convenience, and unless otherwise stated, reference to PDE11 will include reference to PDE11A1 and/or PDE11A2 and/or PDE11A3 and/or PDE11A4.

PDE11 - such as the isoenzymes PDE11A1 and/or PDE11A2 - is believed to be present in, and obtainable from, a variety of sources. By way of example, PDE-11 is found in the striatum and in the corpus cavernosum. By way of further example, and in addition to human and mouse, PDE11 is also available from rat. We also believe that PDE11 is also present in a number of other sources - such as for example: bovine, ovine, porcine, and equine. Preferably, the present invention covers mammalian PDE 11 which includes but is not limited to any of the above sources.

The PDE11 may be the same as the naturally occuring form - for this aspect, preferably the PDE11 is the non-native amino acid sequence (i.e. it is not present in its natural form and in its natural environment) - or is a variant, homologue, fragment or derivative thereof. In addition, or in the alternative, the PDE11 is isolated PDE11 and/or purified PDE11. The PDE11 can be obtainable from or produced by any suitable source, whether natural or not, or it may be synthetic, semi-synthetic or recombinant.

The PDE11 coding sequence may be the same as the naturally occuring form - for this aspect, preferably the PDE11 coding sequence is the non-native nucleotide sequence (i.e. it is not present in its natural form and in its natural environment) - or is a variant, homologue, fragment or derivative thereof. In addition, or in the alternative, the PDE11 coding sequence is an isolated PDE11 coding sequence and/or a purified PDE11 coding sequence. The PDEII coding sequence can be obtainable from or produced by any suitable source, whether natural or not, or it may be synthetic, semi-synthetic or recombinant.

PDE11 is believed to be able to catalyse the conversion of cGMP to GMP. cGMP is the messenger in the male erectile response. Accordingly, inhibiting the activity of PDE11 is likely to increase the concentration of cGMP present and so enhance the male erectile response. Thus, PDE11 and/or its coding sequence and/or a sequence capable of hybridising thereto is/are useful for screening drug candidates for the treatment of male erectile dysfunction. In addition, it is believed that PDE11 and/or its coding sequence and/or a sequence capable of hybridising thereto is/are useful for screening drug candidates for the treatment of female sexual dysfunction. Also, PDE11 and/or its coding sequence and/or a sequence capable of hybridising thereto is/are useful for testing the selectivity of drug candidates between different PDEs.

Preferred aspects of the present invention include a recombinant PDE11 enzyme and a recombinant nucleotide sequence encoding a PDE11 enzyme.

Preferably the recombinant PDE11 enzyme and/or the recombinant nucleotide sequence of the present invention are a recombinant mammalian PDEII enzyme and/or a recombinant mammalian nucleotide sequence.

In accordance with the present invention, the recombinant PDE11 enzyme has at least the formula presented as Formula I.

Either or both of the nucleotide sequence coding for PDE11 or the enzyme PDE11 itself may be used to screen for agents that can affect PDE11 activity. In particular, the nucleotide sequence coding for PDE11 or PDE11 itself may be used to screen for agents that can inhibit PDE11 activity. In addition, the nucleotide sequence coding for PDE11 or the enzyme PDE11 itself may be used to screen for agents that selectively affect PDE11 activity, such as selectively inhibit PDE11 activity.

Furthermore, the nucleotide sequence coding for PDE11 or a sequence that is complementary thereto may also be used in assays to detect the presence of PDE11 coding sequences in human cells. These assays would provide information regarding the tissue distribution of this enzyme and its biological relevance with respect to particular disease states.

The present invention also covers antibodies to PDE11 (including a derivative, fragment, homologue or variant thereof). The antibodies for PDE11 may be used in assays to detect the presence of PDE11 in human cells. These assays would provide information regarding the tissue distribution of this enzyme and its biological relevance with respect to particular disease states.

In particular, any one or more of the PDE11 isozymes (i.e. PDE11A1 and PDE11A2 or PDE11A3 or PDE11A4), the nucleotide sequences coding for same, the nucleotide sequences that are complementary to same, and the antibodies directed to same may be used in assays to screen for agents that selectively affect one of the isozymes. These assays would provide information regarding the tissue distribution of each of the isozymes and to provide information regarding the biological relevance of each of the isozymes with respect to particular disease states. These assays would also allow workers to test for and identify agents that are useful to affect the expression of or activity of PDE11 - such as in a particular tissue or in a particular disease state.

NATURALLY OCCURRING

As used herein "naturally occurring" refers to a PDE11 with an amino acid sequence found in nature.

ISOLATED/PURIFIED

As used herein, the terms "isolated" and "purified" refer to molecules, either nucleic or amino acid sequences, that are removed from their natural environment and isolated or separated from at least one other component with which they are naturally associated.

BIOLOGICALLY ACTIVE

As used herein "biologically active" refers to a PDE11 according to the present invention - such as a recombinant PDE11 - having a similar structural function (but not necessarily to the same degree), and/or similar regulatory function (but not necessarily to the same degree), and/or similar biochemical function (but not necessarily to the same degree) and/or immunological activity (but not necessarily to the same degree) of the naturally occurring PDE11. Specifically, a PDE11 of the present invention has the ability to hydrolyze a cyclic nucleotide, which is one of the characteristic activities of the PDE enzyme of the present invention.

IMMUNOLOGICAL ACTIVITY

As used herein, "immunological activity" is defined as the capability of the natural, recombinant or synthetic PDE11 or any oligopeptide thereof, to induce a specific immune response in appropriate animals or cells and to bind with specific antibodies.

DERIVATIVE

The term "derivative" as used herein in relation to the amino acid sequence includes chemical modification of a PDE11. Illustrative of such modifications would be replacement of hydrogen by an alkyl, acyl, or amino group.

DELETION

As used herein a "deletion" is defined as a change in either nucleotide or amino acid sequence in which one or more nucleotides or amino acid residues, respectively, are absent.

INSERTION/ADDITION

As used herein an "insertion" or "addition" is a change in a nucleotide or amino acid sequence which has resulted in the addition of one or more nucleotides or amino acid residues, respectively, as compared to the naturally occurring PDE.

SUBSTITUTION

As used herein "substitution" results from the replacement of one or more nucleotides or amino acids by different nucleotides or amino acids, respectively.

VARIANT/HOMOLOGUE

The terms "variant" or "homologue" with respect to the nucleotide sequence of the present invention and the amino acid sequence of the present invention are synonymous with allelic variations of the sequences.

In particular, the term "homology" as used herein may be equated with the term "identity". Here, sequence homology with respect to the nucleotide sequence of the present invention and the amino acid sequence of the present invention can be determined by a simple "eyeball" comparison (i.e. a strict comparison) of any one or more of the sequences with another sequence to see if that other sequence has at least 75 % identity to the sequence(s). Relative sequence homology (i.e. sequence identity) can also be determined by commercially available computer programs that can calculate % homology between two or more sequences. A typical example of such a computer program is CLUSTAL.

Sequence homology (or identity) may even be determined using any suitable homology algorithm, using for example default parameters. Advantageously, the BLAST algorithm is employed, with parameters set to default values. The BLAST algorithm is described in detail at http://www.ncbi.nih.gov/BLAST/blast_help.html, which is incorporated herein by reference. The search parameters are defined as follows, and are advantageously set to the defined default parameters.

Advantageously, "substantial homology" when assessed by BLAST equates to sequences which match with an EXPECT value of at least about 7, preferably at least about 9 and most preferably 10 or more. The default threshold for EXPECT in BLAST searching is usually 10.

BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul (see http://www.ncbi.nib.gov/BLAST/blast_help.html) with a few enhancements. The BLAST programs were tailored for sequence similarity searching, for example to identify homologues to a query sequence. The programs are not generally useful for motif-style searching. For a discussion of basic issues in similarity searching of sequence databases, see Altschul et al (1994) Nature Genetics 6:119-129.

The five BLAST programs available at http://www.ncbi.nlm.nih.gov perform the following tasks:

> **blastp** compares an amino acid query sequence against a protein sequence database;
>
> **blastn** compares a nucleotide query sequence against a nucleotide sequence database;
>
> **blastx** compares the six-frame conceptual translation products of a nucleotide query sequence (both strands) against a protein sequence database;
>
> **tblastn** compares a protein query sequence against a nucleotide sequence database dynamically translated in all six reading frames (both strands).
>
> **tblastx** compares the six-frame translations of a nucleotide query sequence against the six-frame translations of a nucleotide sequence database.

BLAST uses the following search parameters:

HISTOGRAM Display a histogram of scores for each search; default is yes. (See parameter H in the BLAST Manual).

DESCRIPTIONS Restricts the number of short descriptions of matching sequences reported to the number specified; default limit is 100 descriptions. (See parameter V in the manual page). See also EXPECT and CUTOFF.

ALIGNMENTS Restricts database sequences to the number specified for which high-scoring segment pairs (HSPs) are reported; the default limit is 50. If more database sequences than this happen to satisfy the statistical significance threshold for reporting (see EXPECT and CUTOFF below), only the matches ascribed the greatest statistical significance are reported. (See parameter B in the BLAST Manual).

EXPECT The statistical significance threshold for reporting matches against database sequences; the default value is 10, such that 10 matches are expected to be found merely by chance, according to the stochastic model of Karlin and Altschul (1990). If the statistical significance ascribed to a match is greater than the EXPECT threshold, the match will not be reported. Lower EXPECT thresholds are more stringent, leading to fewer chance matches being reported. Fractional values are acceptable. (See parameter E in the BLAST Manual).

CUTOFF Cutoff score for reporting high-scoring segment pairs. The default value is calculated from the EXPECT value (see above). HSPs are reported for a database sequence only if the statistical significance ascribed to them is at least as high as would be ascribed to a lone HSP having a score equal to the CUTOFF value. Higher CUTOFF values are more stringent, leading to fewer chance matches being reported. (See parameter S in the BLAST Manual).

Typically, significance thresholds can be more intuitively managed using EXPECT.

MATRIX Specify an alternate scoring matrix for BLASTP, BLASTX, TBLASTN and TBLASTX. The default matrix is BLOSUM62 (Henikoff & Henikoff, 1992). The valid alternative choices include: PAM40, PAM120, PAM250 and IDENTITY. No alternate scoring matrices are available for BLASTN; specifying the MATRIX directive in BLASTN requests returns an error response.

STRAND Restrict a TBLASTN search to just the top or bottom strand of the database sequences; or restrict a BLASTN, BLASTX or TBLASTX search to just reading frames on the top or bottom strand of the query sequence.

FILTER Mask off segments of the query sequence that have low compositional complexity, as determined by the SEG program of Wootton & Federhen (1993) Computers and Chemistry 17:149-163, or segments consisting of short-periodicity internal repeats, as determined by the XNU program of Claverie & States (1993) Computers and Chemistry 17:191-201, or, for BLASTN, by the DUST program of Tatusov and Lipman (see http://www.ncbi.nlm.nih.gov). Filtering can eliminate statistically significant but biologically uninteresting reports from the blast output (e.g., hits against common acidic-, basic- or proline-rich regions), leaving the more biologically interesting regions of the query sequence available for specific matching against database sequences.

Low complexity sequence found by a filter program is substituted using the letter "N" in nucleotide sequence (e.g., "NNNNNNNNNNNNN") and the letter "X" in protein sequences (e.g., "XXXXXXXXX").

Filtering is only applied to the query sequence (or its translation products), not to database sequences. Default filtering is DUST for BLASTN, SEG for other programs.

It is not unusual for nothing at all to be masked by SEG, XNU, or both, when applied to sequences in SWISS-PROT, so filtering should not be expected to always yield an effect. Furthermore, in some cases, sequences are masked in their entirety, indicating that the statistical significance of any matches reported against the unfiltered query sequence should be suspect.

NCBI-gi Causes NCBI gi identifiers to be shown in the output, in addition to the accession and/or locus name.

Most preferably, sequence comparisons are conducted using the simple BLAST search algorithm provided at http://www.ncbi.nlm .nih.gov/BLAST.

Other computer program methods to determine identify and similarity between the two sequences include but are not limited to the GCG program package (Devereux et al 1984 Nucleic Acids Research 12: 387 and FASTA (Atschul et al 1990 J Molec Biol 403-410).

Should Gap Penalties be used when determining sequence identity, then preferably the following parameters are used:

| **FOR BLAST** | |
|---|---|
| GAP OPEN | 0 |
| GAP EXTENSION | 0 |

| **FOR CLUSTAL** | DNA | PROTEIN | |
|---|---|---|---|
| WORD SIZE | 2 | 1 | K triple |
| GAP PENALTY | 10 | 10 | |
| GAP EXTENSION | 0.1 | 0.1 | |

As used herein, the terms "variant", "homologue", "fragment" and "derivative" embrace allelic variations of the sequences.

The term "variant" also encompasses sequences that are complementary to sequences that are capable of hydridising to the nucleotide sequences presented herein.

HYBRIDISATION

The term "hybridization" as used herein shall include "the process by which a strand of nucleic acid joins with a complementary strand through base pairing" (Coombs J (1994) Dictionary of Biotechnology, Stockton Press, New York NY) as well as the process of amplification as carried out in polymerase chain reaction technologies as described in Dieffenbach CW and GS Dveksler (1995, PCR Primer, a Laboratory Manual, Cold Spring Harbor Press, Plainview NY).

Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

Stringency of hybridisation refers to conditions under which polynucleic acids hybrids are stable. Such conditions are evident to those of ordinary skill in the field. As known to those of skill in the art, the stability of hybrids is reflected in the melting temperature (Tm) of the hybrid which decreases approximately 1 to 1.5°C with every 1 % decrease in sequence homology. In general, the stability of a hybrid is a function of sodium ion concentration and temperature. Typically, the hybridisation reaction is performed under conditions of higher stringency, followed by washes of varying stringency.

As used herein, high stringency refers to conditions that permit hybridisation of only those nucleic acid sequences that form stable hybrids in 1 M Na+ at 65-68 °C

Maximum stringency typically occurs at about Tm-5°C (5°C below the Tm of the probe).

High stringency at about 5°C to 10°C below the Tm of the probe. High stringency conditions can be provided, for example, by hybridisation in an aqueous solution containing 6x SSC, 5x Denhardt's, 1 % SDS (sodium dodecyl sulphate), 0.1 Na+ pyrophosphate and 0.1 mg/ml denatured salmon sperm DNA as non specific competitor. Following hybridisation, high stringency washing may be done in several steps, with a final wash (about 30 min) at the hybridisation temperature in 0.2 - 0.1x SSC, 0.1 % SDS.

Moderate, or intermediate, stringency typically occurs at about 10°C to 20°C below the Tm of the probe.

Low stringency typically occurs at about 20°C to 25°C below the Tm of the probe.

As will be understood by those of skill in the art, a maximum stringency hybridization can be used to identify or detect identical polynucleotide sequences while an intermediate (or low) stringency hybridization can be used to identify or detect similar or related polynucleotide sequences.

Moderate stringency refers to conditions equivalent to hybridisation in the above described solution but at about 60-62°C. In that case the final wash is performed at the hybridisation temperature in 1x SSC, 0.1 % SDS.

Low stringency refers to conditions equivalent to hybridisation in the above described solution at about 50-52°C. In that case, the final wash is performed at the hybridisation temperature in 2x SSC, 0.1 % SDS.

It is understood that these conditions may be adapted and duplicated using a variety of buffers, e.g. formamide-based buffers, and temperatures. Denhardt's solution and SSC are well known to those of skill in the art as are other suitable hybridisation buffers (see, e.g. Sambrook, et al., eds. (1989) Molecular Cloning: A Laboratory Manual, Cold Spring Harbor Laboratory Press, New York or Ausubel, et al., eds. (1990) Current Protocols in Molecular Biology, John Wiley & Sons, Inc.). Optimal hybridisation conditions have to be determined empirically, as the length and the GC content of the probe also play a role.

POLYPEPTIDE OF THE PRESENT INVENTION

Polypeptides of the present invention may be in a substantially isolated form. It will be understood that the polypeptide may be mixed with carriers or diluents which will not interfere with the intended purpose of the polypeptide and still be regarded as substantially isolated. A polypeptide of the present invention may also be in a substantially purified form, in which case it will generally comprise the polypeptide in a preparation in which more than 90%, e.g. 95%, 98% or 99% of the polypeptide in the preparation is a polypeptide of the present invention. Polypeptides of the present invention may be modified for example by the addition of histidine residues to assist their purification or by the addition of a signal sequence to promote their secretion from a cell as discussed below.

Polypeptides of the present invention may be produced by synthetic means (e.g. as described by Geysen *et al.,* 1996) or recombinantly, as described below.

In a preferred embodiment, the amino acid sequence *per se* the present invention does not cover the native PDE11 according to the present invention when it is in its natural environment and when it has been expressed by its native nucleotide coding sequence which is also in its natural environment and when that nucleotide sequence is under the control of its native promoter which is also in its natural environment. For ease of reference, we shall call this preferred embodiment the "non-native amino acid sequence".

The terms "variant", "homologue" or "fragment" in relation to the amino acid sequence for the enzyme of the present invention include any substitution of, variation of, modification of, replacement of, deletion of or addition of one (or more) amino acid from or to the sequence providing the resultant enzyme has PDE11 activity, preferably being at least as biologically active as the enzyme shown as SEQ ID No. 2. or SEQ ID No. 4 or SEQ ID No. 15. In particular, the term "homologue" covers homology with respect to structure and/or function. With respect to sequence homology, preferably there is at least 75%, more preferably at least 85%, more preferably at least 90% homology to the sequence shown as Formula I. More preferably there is at least 95 %, more preferably at least 98%, homology to the sequence shown as Formula I. More preferably there is at least 75%, more preferably at least 85%, more preferably at least 90% homology to the sequence shown as SEQ ID No. 2. or SEQ ID No. 4 or SEQ ID No. 15, or an amino acid sequence comprising SEQ ID No. 17 and/or SEQ ID No. 19. More preferably there is at least 95%, more preferably at least 98%, homology to the sequence shown as SEQ ID No. 2. or SEQ ID No. 4 or SEQ ID No. 15.

Typically, the types of amino acid substitutions that could be made should maintain the hydrophobicity/hydrophilicity of the amino acid sequence. Amino acid substitutions may be made, for example from 1, 2 or 3 to 10, 20 or 30 substitutions provided that the modified sequence retains the ability to act as a PDE enzyme in accordance with present invention. Amino acid substitutions may include the use of non-naturally occurring analogues, for example to increase blood plasma half-life.

The amino acid sequence of the present invention may be produced by expression of a nucleotide sequence coding for same in a suitable expression system.

In addition, or in the alternative, the protein itself could be produced using chemical methods to synthesize a PDE amino acid sequence, in whole or in part. For example, peptides can be synthesized by solid phase techniques, cleaved from the resin, and purified by preparative high performance liquid chromatography (e.g., Creighton (1983) Proteins Structures And Molecular Principles, WH Freeman and Co, New York NY). The composition of the synthetic peptides may be confirmed by amino acid analysis or sequencing (e.g., the Edman degradation procedure).

Direct peptide synthesis can be performed using various solid-phase techniques (Roberge JY *et al* (1995) Science 269: 202-204) and automated synthesis may be achieved, for example, using the ABI 43 1 A Peptide Synthesizer (Perkin Elmer) in accordance with the instructions provided by the manufacturer. Additionally, the amino acid sequence of PDE, or any part thereof, may be altered during direct synthesis and/or combined using chemical methods with a sequence from other subunits, or any part thereof, to produce a variant polypeptide.

In another embodiment of the invention, a PDE natural, modified or recombinant sequence may be ligated to a heterologous sequence to encode a fusion protein. For example, for screening of peptide libraries for inhibitors of PDE activity, it may be useful to encode a chimeric PDE protein expressing a heterologous epitope that is recognized by a commercially available antibody. A fusion protein may also be engineered to contain a cleavage site located between a PDE sequence and the heterologous protein sequence, so that the PDE may be cleaved and purified away from the heterologous moiety.

PDE may also be expressed as a recombinant protein with one or more additional polypeptide domains added to facilitate protein purification. Such purification facilitating domains include, but are not limited to, metal chelating peptides such as histidine-tryptophan modules that allow purification on immobilized metals (Porath J (1992) Protein Expr Purif 3 -.26328 1), protein A domains that allow purification on immobilized immunoglobulin, and the domain utilized in the FLAGS extension/affinity purification system (Immunex Corp, Seattle, WA). The inclusion of a cleavable linker sequence such as Factor XA or enterokinase (Invitrogen, San Diego, CA) between the purification domain and PDE is useful to facilitate purification.

Specific amino acid sequences of PDE11 are shown as SEQ ID No. 2 and SEQ ID No. 4 and SEQ ID No. 15 and SEQ ID No. 17 and SEQ ID No. 19. However, the present invention encompasses amino acid sequences encoding other members from the PDE11 family which would include amino acid sequences having at least 60% identity (more preferably at least 75% identity) to the amino acid sequence of SEQ ID Nos. 2 or 4 or 15 or 17 or 19. As indicated, suitable generic formulae for the PDE11 family in accordance with the present invention are presented as Formula I or any one of Formula II-Formula VII.

Polypeptides of the present invention also include fragments of the presented amino acid sequence and variants thereof. Suitable fragments will be at least 5, e.g. at least 10, 12, 15 or 20 amino acids in size.

Polypeptides of the present invention may also be modified to contain one or more (e.g. at least 2, 3, 5, or 10) substitutions, deletions or insertions, including conserved substitutions.

Conserved substitutions may be made according to the following table which indicates conservative substitutions, where amino acids on the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |
| OTHER | | N Q D E |

NUCLEOTIDE SEQUENCE OF THE PRESENT INVENTION

The term "nucleotide sequence" as used herein refers to an oligonucleotide sequence or polynucleotide sequence, and variants, homologues, fragments and derivatives thereof (such as portions thereof). The nucleotide sequence may be DNA or RNA which may be of genomic or synthetic or recombinant origin which may be double-stranded or single-stranded whether representing the sense or antisense strand.

Preferably, the term "nucleotide sequence" means DNA.

More preferably, the term "nucleotide sequence" means DNA prepared by use of recombinant DNA techniques (i.e. recombinant DNA).

In a preferred embodiment, the nucleotide sequence *per se* of the present invention does not cover the native nucleotide coding sequence according to the present invention in its natural environment when it is under the control of its native promoter which is also in its natural environment. For ease of reference, we shall call this preferred embodiment the "non-native nucleotide sequence".

The nucleotide sequences of the present invention may include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present invention, it is to be understood that the nucleotide sequences described herein may be modified by any method available in the art. Such modifications may be carried out in to enhance the *in vivo* activity or life span of nucleotide sequences of the present invention.

The present invention also encompasses nucleotide sequences that are complementary to the sequences presented herein, or any derivative, fragment or derivative thereof. If the sequence is complementary to a fragment thereof then that sequence can be used a probe to identify similar coding sequences in other organisms etc.

The present invention also encompasses nucleotide sequences that are capable of hybridising to the sequences presented herein, or any derivative, fragment or derivative thereof.

The present invention also encompasses nucleotide sequences that are capable of hybridising to the sequences that are complementary to the sequences presented herein, or any derivative, fragment or derivative thereof.

The term "variant" also encompasses sequences that are complementary to sequences that are capable of hydridising to the nucleotide sequences presented herein.

Preferably, the term "variant" encompasses sequences that are complementary to sequences that are capable of hydridising under stringent conditions (e.g. 65°C and 0.1xSSC {1xSSC = 0.15 M NaCl, 0.015 $Na_3$ citrate pH 7.0}) to the nucleotide sequences presented herein.

The present invention also relates to nucleotide sequences that can hybridise to the nucleotide sequences of the present invention (including complementary sequences of those presented herein).

The present invention also relates to nucleotide sequences that are complementary to sequences that can hybridise to the nucleotide sequences of the present invention (including complementary sequences of those presented herein).

Also included within the scope of the present invention are polynucleotide sequences that are capable of hybridizing to the nucleotide sequences presented herein under conditions of intermediate to maximal stringency.

In a preferred aspect, the present invention covers nucleotide sequences that can hybridise to the nucleotide sequence of the present invention under stringent conditions (e.g. 65°C and 0.1xSSC).

Exemplary nucleic acids can alternatively be characterised as those nucleotide sequences which encode a PDE 11 protein and hybridise to the DNA sequences set forth as SEQ ID No. 1 or SEQ ID No. 3 or SEQ ID No. 14 or SEQ ID No. 16 or SEQ ID No. 18, or a selected fragment of said DNA sequence. Preferred are such sequences encoding PDE 11 which hybridise under high-stringency conditions to the sequence of SEQ ID No. 1 or SEQ ID No. 3 or SEQ ID No. 14 or SEQ ID No. 16 or SEQ ID No. 18.

Advantageously, the invention provides nucleic acid sequences which are capable of hybridising, under stringent conditions, to a fragment of SEQ. ID. No. 1 or to a fragment of SEQ ID No. 3 or to a fragment of SEQ ID No. 14 or SEQ ID No. 16 or SEQ ID No. 18. Preferably, the fragment is between 15 and 50 bases in length. Advantageously, it is about 25 bases in length.

The terms "variant", "homologue" or "fragment" in relation to the nucleotide sequence coding for the preferred enzyme of the present invention include any substitution of, variation of, modification of, replacement of, deletion of or addition of one (or more) nucleic acid from or to the sequence providing the resultant nucleotide sequence codes for or is capable of coding for an enzyme having PDE11 activity, preferably being at least as biologically active as the enzyme encoded by the sequences shown as SEQ ID No. 1 or SEQ ID No. 3 or SEQ ID No. 14. In particular, the term "homologue" covers homology with respect to structure and/or function providing the resultant nucleotide sequence codes for or is capable of coding for an enzyme having PDE11 activity. With respect to sequence homology, preferably there is at least 75%, more preferably at least 85 %, more preferably at least 90% homology to a nucleotide sequence coding for the amino acid sequence shown as Formula I. More preferably there is at least 95%, more preferably at least 98 homology to a nucleotide sequence coding for the amino acid sequence shown as Formula I. Preferably, with respect to sequence homology, preferably there is at least 75%, more preferably at least 85%, more preferably at least 90% homology to the sequence shown as SEQ ID No. 1 or SEQ ID No. 3 or SEQ ID No. 14 or SEQ ID No. 16 or SEQ ID No. 18. More preferably there is at least 95%, more preferably at least 98%, homology to the sequence shown as SEQ ID No. 1 or SEQ ID No. 3 or SEQ ID No. 14.

As indicated, the present invention relates to a DNA sequence (preferably a cDNA sequence) encoding PDE11. In particular, the present invention relates to cDNA sequences encoding PDE11A1 or PDE11A2 or PDE11A3 or PDE11A4.

The present invention also relates to DNA segments comprising the DNA sequence of SEQ ID No. 1 or SEQ ID No. 3 or SEQ ID No. 14 or SEQ ID No. 16 or SEQ ID No. 18 or allelic variations of such sequences.

The present invention also relates to polypeptides produced by expression in a host cell into which has been incorporated the foregoing DNA sequences or allelic variations thereof.

A highly preferred aspect of the present invention relates to a polypeptide comprising the amino acid sequence of Formula I (such as SEQ ID No. 2 or SEQ ID No. 4 or SEQ ID No. 15 or SEQ ID No. 17 or SEQ ID No. 19). For example, the present invention relates to an isolated polypeptide comprising the amino acid sequence of Formula I (such as SEQ ID No. 2 or SEQ ID No. 4 or SEQ ID No. 15 or SEQ ID No. 17 or SEQ ID No. 19).

The present invention also relates to DNA comprising the DNA sequence of SEQ ID No. 1 or SEQ ID No. 3 or SEQ ID No. 14 or SEQ ID No. 16 or SEQ ID No. 18 or an allelic variation thereof.

The present invention also relates to non-native DNA comprising the DNA sequence of SEQ ID No. 1 or SEQ ID No. 3 or SEQ ID No. 14 or SEQ ID No. 16 or SEQ ID No. 18 or an allelic variation thereof.

A highly preferred aspect of the present invention relates to recombinant DNA comprising the DNA sequence of SEQ ID No. 1 or SEQ ID No. 3 or SEQ ID No. 14 or SEQ ID No. 16 or SEQ ID No. 18 or an allelic variation thereof.

Polynucleotides of the present invention include nucleotide acid sequences encoding the polypeptides of the present invention. It will appreciated that a range of different polynucleotides encode a given amino acid sequence as a consequence of the degeneracy of the genetic code.

By knowledge of the amino acid sequences set out herein it is possible to devise partial and full-length nucleic acid sequences such as cDNA and/or genomic clones that encode the polypeptides of the present invention. For example, polynucleotides of the present invention may be obtained using degenerate PCR which will use primers designed to target sequences encoding the amino acid sequences presented herein. The primers will typically contain multiple degenerate positions. However, to minimise degeneracy, sequences will be chosen that encode regions of the amino acid sequences presented herein containing amino acids such as methionine which are coded for by only one triplet. In addition, sequences will be chosen to take into account codon usage in the organism whose nucleic acid is used as the template DNA for the PCR procedure. PCR will be used at stringency conditions lower than those used for cloning sequences with single sequence (non-denegerate) primers against known sequences.

Nucleic acid sequences obtained by PCR that encode polypeptide fragments of the present invention may then be used to obtain larger sequences using hybridization library screening techniques. For example a PCR clone may be labelled with radioactive atoms and used to screen a cDNA or genomic library from other species, preferably other mammalian species. Hybridization conditions will typically be conditions of medium to high stringency (for example 0.03M sodium chloride and 0.03M sodium citrate at from about 50°C to about 60°C).

Degenerate nucleic acid probes encoding all or part of the amino acid sequence may also be used to probe cDNA and/or genomic libraries from other species, preferably other mammalian species. However, it is preferred to carry out PCR techniques initially to obtain a single sequence for use in further screening procedures.

In accordance with the present invention, PDE11 polynucleotide sequences which encode PDE11, fragments of the polypeptide, fusion proteins or functional equivalents thereof, may be used to generate recombinant DNA molecules that direct the expression of PDE11 in appropriate host cells. Due to the inherent degeneracy of the genetic code, other DNA sequences which encode substantially the same or a functionally equivalent amino acid sequence, may be used to clone and express PDE11. As will be understood by those of skill in the art, it may be advantageous to produce PDE-encoding nucleotide sequences possessing non-naturally occurring codons. Codons preferred by a particular prokaryotic or eukaryotic host (Murray E *et al* (1989) Nuc Acids Res 17:477-508) can be selected, for example, to increase the rate of PDE11 expression or to produce recombinant RNA transcripts having desirable properties, such as a longer half-life, than transcripts produced from naturally occurring sequence.

Polynucleotide sequences of the present invention obtained using the techniques described above may be used to obtain further homologous sequences and variants using the techniques described above. They may also be modified for use in expressing the polypeptides of the present invention in a variety of host cells systems, for example to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

Altered PDE11 polynucleotide sequences which may be used in accordance with the invention include deletions, insertions or substitutions of different nucleotide residues resulting in a polynucleotide that encodes the same or a functionally equivalent PDE. The protein may also have deletions, insertions or substitutions of amino acid residues which produce a silent change and result in a functionally equivalent PDE. Deliberate amino acid substitutions may be made on the basis of similarity in polarity, charge, solubility, hydrophobicity, hydrophilicity, and/or the amphipathic nature of the residues as long as the biological activity of PDE is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Included within the scope of the present invention are alleles of PDE. As used herein, an "allele" or "allelic sequence" is an alternative form of PDE. Alleles result from a mutation, i.e., a change in the nucleic acid sequence, and generally produce altered mRNAs or polypeptides whose structure or function may or may not be altered. Any given gene may have none, one or many allelic forms. Common mutational changes which give rise to alleles are generally ascribed to deletions, additions or substitutions of amino acids. Each of these types of changes may occur alone, or in combination with the others, one or more times in a given sequence.

The nucleotide sequences of the present invention may be engineered in order to alter a PDE coding sequence for a variety of reasons, including but not limited to, alterations which modify the cloning, processing and/or expression of the gene product. For example, mutations may be introduced using techniques which are well known in the art, e. g., site-directed mutagenesis to insert new restriction sites, to alter glycosylation patterns or to change codon preference.

Polynucleotides of the present invention may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides of the present invention as used herein.

Polynucleotides or primers of the present invention may carry a revealing label. Suitable labels include radioisotopes such as $^{32}P$ or $^{35}S$, enzyme labels, or other protein labels such as biotin. Such labels may be added to polynucleotides or primers of the present invention and may be detected using by techniques known *per se*.

Polynucleotides such as a DNA polynucleotide and primers according to the present invention may be produced recombinantly, synthetically, or by any means available to those of skill in the art. They may also be cloned by standard techniques.

In general, primers will be produced by synthetic means, involving a step wise manufacture of the desired nucleic acid sequence one nucleotide at a time. Techniques for accomplishing this using automated techniques are readily available in the art.

Longer polynucleotides will generally be produced using recombinant means, for example using a PCR (polymerase chain reaction) cloning techniques. This will involve making a pair of primers (e.g. of about 15-30 nucleotides) to a region of the nucleotide sequence which it is desired to clone, bringing the primers into contact with mRNA or cDNA obtained from a fungal, plant or prokaryotic cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

DNA molecules may be modified to increase intracellular stability and half-life. Possible modifications include, but are not limited to, the addition of flanking sequences of the 5' and/or 3' ends of the molecule or the use of phosphorothioate or 2' O-methyl rather than phosphodiesterase linkages within the backbone of the molecule.

As mentioned earlier, the present invention also relates to nucleotide sequences that are capable of hybridising to all or part of SEQ ID No. 1 or SEQ ID No. 3 or SEQ ID No. 14 or SEQ ID No. 16 or SEQ ID No. 18 or an allelic variation thereof. These nucleotide sequences may be used in anti-sense techniques to modify PDE11 expression. Alternatively, these sequences (or portions thereof) can be used as a probe, or for amplifying all or part of such sequence when used as a polymerase chain reaction primer.

In addition to the recombinant DNA sequences, genomic sequences are also of utility in the context of drug discovery. It may be valuable to inhibit the mRNA transcription of a particular isoform rather than to inhibit its translated protein. This is particularly true with PDE11, since the different splice variants may be transcribed from different promoters. There is precedent for multiple promoters directing the transcription of a mouse brain 2',3'-cyclic-nucleotide 3' phosphodiesterase (Kurihara T *et al.,* Biochem. Biophys. Res. Comm. 170: 1074 [1990]).

Another utility of the invention is that the DNA sequences, once known, give the information needed to design assays to specifically detect each isoenzyme or splice variant. Isozyme-specific PCR primer pairs are but one example of an assay that depends completely on the knowledge of the specific DNA sequence of the isozyme or splice variant. Such an assay allows detection of mRNA for the isozyme to access the tissue distribution and biological relevance of each isozyme to a particular disease state. It also allows identification of cell lines that may naturally express only one isozyme - a discovery that might obviate the need to express recombinant genes. If specific PDE11 isozymes are shown to associated with a particular disease state, the invention would be valuable in the design of diagnostic assays to detect the presence of isozyme mRNA.

An abnormal level of nucleotide sequences encoding a PDE11 in a biological sample may reflect a chromosomal aberration, such as a nucleic acid deletion or mutation. Accordingly, nucleotide sequences encoding a PDE11 provide the basis for probes which can be used diagnostically to detect chromosomal aberrations such as deletions, mutations or chromosomal translocations in the gene encoding PDE. PDE11 gene expression may be altered in such disease states or there may be a chromosomal aberration present in the region of the gene encoding a PDE11.

In an alternative embodiment of the invention, the coding sequence of PDE could be synthesized, in whole or in part, using chemical methods well known in the art (see Caruthers MH *et al* (1980) Nuc Acids Res Symp Ser 215-23, Horn T *et al* (1980) Nuc Acids Res Symp Ser 225-232).

REGULATORY SEQUENCES

Preferably, the polynucleotide of the present invention is operably linked to a regulatory sequence which is capable of providing for the expression of the coding sequence, such as by the chosen host cell. By way of example, the present invention covers a vector comprising the polynucleotide of the present invention operably linked to such a regulatory sequence, i.e. the vector is an expression vector.

The term "operably linked" refers to a juxtaposition wherein the components described are in a relationship permitting them to function in their intended manner. A regulatory sequence "operably linked" to a coding sequence is ligated in such a way that expression of the coding sequence is achieved under condition compatible with the control sequences.

The term "regulatory sequences" includes promoters and enhancers and other expression regulation signals.

The term "promoter" is used in the normal sense of the art, e.g. an RNA polymerase binding site.

Enhanced expression of the polynucleotide encoding the polypeptide of the present invention may also be achieved by the selection of heterologous regulatory regions, e.g. promoter, secretion leader and terminator regions, which serve to increase expression and, if desired, secretion levels of the protein of interest from the chosen expression host and/or to provide for the inducible control of the expression of' the polypeptide of the present invention

Preferably, the nucleotide sequence of the present invention may be operably linked to at least a promoter.

Aside from the promoter native to the gene encoding the polypeptide of the present invention, other promoters may be used to direct expression of the polypeptide of the present invention. The promoter may be selected for its efficiency in directing the expression of the polypeptide of the present invention in the desired expression host.

In another embodiment, a constitutive promoter may be selected to direct the expression of the desired polypeptide of the present invention. Such an expression construct may provide additional advantages since it circumvents the need to culture the expression hosts on a medium containing an inducing substrate.

Examples of strong constitutive and/or inducible promoters which are preferred for use in fungal expression hosts are those which are obtainable from the fungal genes for xylanase (*xln*A), phytase, ATP-synthetase, subunit 9 (*oli*C), triose phosphate isomerase (*tpi*), alcohol dehydrogenase (*Adh*A), α-amylase (*amy*), amyloglucosidase (AG - from the *glaA* gene), acetamidase (*amd*S) and glyceraldehyde-3-phosphate dehydrogenase (*gpd*) promoters.

Examples of strong yeast promoters are those obtainable from the genes for alcohol dehydrogenase, lactase, 3-phosphoglycerate kinase and triosephosphate isomerase.

Examples of strong bacterial promoters are the α-amylase and *SP02* promoters as well as promoters from extracellular protease genes.

Hybrid promoters may also be used to improve inducible regulation of the expression construct.

The promoter can additionally include features to ensure or to increase expression in a suitable host. For example, the features can be conserved regions such as a Pribnow Box or a TATA box. The promoter may even contain other sequences to affect (such as to maintain, enhance, decrease) the levels of expression of the nucleotide sequence of the present invention. For example, suitable other sequences include the Sh1-intron or an ADH intron. Other sequences include inducible elements - such as temperature, chemical, light or stress inducible elements. Also, suitable elements to enhance transcription or translation may be present. An example of the latter element is the TMV 5' signal sequence (see Sleat Gene 217 [1987] 217-225; and Dawson Plant Mol. Biol. 23 [1993] 97).

SECRETION

Often, it is desirable for the polypeptide of the present invention to be secreted from the expression host into the culture medium from where the polypeptide of the present invention may be more easily recovered. According to the present invention, the secretion leader sequence may be selected on the basis of the desired expression host. Hybrid signal sequences may also be used with the context of the present invention.

Typical examples of heterologous secretion leader sequences are those originating from the fungal amyloglucosidase (AG) gene (*glaA* - both 18 and 24 amino acid versions e.g. from *Aspergillus*), the α-factor gene (yeasts e.g. *Saccharomyces* and *Kluyveromyces*) or the α-amylase gene (*Bacillus*).

CONSTRUCTS

The term "construct" - which is synonymous with terms such as "conjugate", "cassette" and "hybrid" - includes the nucleotide sequence according to the present invention directly or indirectly attached to a promoter. An example of an indirect attachment is the provision of a suitable spacer group such as an intron sequence, such as the ShI-intron or the ADH intron, intermediate the promoter and the nucleotide sequence of the present invention. The same is true for the term "fused" in relation to the present invention which includes direct or indirect attachment. In each case, the terms do not cover the natural combination of the nucleotide sequence coding for the protein ordinarily associated with the wild type gene promoter and when they are both in their natural environment.
The construct may even contain or express a marker which allows for the selection of the genetic construct in, for example, a bacterium, preferably of the genus Bacillus, such as *Bacillus subtilis,* or plants, such as potatoes, sugar beet etc., into which it has been transferred. Various markers exist which may be used, such as for example those encoding mannose-6-phosphate isomerase (especially for plants) or those markers that provide for antibiotic resistance - e.g. resistance to G418, hygromycin, bleomycin, kanamycin and gentamycin.
Preferably the construct of the present invention comprises at least the nucleotide sequence of the present invention operably linked to a promoter.

VECTORS

The term "vector" includes expression vectors and transformation vectors and shuttle vectors.
The term "expression vector" means a construct capable of *in vivo* or *in vitro* expression.
The term "transformation vector" means a construct capable of being transferred from one entity to another entity - which may be of the species or may be of a different species. If the construct is capable of being transferred from one species to another - such as from an *E.coli* plasmid to a bacterium, such as of the genus Bacillus, then the transformation vector is sometimes called a "shuttle vector". It may even be a construct capable of being transferred from an *E. coli* plasmid to an Agrobacterium to a plant.
The vectors of the present invention may be transformed into a suitable host cell as described below to provide for expression of a polypeptide of the present invention. Thus, in a further aspect the invention provides a process for preparing polypeptides according to the present invention which comprises cultivating a host cell transformed or transfected with an expression vector as described above under conditions to provide for expression by the vector of a coding sequence encoding the polypeptides, and recovering the expressed polypeptides.
The vectors may be for example, plasmid, virus or phage vectors provided with an origin of replication, optionally a promoter for the expression of the said polynucleotide and optionally a regulator of the promoter.
The vectors of the present invention may contain one or more selectable marker genes. The most suitable selection systems for industrial micro-organisms are those formed by the group of selection markers which do not require a mutation in the host organism. Examples of fungal selection markers are the genes for acetamidase (*amd*S), ATP synthetase, subunit 9 (*oli*C), orotidine-5'-phosphate-decarboxylase (*pvr*A), phleomycin and benomyl resistance (benA). Examples of non-fungal selection markers are the bacterial G418 resistance gene (this may also be used in yeast, but not in filamentous fungi), the ampicillin resistance gene (*E. coli*), the neomycin resistance gene (*Bacillus*) and the *E.coli uid*A gene, coding for β-glucuronidase (GUS).
Vectors may be used *in vitro,* for example for the production of RNA or used to transfect or transform a host cell.
Thus, polynucleotides of the present invention can be incorporated into a recombinant vector (typically a replicable vector), for example a cloning or expression vector. The vector may be used to replicate the nucleic acid in a compatible host cell. Thus in a further embodiment, the invention provides a method of making polynucleotides of the present invention by introducing a polynucleotide of the present invention into a replicable vector, introducing the vector into a compatible host cell, and growing the host cell under conditions which bring about replication of the vector. The vector may be recovered from the host cell. Suitable host cells are described below in connection with expression vectors.
The present invention also relates to the use of genetically engineered host cells expressing a PDE11 or variant, homologue, fragment or derivative thereof in screening methods for the identification of inhibitors and antagonists of the PDE11 that would modulate phosphodiesterase activity thereby modulating cyclic nucleotide levels. Such genetically engineered host cells could be used to screen peptide libraries or organic molecules capable of modulating PDE11 activity. Antagonists and inhibitors of PDE11, such as antibodies, peptides or small organic molecules will provide the basis for pharmaceutical compositions for the treatment of diseases associated with, for example, male erectile dysfunction. Such inhibitors or antagonists can be administered alone or in combination with other therapeutics for the treatment of such diseases.

The present invention also relates to expression vectors and host cells comprising polynucleotide sequences encoding PDE11 or variant, homologue, fragment or derivative thereof for the *in vivo* or *in vitro* production of PDE11 protein or to screen for agents that can affect PDE11 expression or activity.

TISSUE

The term "tissue" as used herein includes tissue *per se* and organ.

HOST CELLS

The term "host cell" - in relation to the present invention includes any cell that could comprise the nucleotide sequence coding for the recombinant protein according to the present invention and/or products obtained therefrom, wherein a promoter can allow expression of the nucleotide sequence according to the present invention when present in the host cell.

Thus, a further embodiment of the present invention provides host cells transformed or transfected with a polynucleotide of the present invention. Preferably said polynucleotide is carried in a vector for the replication and expression of said polynucleotides. The cells will be chosen to be compatible with the said vector and may for example be prokaryotic (for example bacterial), fungal, yeast or plant cells.

The gram-negative bacterium *E. coli* is widely used as a host for heterologous gene expression. However, large amounts of heterologous protein tend to accumulate inside the cell. Subsequent purification of the desired protein from the bulk of *E.coli* intracellular proteins can sometimes be difficult.

In contrast to *E.coli,* bacteria from the genus *Bacillus* are very suitable as heterologous hosts because of their capability to secrete proteins into the culture medium. Other bacteria suitable as hosts are those from the genera *Streptomyces* and *Pseudomonas.*

Depending on the nature of the polynucleotide encoding the polypeptide of the present invention, and/or the desirability for further processing of the expressed protein, eukaryotic hosts such as yeasts or other fungi may be preferred. In general, yeast cells are preferred over fungal cells because they are easier to manipulate. However, some proteins are either poorly secreted from the yeast cell, or in some cases are not processed properly (e.g. hyperglycosylation in yeast). In these instances, a different fungal host organism should be selected.

Examples of suitable expression hosts within the scope of the present invention are fungi such as *Aspergillus* species (such as those described in EP-A-0184438 and EP-A-0284603) and *Trichoderma* species; bacteria such as *Bacillus* species (such as those described in EP-A-0134048 and EP-A-0253455), *Streptomyces* species and *Pseudomonas* species; and yeasts such as *Kluyveromyces* species (such as those described in EP-A-0096430 and EP-A-0301670) and *Saccharomyces* species. By way of example, typical expression hosts may be selected from *Aspergillus niger, Aspergillus niger var. tubigenis, Aspergillus niger var. awamori, Aspergillus aculeatis, Aspergillus nidulans, Aspergillus orvzae, Trichoderma reesei, Bacillus subtilis, Bacillus licheniformis, Bacillus amyloliquefaciens, Kluyveromyces lactis* and *Saccharomyces cerevisiae.*

The use of suitable host cells - such as yeast, fungal and plant host cells - may provide for post-translational modifications (e.g. myristoylation, glycosylation, truncation, lapidation and tyrosine, serine or threonine phosphorylation) as may be needed to confer optimal biological activity on recombinant expression products of the present invention.

ORGANISM

The term "organism" in relation to the present invention includes any organism that could comprise the nucleotide sequence coding for the recombinant protein according to the present invention and/or products obtained therefrom, wherein a promoter can allow expression of the nucleotide sequence according to the present invention when present in the organism. Examples of organisms may include a fungus, yeast or a plant.

The term "transgenic organism" in relation to the present invention includes any organism that comprises the nucleotide sequence coding for the protein according to the present invention and/or products obtained therefrom, wherein the promoter can allow expression of the nucleotide sequence according to the present invention within the organism. Preferably the nucleotide sequence is incorporated in the genome of the organism.

The term "transgenic organism" does not cover the native nucleotide coding sequence according to the present invention in its natural environment when it is under the control of its native promoter which is also in its natural environment. In addition, the present invention does not cover the native protein according to the present invention when it is in its natural environment and when it has been expressed by its native nucleotide coding sequence which is also in its natural environment and when that nucleotide sequence is under the control of its native promoter which is also in its natural environment.

Therefore, the transgenic organism of the present invention includes an organism comprising any one of, or combinations of, the nucleotide sequence coding for the amino acid sequence according to the present invention, constructs according to the present invention (including combinations thereof), vectors according to the present invention, plasmids according to the present invention, cells according to the present invention, tissues according to the present invention or the products thereof. The transformed cell or organism could prepare acceptable quantities of the desired compound which would be easily retrievable from, the cell or organism.

TRANSFORMATION OF HOST CELLS/HOST ORGANISMS

As indicated earlier, the host organism can be a prokaryotic or a eukaryotic organism. Examples of suitable prokaryotic hosts include *E. coli* and *Bacillus subtilis.* Teachings on the transformation of prokaryotic hosts is well documented in the art, for example see Sambrook et al (Molecular Cloning: A Laboratory Manual, 2nd edition, 1989, Cold Spring Harbor Laboratory Press) and Ausubel *et al.,* Current Protocols in Molecular Biology (1995), John Wiley & Sons, Inc.

If a prokaryotic host is used then the nucleotide sequence may need to be suitably modified before transformation - such as by removal of introns.

In another embodiment the transgenic organism can be a yeast. In this regard, yeast have also been widely used as a vehicle for heterologous gene expression. The species *Saccharomyces cerevisiae* has a long history of industrial use, including its use for heterologous gene expression. Expression of heterologous genes in Saccharomyces cerevisiae has been reviewed by Goodey et al (1987, Yeast Biotechnology, D R Berry et al, eds, pp 401-429, Allen and Unwin, London) and by King et al (1989, Molecular and Cell Biology of Yeasts, E F Walton and G T Yarronton, eds, pp 107-133, Blackie, Glasgow).

For several reasons *Saccharomyces cerevisiae* is well suited for heterologous gene expression. First, it is non-pathogenic to humans and it is incapable of producing certain endotoxins. Second, it has a long history of safe use following centuries of commercial exploitation for various purposes. This has led to wide public acceptability. Third, the extensive commercial use and research devoted to the organism has resulted in a wealth of knowledge about the genetics and physiology as well as large-scale fermentation characteristics of *Saccharomyces cerevisiae.*

A review of the principles of heterologous gene expression in Saccharomyces cerevisiae and secretion of gene products is given by E Hinchcliffe E Kenny (1993, "Yeast as a vehicle for the expression of heterologous genes", Yeasts, Vol 5, Anthony H Rose and J Stuart Harrison, eds, 2nd edition, Academic Press Ltd.).

Several types of yeast vectors are available, including integrative vectors, which require recombination with the host genome for their maintenance, and autonomously replicating plasmid vectors.

In order to prepare the transgenic Saccharomyces, expression constructs are prepared by inserting the nucleotide sequence of the present invention into a construct designed for expression in yeast. Several types of constructs used for heterologous expression have been developed. The constructs contain a promoter active in yeast fused to the nucleotide sequence of the present invention, usually a promoter of yeast origin, such as the GAL1 promoter, is used. Usually a signal sequence of yeast origin, such as the sequence encoding the SUC2 signal peptide, is used. A terminator active in yeast ends the expression system.

For the transformation of yeast several transformation protocols have been developed. For example, a transgenic Saccharomyces according to the present invention can be prepared by following the teachings of Hinnen et al (1978, Proceedings of the National Academy of Sciences of the USA 75, 1929); Beggs, J D (1978, Nature, London, 275, 104); and Ito, H et al (1983, J Bacteriology 153, 163-168).

The transformed yeast cells are selected using various selective markers. Among the markers used for transformation are a number of auxotrophic markers such as LEU2, HIS4 and TRP1, and dominant antibiotic resistance markers such as aminoglycoside antibiotic markers, eg G418.

Another host organism is a plant. The basic principle in the construction of genetically modified plants is to insert genetic information in the plant genome so as to obtain a stable maintenance of the inserted genetic material.

Several techniques exist for inserting the genetic information, the two main principles being direct introduction of the genetic information and introduction of the genetic information by use of a vector system. A review of the general techniques may be found in articles by Potrykus (Annu Rev Plant Physiol Plant Mol Biol [1991] 42:205-225) and Christou (Agro-Food-Industry Hi-Tech March/April 1994 17-27). Further teachings on plant transformation may be found in EP-A-0449375.

Thus, the present invention also provides a method of transforming a host cell with a nucleotide sequence shown as SEQ ID No. 1 or SEQ ID No. 3 or SEQ ID No. 14 or SEQ ID No. 16 or SEQ ID No. 18 or a derivative, homologue, variant or fragment thereof.

Host cells transformed with a PDE nucleotide coding sequence may be cultured under conditions suitable for the expression and recovery of the encoded protein from cell culture. The protein produced by a recombinant cell may be secreted or may be contained intracellularly depending on the sequence and/or the vector used. As will be understood by those of skill in the art, expression vectors containing PDE coding sequences can be designed with signal sequences which direct secretion of PDE coding sequences through a particular prokaryotic or eukaryotic cell membrane. Other recombinant constructions may join PDE coding sequence to nucleotide sequence encoding a polypeptide domain which will facilitate purification of soluble proteins (Kroll *DJ et al* (1993) DNA Cell Biol 12:441-53, see also above discussion of vectors containing fusion proteins).

PRODUCTION OF THE POLYPEPTIDE

According to the present invention, the production of the polypeptide of the present invention can be effected by the culturing of, for example, microbial expression hosts, which have been transformed with one or more polynucleotides of the present invention, in a conventional nutrient fermentation medium. The selection of the appropriate medium may be based on the choice of expression hosts and/or based on the regulatory requirements of the expression construct. Such media are well-known to those skilled in the art. The medium may, if desired, contain additional components favouring the transformed expression hosts over other potentially contaminating microorganisms.

Thus, the present invention also provides a method for producing a polypeptide having PDE11 activity, the method comprising the steps of a) transforming a host cell with a nucleotide sequence shown as SEQ ID No. 1 or SEQ ID No. 3 or SEQ ID No. 14 or SEQ ID No. 16 or SEQ ID No. 18 or a derivative, homologue, variant or fragment thereof; and b) culturing the transformed host cell under conditions suitable for the expression of said polypeptide.

The present invention also provides a method for producing a polypeptide having PDE11 activity, the method comprising the steps of a) culturing a host cell that has been transformed with a nucleotide sequence shown as SEQ ID No. 1 or SEQ ID No. 3 or SEQ ID No. 14 or SEQ ID No. 16 or SEQ ID No. 18 or a derivative, homologue, variant or fragment thereof under conditions suitable for the expression of said polypeptide; and b) recovering said polypeptide from the host cell culture.

The present invention also provides a method for producing a polypeptide having PDE11 activity, the method comprising the steps of a) transforming a host cell with a nucleotide sequence shown as SEQ ID No. 1 or SEQ ID No. 3 or SEQ ID No. 14 or SEQ ID No. 16 or SEQ ID No. 18 or a derivative, homologue, variant or fragment thereof; b) culturing the transformed host cell under conditions suitable for the expression of said polypeptide; and c) recovering said polypeptide from the host cell culture.

RIBOZYMES

Ribozymes are enzymatic RNA molecules capable of catalyzing the specific cleavage of RNA. The mechanism of ribozyme action involves sequence specific hybridization of the ribozyme molecule to complementary target RNA, followed by a endonucleolytic cleavage. Within the scope of the invention are engineered hammerhead motif ribozyme molecules that specifically and efficiently catalyze endonucleolytic cleavage of PDE RNA sequences.

Specific ribozyme cleavage sites within any potential RNA target are initially identified by scanning the target molecule for ribozyme cleavage sites which include the following sequences, GUA, GUU and GUC. Once identified, short RNA sequences of between 15 and 20 ribonucleotides corresponding to the region of the target gene containing the cleavage site may be evaluated for secondary structural features which may render the oligonucleotide sequence inoperable. The suitability of candidate targets may also be evaluated by testing accessibility to hybridization with complementary oligonucleotides using ribonuclease protection assays.

Both antisense RNA and DNA molecules and ribozymes of the invention may be prepared by any method known in the art for the synthesis of RNA molecules. These include techniques for chemically synthesizing oligonucleotides such as solid phase phosphoramidite chemical synthesis. Alternatively, RNA molecules may be generated by *in vitro* or *in vivo* transcription of DNA sequences encoding the antisense RNA molecule. Such DNA sequences may be incorporated into a wide variety of vectors with suitable RNA polymerase promoters such as T7 or SP6. Alternatively, antisense cDNA constructs that synthesize antisense RNA constitutively or inducibly can be introduced into cell lines, cells or tissues.

DETECTION

The presence of the PDE polynucleotide coding sequence can be detected by DNA-DNA or DNA-RNA hybridization or amplification using probes, portions or fragments of the sequence presented as SEQ ID No. 1 or SEQ ID No. 3 or SEQ ID No. 14 or SEQ ID No. 16 or SEQ ID No. 18. Nucleic acid amplification based assays involve the use of oligonucleotides or oligomers based on the PDE coding sequence to detect transformants containing PDE DNA or RNA. As used herein "oligonucleotides" or "oligomers" may refer to a nucleic acid sequence of at least about 10 nucleotides and as many as about 60 nucleotides, preferably about 15 to 30 nucleotides, and more preferably about 20-25 nucleotides which can be used as a probe or amplimer. Preferably, oligonucleotides are derived from the 3' region of the nucleotide sequence shown as SEQ ID No: 1 or SEQ ID No: 3 or SEQ ID No. 14 or SEQ ID No. 16 or SEQ ID No. 18.

A variety of protocols for detecting and measuring the expression of PDE polypeptide, such as by using either polyclonal or monoclonal antibodies specific for the protein, are known in the art. Examples include enzyme-linked immunosorbent assay (ELISA), radioimmunoassay (RIA) and fluorescent activated cell sorting (FACS). A two-site, monoclonal-based immunoassay utilizing monoclonal antibodies reactive to two noninterfering epitopes on PDE polypeptides is preferred, but a competitive binding assay may be employed. These and other assays are described, among other places, in Hampton R *et al* (1990, Serological Methods, A Laboratory Manual, APS Press, St Paul MN) and Maddox DE *et al* (1983, J Exp Med 15 8:121 1).

A wide variety of labels and conjugation techniques are known by those skilled in the art and can be used in various nucleic and amino acid assays. Means for producing labelled hybridization or PCR probes for detecting PDE polynucleotide sequences include oligolabelling, nick translation, end-labelling or PCR amplification using a labelled nucleotide. Alternatively, the PDE coding sequence, or any portion of it, may be cloned into a vector for the production of an mRNA probe. Such vectors are known in the art, are commercially available, and may be used to synthesize RNA probes *in vitro* by addition of an appropriate RNA polymerase such as T7, T3 or SP6 and labeled nucleotides.

A number of companies such as Pharmacia Biotech (Piscataway, NJ), Promega (Madison, WI), and US Biochemical Corp (Cleveland, OH) supply commercial kits and protocols for these procedures. Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US-A-3,817,837; US-A-3,850,752; US-A-3,939,350; US-A-3,996,345; US-A-4,277,437; US-A-4,275,149 and US-A-4,366,241. Also, recombinant immunoglobulins may be produced as shown in US-A-4,816,567.

Additional methods to quantitate the expression of a particular molecule include radiolabeling (Melby PC *et al* 1993 J Immunol Methods 159:235-44) or biotinylating (Duplaa C *et al* 1993 Anal Biochem 229-36) nucleotides, coamplification of a control nucleic acid, and standard curves onto which the experimental results are interpolated. Quantitation of multiple samples may be speeded up by running the assay in an ELISA format where the oligomer of interest is presented in various dilutions and a spectrophotometric or calorimetric response gives rapid quantitation.

Although the presence/absence of marker gene expression suggests that the gene of interest is also present, its presence and expression should be confirmed. For example, if the PDE coding sequence is inserted within a marker gene sequence, recombinant cells containing PDE coding regions can be identified by the absence of marker gene function. Alternatively, a marker gene can be placed in tandem with a PDE coding sequence under the control of a single promoter. Expression of the marker gene in response to induction or selection usually indicates expression of PDE as well.

Alternatively, host cells which contain the coding sequence for PDE and express PDE coding regions may be identified by a variety of procedures known to those of skill in the art. These procedures include, but are not limited to, DNA-DNA or DNA-RNA hybridization and protein bioassay or immunoassay techniques which include membrane-based, solution-based, or chip-based technologies for the detection and/or quantification of the nucleic acid or protein.

ANTIBODIES

The amino acid sequence of the present invention can also be used to generate antibodies - such as by use of standard techniques - against the amino acid sequence.

Procedures well known in the art may be used for the production of antibodies to PDE11 polypeptides. Such antibodies include, but are not limited to, polyclonal, monoclonal, chimeric, single chain, Fab fragments and fragments produced by a Fab expression library. Neutralizing antibodies, i.e., those which inhibit biological activity of PDE polypeptides, are especially preferred for diagnostics and therapeutics.

For the production of antibodies, various hosts including goats, rabbits, rats, mice, etc. may be immunized by injection with the inhibitor or any portion, variant, homologue, fragment or derivative thereof or oligopeptide which retains immunogenic properties. Depending on the host species, various adjuvants may be used to increase immunological response. Such adjuvants include, but are not limited to, Freund's, mineral gels such as aluminium hydroxide, and surface active substances such as lysolecithin, pluronic polyols, polyanions, peptides, oil emulsions, keyhole limpet hemocyanin, and dinitrophenol. BCG (*Bacilli Calmette-Guerin*) and *Corynebacterium parvum* are potentially useful human adjuvants which may be employed.

Monoclonal antibodies to the amino acid sequence may be even prepared using any technique which provides for the production of antibody molecules by continuous cell lines in culture. These include, but are not limited to, the hybridoma technique originally described by Koehler and Milstein (1975 Nature 256:495-497), the human B-cell hybridoma technique (Kosbor *et al* (1983) Immunol Today 4:72; Cote *et al* (1983) Proc Natl Acad Sci 80:2026-2030) and the EBV-hybridoma technique (Cole *et al* (1985) Monoclonal Antibodies and Cancer Therapy, Alan R Liss Inc, pp 77-96). In addition, techniques developed for the production of "chimeric antibodies", the splicing of mouse antibody genes to human antibody genes to obtain a molecule with appropriate antigen specificity and biological activity can be used (Morrison *et al* (1984) Proc Natl Acad Sci 81:6851-6855; Neuberger *et al* (1984) Nature 312:604-608; Takeda *et al* (1985) Nature 314:452-454). Alternatively, techniques described for the production of single chain antibodies (US-A-4946779) can be adapted to produce inhibitor specific single chain antibodies.

Antibodies may also be produced by inducing *in vivo* production in the lymphocyte population or by screening recombinant immunoglobulin libraries or panels of highly specific binding reagents as disclosed in Orlandi *et al* (1989, Proc Natl Acad Sci 86: 3833-3837), and Winter G and Milstein C (1991; Nature 349:293-299).

Antibody fragments which contain specific binding sites for PDE11 may also be generated. For example, such fragments include, but are not limited to, the $F(ab')_2$ fragments which can be produced by pepsin digestion of the antibody molecule and the Fab fragments which can be generated by reducing the disulfide bridges of the $F(ab')_2$ fragments. Alternatively, Fab expression libraries may be constructed to allow rapid and easy identification of monoclonal Fab fragments with the desired specificity (Huse WD *et al* (1989) Science 256:1275-128 1).

PDE11-specific antibodies are useful for the diagnosis of conditions and diseases associated with expression of PDE11 polypeptide. A variety of protocols for competitive binding or immunoradiometric assays using either polyclonal or monoclonal antibodies with established specificities are well known in the art. Such immunoassays typically involve the formation of complexes between PDE11 polypeptides and its specific antibody (or similar PDE11-binding molecule) and the measurement of complex formation. A two-site, monoclonal based immunoassay utilizing monoclonal antibodies reactive to two noninterfering epitopes on a specific PDE11 protein is preferred, but a competitive binding assay may also be employed. These assays are described in Maddox DE *et al* (1983, J Exp Med 158:121 1).

Anti-PDE11 antibodies are useful for the diagnosis of inflammation, conditions associated with proliferation of hematopoietic cells and HIV infection or other disorders or diseases characterized by abnormal expression of a PDE11. Diagnostic assays for a PDE11 include methods utilizing the antibody and a label to detect a PDE11 polypeptide in human body fluids, cells, tissues or sections or extracts of such tissues. The polypeptides and antibodies of the present invention may be used with or without modification. Frequently, the polypeptides and antibodies will be labeled by joining them, either covalently or noncovalently, with a reporter molecule. A wide variety of reporter molecules are known to those of skill in the art.

ASSAYS/IDENTIFICATION METHODS

The present invention also relates to an assay method for detecting the presence of PDE11 in cells (such as human cells) comprising: (a) performing a reverse transcriptase-polymerase chain reaction on RNA (such as total RNA) from such cells using a pair of polymerase chain reaction primers that are specific for PDE11, as determined from the DNA sequence of SEQ ID No. 1 or SEQ ID No. 3 or SEQ ID No. 14 or SEQ ID No. 16 or SEQ ID No. 18 or an allelic variation thereof; and (b) assaying the appearance of an appropriately sized PCR (polymerase chain reaction) fragment - such as by agarose gel electrophoresis.

The present invention also relates to a method of identifying agents (such as compounds, other substances or compositions comprising same) that affect (such as inhibit or otherwise modify) the activity of PDE11 and/or the expression thereof, the method comprising contacting PDE11 or the nucleotide sequence coding for same with the agent and then measuring the activity of PDE11 and/or the expression thereof.

The present invention also relates to a method of identifying agents (such as compounds, other substances or compositions comprising same) that selectively affect (such as inhibit or otherwise modify) the activity of PDE11 and/or the expression thereof, the method comprising contacting PDE11 or the nucleotide sequence coding for same with the agent and then measuring the activity of PDE11 and/or the expression thereof.

The present invention also relates to a method of identifying agents (such as compounds, other substances or compositions comprising same) that selectively affect (such as inhibit or otherwise modify) the activity of PDE11A1 or PDE11A2 or PDE11A3 or PDE11A4 and/or the expression thereof, the method comprising contacting the relevant PDE11 or the nucleotide sequence coding for same with the agent and then measuring the activity of the relevant PDE11 and/or the expression thereof.

The present invention also relates to a method of identifying agents (such as compounds, other substances or compositions comprising same) that affect (such as inhibit or otherwise modify) the activity of PDE11 and/or the expression thereof, the method comprising measuring the activity of PDE11 and/or the expression thereof in the presence of the agent or after the addition of the agent in: (a) a cell line into which has been incorporated recombinant DNA comprising the DNA sequence of SEQ ID No. 1 or SEQ ID No. 3 or SEQ ID No. 14 or SEQ ID No. 16 or SEQ ID No. 18 or an allelic variation thereof, or (b) a cell population or cell line that naturally selectively expresses PDE11. Preferably, the activity of PDE11 is determined by the assay method described above.

The present invention also relates to a method of identifying agents (such as compounds, other substances or compositions comprising same) that selectively affect (such as inhibit or otherwise modify) the activity of PDE11 and/or the expression thereof, the method comprising measuring the activity of PDE11 and/or the expression thereof in the presence of the agent or after the addition of the agent in: (a) a cell line into which has been incorporated recombinant DNA comprising the DNA sequence of SEQ ID No. 1 or SEQ ID No. 3 or SEQ ID No. 14 or SEQ ID No. 16 or SEQ ID No. 18 or an allelic variation thereof, or (b) a cell population or cell line that naturally selectively expresses PDE11. Preferably, the activity of PDE11 is determined by the assay method described above.

The present invention also relates to a method of identifying agents (such as compounds, other substances or compositions comprising same) that selectively affect (such as inhibit or otherwise modify) the activity of PDE11A1 or PDE11A2 or PDE11A3 or PDE11A4 and/or the expression thereof, the method comprising measuring the activity of the respective PDE11 and/or the expression thereof in the presence of the agent or after the addition of the agent in: (a) a cell line into which has been incorporated recombinant DNA comprising the respective DNA sequence of SEQ ID No. 1 or SEQ ID No. 3 or SEQ ID No. 14 or SEQ ID No. 16 or SEQ ID No. 18 or an allelic variation thereof, or (b) a cell population or cell line that naturally selectively expresses the respective PDE11. Preferably, the activity of PDE11 is determined by the assay method described above.

The present invention also provides a method of screening an agent for modulation (preferably for specific modulation) of PDE11 (or a derivative, homologue, variant or fragment thereof) activity or the expression of the nucleotide sequence coding for same (including a derivative, homologue, variant or fragment thereof), the method comprising the steps of: a) providing a candidate agent; b) combining PDE11 (or the derivative, homologue, variant or fragment thereof) or the nucleotide sequence coding for same (or the derivative, homologue, variant or fragment thereof) with the candidate agent for a time sufficient to allow modulation under suitable conditions; and c) detecting modulation of the candidate agent to PDE11 (or the derivative, homologue, variant or fragment thereof) or the nucleotide sequence coding for same (or the derivative, homologue, variant or fragment thereof) in order to ascertain if the candidate agent modulates PDE11 (or the derivative, homologue, variant or fragment thereof) activity or the expression of the nucleotide sequence coding for same (or the derivative, homologue, variant or fragment thereof).

The present invention also provides a method of screening an agent for specific binding affinity with PDE11 (or a derivative, homologue, variant or fragment thereof) or the nucleotide sequence coding for same (including a derivative, homologue, variant or fragment thereof), the method comprising the steps of: a) providing a candidate agent; b) combining PDE11 (or the derivative, homologue, variant or fragment thereof) or the nucleotide sequence coding for same (or the derivative, homologue, variant or fragment thereof) with the candidate agent for a time sufficient to allow binding under suitable conditions; and c) detecting binding of the candidate agent to PDE11 (or the derivative, homologue, variant or fragment thereof) or the nucleotide sequence coding for same (or the derivative, homologue, variant or fragment thereof) in order to ascertain if the candidate agent binds to PDE11 (or the derivative, homologue, variant or fragment thereof) or the nucleotide sequence coding for same (or the derivative, homologue, variant or fragment thereof).

The present invention also provides a method of identifying an agent which is capable of modulating PDE11, the method comprising the steps of: a) contacting the agent with PDE11 (or a derivative, homologue, variant or fragment thereof) or the nucleotide sequence coding for same (or the derivative, homologue, variant or fragment thereof), b) incubating the mixture of step a) with a cyclic nucleotide under conditions suitable for the hydrolysis of the cyclic nucleotide, c) measuring the amount of cyclic nucleotide hydrolysis, and d) comparing the amount of cyclic nucleotide hydrolysis of step c) with the amount of cyclic nucleotide hydrolysis obtained with PDE11 (or the derivative, homologue, variant or fragment thereof) or the nucleotide sequence coding for same (or the derivative, homologue, variant or fragment thereof) incubated without the compound, thereby determining whether the agent affects (such as stimulates or inhibits) cyclic nucleotide hydrolysis.

Thus, in certain embodiments of the present invention, PDE11 or a variant, homologue, fragment or derivative thereof and/or a cell line that expresses the PDE11 or variant, homologue, fragment or derivative thereof may be used to screen for antibodies, peptides, or other agent, such as organic or inorganic molecules, that act as modulators of phosphodiesterase activity or for the expression thereof, thereby identifying a therapeutic agent capable of modulating cyclic nucleotide levels. For example, anti-PDE11 antibodies capable of neutralizing the activity of PDE11 may be used to inhibit PDE11 hydrolysis of cyclic nucleotides, thereby increasing their levels. Alternatively, screening of peptide libraries or organic libraries made by combinatorial chemistry with recombinantly expressed PDE11 or a variant, homologue, fragment or derivative thereof or cell lines expressing PDE11 or a variant, homologue, fragment or derivative thereof may be useful for identification of therapeutic agents that function by modulating PDE11 hydrolysis of cyclic nucleotides. Synthetic compounds, natural products, and other sources of potentially biologically active materials can be screened in a number of ways deemed to be routine to those of skill in the art. For example, nucleotide sequences encoding the N-terminal region of PDE11 may be expressed in a cell line which can be used for screening of allosteric modulators, either agonists or antagonists, of PDE11 activity. Alternatively, nucleotide sequences encoding the conserved catalytic domain of PDE11 can be expressed in cell lines and used to screen for inhibitors of cyclic nucleotide hydrolysis.

The ability of a test agent to interfere with PDE11 activity or cyclic nucleotide hydrolysis may be determined by measuring PDE11 levels or cyclic nucleotide levels (as disclosed in Smith *et al* 1993 Appl. Biochem. Biotechnol. 41:189-218). There are also commercially available immunoassay kits for the measurement of cAMP and cGMP (eg Amersham International, Arlington Heights, IL and DuPont, Boston, MA). The activity of PDE11 may also be monitored by measuring other responses such as phosphorylation or dephosphorylation of other proteins using conventional techniques developed for these purpose.

Accordingly, the present invention provides a method of identifying a compound which is capable of modulating the cyclic nucleotide phosphodiesterase activity of a PDE11, or a variant, homologue, fragment or derivative thereof, comprising the steps of a) contacting the compound with a PDE11, or a variant, homologue, fragment or derivative thereof; b) incubating the mixture of step a) with a cyclic nucleotide under conditions suitable for the hydrolysis of the cyclic nucleotide; c) measuring the amount of cyclic nucleotide hydrolysis; and d) comparing the amount of cyclic nucleotide hydrolysis of step c) with the amount of cyclic nucleotide hydrolysis obtained with the PDE11, or a variant, homologue, fragment or derivative thereof, incubated without the compound, thereby determining whether the compound stimulates or inhibits cyclic nucleotide hydrolysis. In one embodiment of the method, the fragment may be from the N-terminal region of the PDE11 and provides a method to identify allosteric modulators of the PDE11. In another embodiment of the present invention, the fragment may be from the carboxy terminal region of the PDE11 and provides a method to identify inhibitors of cyclic nucleotide hydrolysis.

A PDE11 polypeptide, its immunogenic fragments or oligopeptides thereof can be used for screening therapeutic compounds in any of a variety of drug screening techniques. The polypeptide employed in such a test may be free in solution, affixed to a solid support, borne on a cell surface, or located intracellularly. The abolition of activity or the formation of binding complexes between a PDE11 polypeptide and the agent being tested may be measured.

Accordingly, the present invention provides a method for screening one or a plurality of compounds for modulation (preferably specific modulation, such as specific binding affinity) of PDE11 or the expression thereof, or a portion thereof or variant, homologue, fragment or derivative thereof, comprising providing one or a plurality of compounds; combining a PDE11 or a nucleotide sequence coding for same or a portion thereof or variant, homologue, fragment or derivative thereof with the or each of a plurality of compounds for a time sufficient to allow modulation under suitable conditions; and detecting binding of a PDE11, or portion thereof or variant, homologue, fragment or derivative thereof, to each of the plurality of compounds, thereby identifying the compound or compounds which modulate a PDE11 or a nucleotide sequence coding for same. In such an assay, the plurality of compounds may be produced by combinatorial chemistry techniques known to those of skill in the art.

Another technique for drug screening provides for high throughput screening of compounds having suitable binding affinity to the PDE11 polypeptides and is based upon the method described in detail in Geysen, European Patent Application 84/03564, published on September 13, 1984. In summary, large numbers of different small peptide test compounds are synthesized on a solid substrate, such as plastic pins or some other surface. The peptide test compounds are reacted with PDE11 fragments and washed. A bound PDE11 is then detected - such as by appropriately adapting methods well known in the art. A purified PDE11 can also be coated directly onto plates for use in the aforementioned drug screening techniques. Alternatively, non-neutralizing antibodies can be used to capture the peptide and immobilize it on a solid support.

This invention also contemplates the use of competitive drug screening assays in which neutralizing antibodies capable of binding a PDE11 specifically compete with a test compound for binding a PDE11. In this manner, the antibodies can be used to detect the presence of any peptide which shares one or more antigenic determinants with a PDE11.

AGENTS

The present invention also provides one or more agents indentified by the assays methods and identification methods of the present invention.

The agent of the present invention can be, for example, an organic compound or an inorganic compound. The agent can be, for example, a nucleotide sequence that is anti-sense to all or part of the sequence shown as SEQ ID No. 1 or SEQ ID No. 3 or SEQ ID No. 14 or SEQ ID No. 16 or SEQ ID No. 18.

The invention further provides an agent of the present invention (or even a pharmaceutically acceptable salt thereof, or a pharmaceutically acceptable solvate thereof) or a pharmaceutical composition containing any of the foregoing, for use as a medicament.

The present invention also provides the use of an agent to affect PDE11 activity (such as to inhibit, modulate or agonise) in the stratum of the brain.

DIAGNOSTICS

The present invention also provides a diagnostic composition for the detection of PDE11 polynucleotide sequences. The diagnostic composition may comprise the polynucleotide SEQ ID No. 1 or SEQ ID No. 3 or SEQ ID No. 14 or SEQ ID No. 16 or SEQ ID No. 18 or a variant, homologue, fragment or derivative thereof, or a sequence capable of hybridising to all or part of SEQ ID No. 1 or SEQ ID No. 3 or SEQ ID No. 14 or SEQ ID No. 16 or SEQ ID No. 18 or an allelic variation thereof.

In order to provide a basis for the diagnosis of disease, normal or standard values from a PDE11 polypeptide expression must be established. This is accomplished by combining body fluids or cell extracts taken from normal subjects, either animal or human, with antibody to a PDE11 polypeptide under conditions suitable for complex formation which are well known in the art. The amount of standard complex formation may be quantified by comparing it to a dilution series of positive controls where a known amount of antibody is combined with known concentrations of a purified PDE11 polypeptide. Then, standard values obtained from normal samples may be compared with values obtained from samples from subjects potentially affected by a disorder or disease related to a PDE11 polypeptide expression. Deviation between standard and subject values establishes the presence of the disease state.

A PDE11 polynucleotide, or any part thereof, may provide the basis for a diagnostic and/or a therapeutic compound. For diagnostic purposes, PDE11 polynucleotide sequences may be used to detect and quantitate gene expression in conditions, disorders or diseases in which PDE11 activity may be implicated, for example, in male erectile dysfunction.

A PDE11 encoding polynucleotide sequence may be used for the diagnosis of diseases resulting from expression of PDE11. For example, polynucleotide sequences encoding PDE11 may be used in hybridization or PCR assays of tissues from biopsies or autopsies or biological fluids, such as serum, synovial fluid or tumor biopsy, to detect abnormalities in PDE11 expression. The form of such qualitative or quantitative methods may include Southern or northern analysis, dot blot or other membrane-based technologies; PCR technologies; dip stick, pin or chip technologies; and ELISA or other multiple sample formal technologies. All of these techniques are well known in the art and are in fact the basis of many commercially available diagnostic kits.

Such assays may be tailored to evaluate the efficacy of a particular therapeutic treatment regime and may be used in animal studies, in clinical trials, or in monitoring the treatment of an individual patient. In order to provide a basis for the diagnosis of disease, a normal or standard profile for PDE expression must be established. This is accomplished by combining body fluids or cell extracts taken from normal subjects, either animal or human, with PDE11 or a portion thereof, under conditions suitable for hybridization or amplification. Standard hybridization may be quantified by comparing the values obtained for normal subjects with a dilution series of positive controls run in the same experiment where a known amount of purified PDE11 is used. Standard values obtained from normal samples may be compared with values obtained from samples from subjects potentially affected by a disorder or disease related to expression of the PDE coding sequence. Deviation between standard and subject values establishes the presence of the disease state. If disease is established, an existing therapeutic agent is administered, and treatment profile or values may be generated. Finally, the assay may be repeated on a regular basis to evaluate whether the values progress toward or return to the normal or standard pattern. Successive treatment profiles may be used to show the efficacy of treatment over a period of several days or several months.

Thus, the present invention relates to the use of a PDE11 polypeptide, or variant, homologue, fragment or derivative thereof, to produce anti-PDE11 antibodies which can, for example, be used diagnostically to detect and quantitate PDE11 levels in disease states.

The present invention further provides diagnostic assays and kits for the detection of PDE11 in cells and tissues comprising a purified PDE11 which may be used as a positive control, and anti-PDE11 antibodies. Such antibodies may be used in solution-based, membrane-based, or tissue-based technologies to detect any disease state or condition related to the expression of PDE11 protein or expression of deletions or a variant, homologue, fragment or derivative thereof.

PROBES

Another aspect of the subject invention is the provision of nucleic acid hybridization or PCR probes which are capable of detecting polynucleotide sequences, including genomic sequences, encoding PDE coding region or closely related molecules, such as alleles. The specificity of the probe, i.e., whether it is derived from a highly conserved, conserved or non-conserved region or domain, and the stringency of the hybridization or amplification (high, intermediate or low) will determine whether the probe identifies only naturally occurring PDE coding sequence, or related sequences. Probes for the detection of related nucleic acid sequences are selected from conserved or highly conserved nucleotide regions of cyclic nucleotide PDE family members, such as the 3' region, and such probes may be used in a pool of degenerate probes. For the detection of identical nucleic acid sequences, or where maximum specificity is desired, nucleic acid probes are selected from the non-conserved nucleotide regions or unique regions of PDE polynucleotides. As used herein, the term "non-conserved nucleotide region" refers to a nucleotide region that is unique to the PDE coding sequence disclosed herein and does not occur in related family members, such as known cyclic nucleotide PDEs.

PCR as described in US-A-4,683,195; US-A-4,800,195; and US-A-4,965,188 provides additional uses for oligonucleotides based upon the PDE11 sequence. Such oligomers are generally chemically synthesized, but they may be generated enzymatically or produced from a recombinant source. Oligomers generally comprise two nucleotide sequences, one with sense orientation (5'->3') and one with antisense (3'<-5') employed under optimized conditions for identification of a specific gene or condition. The same two oligomers, nested sets of oligomers, or even a degenerate pool of oligomcrs may be employed under less stringent conditions for detection and/or quantitation of closely related DNA or RNA sequences.

The nucleic acid sequence for PDE11 can also be used to generate hybridization probes as previously described, for mapping the endogenous genomic sequence. The sequence may be mapped to a particular chromosome or to a specific region of the chromosome using well known techniques. These include *in situ* hybridization to chromosomal spreads (Verma *et al* (1988) Human Chromosomes: A Manual of Basic Techniques, Pergamon Press, New York City), flow-sorted chromosomal preparations, or artificial chromosome constructions such as YACs, bacterial artificial chromosomes (BACs), bacterial PI constructions or single chromosome cDNA libraries.

*In situ* hybridization of chromosomal preparations and physical mapping techniques such as linkage analysis using established chromosomal markers are invaluable in extending genetic maps. Examples of genetic maps can be found in Science (1995; 270:410f and 1994; 265:1981f). Often the placement of a gene on the chromosome of another mammalian species may reveal associated markers even if the number or arm of a particular human chromosome is not known. New sequences can be assigned to chromosomal arms, or parts thereof, by physical mapping. This provides valuable information to investigators searching for disease genes using positional cloning or other gene discovery techniques. Once a disease or syndrome, such as ataxia telangiectasia (AT), has been crudely localized by genetic linkage to a particular genomic region, for example, AT to 1lq22-23 (Gatti *et al* (1988) Nature 336:577-580), any sequences mapping to that area may represent associated or regulatory genes for further investigation. The nucleotide sequence of the subject invention may also be used to detect differences in the chromosomal location due to translocation, inversion, etc between normal, carrier or affected individuals.

PHARMACEUTICALS

The present invention also provides a pharmaceutical composition for treating an individual in need of same due to PDE11 activity, the composition comprising a therapeutically effective amount of an agent that affects (such as inhibits) said activity and a pharmaceutically acceptable carrier, diluent, excipient or adjuvant.

Thus, the present invention also covers pharmaceutical compositions comprising the agents of the present invention (an agent capable of modulating the expression pattern of the nucleotide sequence of the present invention or the activity of the expression product thereof and/or an agent identified by an assay according to the present invention). In this regard, and in particular for human therapy, even though the agents of the present invention can be administered alone, they will generally be administered in admixture with a pharmaceutical carrier, excipient or diluent selected with regard to the intended route of administration and standard pharmaceutical practice.

By way of example, in the pharmaceutical compositions of the present invention, the agents of the present invention may be admixed with any suitable binder(s), lubricant(s), suspending agent(s), coating agent(s), or solubilising agent(s).

In general, a therapeutically effective daily oral or intravenous dose of the agents of the present invention is likely to range from 0.01 to 50 mg/kg body weight of the subject to be treated, preferably 0.1 to 20 mg/kg. The agents of the present invention may also be administered by intravenous infusion, at a dose which is likely to range from 0.001-10 mg/kg/hr.

Tablets or capsules of the agents may be administered singly or two or more at a time, as appropriate. It is also possible to administer the agents of the present invention in sustained release formulations.

Thus, the present invention also provides a method of treating an individual in need of same due to PDE11 activity comprising administering to said individual an effective amount of the pharmaceutical composition of the present invention.

Typically, the physician will determine the actual dosage which will be most suitable for an individual patient and it will vary with the age, weight and response of the particular patient. The above dosages are exemplary of the average case. There can, of course, be individual instances where higher or lower dosage ranges are merited, and such are within the scope of this invention.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intracavernosally, intravenously, intramuscularly or subcutaneously.

For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

For some applications, preferably the compositions are administered orally in the form of tablets containing excipients such as starch or lactose, or in capsules or ovules either alone or in admixture with excipients, or in the form of elixirs, solutions or suspensions containing flavouring or colouring agents.

For parenteral administration, the compositions are best used in the form of a sterile aqueous solution which may contain other substances, for example enough salts or monosaccharides to make the solution isotonic with blood.

For buccal or sublingual administration the compositions may be administered in the form of tablets or lozenges which can be formulated in a conventional manner.

For oral, parenteral, buccal and sublingual administration to subjects (such as patients), the daily dosage level of the agents of the present invention may typically be from 10 to 500 mg (in single or divided doses). Thus, and by way of example, tablets or capsules may contain from 5 to 100 mg of active agent for administration singly, or two or more at a time, as appropriate. As indicated above, the physician will determine the actual dosage which will be most suitable for an individual patient and it will vary with the age, weight and response of the particular patient. It is to be noted that whilst the above-mentioned dosages are exemplary of the average case there can, of course, be individual instances where higher or lower dosage ranges are merited and such dose ranges are within the scope of this invention.

Generally, in humans, oral administration of the agents of the present invention is the preferred route, being the most convenient and, for example in male erectile dysfunction (MED), avoiding the well-known disadvantages associated with intracavernosal (i.c.) administration. A preferred oral dosing regimen in MED for a typical man is from 25 to 100 mg of agent when required. In circumstances where the recipient suffers from a swallowing disorder or from impairment of drug absorption after oral administration, the drug may be administered parenterally, e.g. sublingually or buccally.

For veterinary use, the agent of the present invention is typically administered as a suitably acceptable formulation in accordance with normal veterinary practice and the veterinary surgeon will determine the dosing regimen and route of administration which will be most appropriate for a particular animal. However, as with human treatment, it may be possible to administer the agent alone for veterinary treatments.

Typically, the pharmaceutical compositions - which may be for human or animal usage - will comprise any one or more of a pharmaceutically acceptable diluent, carrier, excipient or adjuvant. The choice of pharmaceutical carrier, excipient or diluent can be selected with regard to the intended route of administration and standard pharmaceutical practice. As indicated above, the pharmaceutical compositions may comprise as - or in addition to - the carrier, excipient or diluent any suitable binder(s), lubricant(s), suspending agent(s), coating agent(s), solubilising agent(s).

In some embodiments of the present invention, the pharmaceutical compositions will comprise one or more of: an agent that has been screened by an assay of the present invention; an agent that is capable of interacting with any one or more of SEQ ID No. 1, SEQ ID No 2, SEQ ID No. 3 or SEQ ID No. 4 or SEQ ID No. 14 or SEQ ID No. 15 or SEQ ID No. 16 or SEQ ID No. 17 or SEQ ID No. 18 or SEQ ID No. 19 including derivatives, fragments, homologues or variants thereof or sequences capable of hybridising to SEQ ID No. 1 or SEQ ID No. 3 or SEQ ID No. 14 or SEQ ID No. 16 or SEQ ID No. 18.

Included in the scope of the invention are oligonucleotide sequences, antisense RNA and DNA molecules and ribozymes, which function to destabilize PDE11 mRNA or inhibit translation of a PDE11. Such nucleotide sequences may be used in conditions where it would be preferable to increase cyclic nucleotide levels, such as in inflammation.

A PDE11 antisense molecule may provide the basis for treatment of various abnormal conditions related to, for example, increased PDE11 activity - such as male erectile dysfunction.

A PDE11 nucleic acid antisense molecule may be used to block the activity of the PDE11 in conditions where it would be preferable to elevate cyclic nucleotide levels.

Expression vectors derived from retroviruses, adenovirus, herpes or vaccinia viruses, or from various bacterial plasmids, may be used for delivery of recombinant PDE11 sense or antisense molecules to the targeted cell population. Methods which are well known to those skilled in the art can be used to construct recombinant vectors containing PDE11. Alternatively, recombinant PDE11 can be delivered to target cells in liposomes.

The full length cDNA sequence and/or its regulatory elements enable researchers to use PDE11 as a tool in sense (Youssoufian H and HF Lodish 1993 Mol Cell Biol 13:98-104) or antisense (Eguchi *et al* (1991) Annu Rev Biochem 60:631-652) investigations of gene function. Oligonucleotides, designed from the cDNA or control sequences obtained from the genomic DNA can be used *in vitro* or *in vivo* to inhibit expression. Such technology is now well known in the art, and sense or antisense oligonucleotides or larger fragments can be designed from various locations along the coding or control regions. Appropriate oligonucleotides, which can be 20 nucleotides in length, may be used to isolate PDE 11 sequences or closely related molecules from human libraries.

Additionally, PDE11 expression can be modulated by transfecting a cell or tissue with expression vectors which express high levels of a PDE11 fragment in conditions where it would be preferable to block phosphodiesterase activity thereby increasing cyclic nucleotide levels. Such constructs can flood cells with untranslatable sense or antisense sequences. Even in the absence of integration into the DNA, such vectors may continue to transcribe RNA molecules until all copies of the vector are disabled by endogenous nucleases. Such transient expression may last for a month or more with a non-replicating vector and even longer if appropriate replication elements are part of the vector system.

Modifications of gene expression can be obtained by designing antisense sequences to the control regions of the PDE gene, such as the promoters, enhancers, and introns.

Oligonucleotides derived from the transcription initiation site, e.g., between -10 and +10 regions of the leader sequence, are preferred. Antisense RNA and DNA molecules may also be designed to block translation of mRNA by preventing the transcript from binding to ribosomes. Similarly, inhibition can be achieved using Hogeboom base-pairing methodology, also known as "triple helix" base pairing. Triple helix pairing compromises the ability of the double helix to open sufficiently for the binding of polymerases, transcription factors, or regulatory molecules.

Thus the invention provides a pharmaceutical composition comprising an agent of the present invention (or even a pharmaceutically acceptable salt thereof, or a pharmaceutically acceptable solvate thereof) together with a pharmaceutically acceptable diluent, excipient or carrier.

The pharmaceutical composition could be for veterinary (i.e. animal) usuage or for human usuage.

Thus, the present invention therefore also relates to pharmaceutical compositions comprising effective amounts of inhibitors or antagonists of PDE11 protein (including anti-sense nucleic acid sequences) in admixture with a pharmaceutically acceptable diluent, carrier, excipient or adjuvant (including combinations thereof).

The present invention relates to pharmaceutical compositions which may comprise all or portions of PDE11 polynucleotide sequences, PDE11 antisense molecules, PDE11 polypeptides, protein, peptide or organic modulators of PDE11 bioactivity, such as inhibitors, antagonists (including antibodies) or agonists, alone or in combination with at least one other agent, such as stabilizing compound, and may be administered in any sterile, biocompatible pharmaceutical carrier, including, but not limited to, saline, buffered saline, dextrose, and water.

<u>SUMMARY</u>

In summary the present invention provides *inter alia:*

1. Novel amino acids.
2. Novel nucleotide sequences.
3. Assays using said novel sequences.
4. Compounds/compositions identified by use of said assays.
5. Expression systems comprising or expressing said novel sequences.
6. Methods of treatment based on said novel sequences.
7. Pharmaceutical compositions based on said novel sequences.

<u>DEPOSITS</u>

The following samples were deposited in accordance with the Budapest Treaty at the recognised depositary The National Collections of Industrial and Marine Bacteria Limited (NCIMB) at 23 St. Machar Drive, Aberdeen, Scotland, United Kingdom, AB2 1RY on 6 March 1998:

| | |
|---|---|
| *E. coli* pPDE11A1 | NCIMB number NCIMB 40925 |
| *E. coli* pPDE11A2 | NCIMB number NCIMB 40926 |

NCIMB 40925 comprises HSPDE11A1.
NCIMB 40926 comprises HSPDE11A2.

The following sample was deposited in accordance with the Budapest Treaty at the recognised depositary The National Collections of Industrial and Marine Bacteria Limited (NCIMB) at 23 St. Machar Drive, Aberdeen, Scotland, United Kingdom, AB2 1RY on 9 February 1999:

| | |
|---|---|
| *E. coli* pMmPDE11A3 | NCIMB number NCIMB 41007 |

NCIMB 41007 comprises MmPDE11A3.

The present invention also encompasses sequences derivable and/or expressable from those deposits and embodiments comprising the same. The present invention also encompasses partial sequences derivable and/or expressable from those deposits and embodiments comprising the same, wherein those partial sequences code for active enzymatic sites. The present invention also encompasses proteins comprising sequences derivable and/or expressable from those deposits and embodiments comprising the same. The present invention also encompasses proteins comprising partial sequences derivable and/or expressable from those deposits and embodiments comprising the same, wherein those partial sequences code for active enzymatic sites.
The present invention also encompasses sequences derivable and/or expressable from those deposits and embodiments comprising the same.

INTRODUCTION TO THE EXAMPLES SECTION AND THE FIGURES

The present invention will now be described, by way of example only, with reference to the accompanying drawings in which:-

Figure 1 which presents a schematic diagram;

Figure 2 which presents a Northen blot photographic image;

Figure 3 which presents a schematic diagram;

Figure 4 which presents a nucleotide sequence and an amino acid sequence;

Figure 5 which presents a nucleotide sequence, an amino acid sequence and an alignment of protein sequences;

Figure 6 which presents a graph;

Figure 7 which presents Hanes Plots;

Figure 8 which presents photomicrographs;

Figure 9 which presents a photograph;

Figure 10 which presents a sequence alignment; and

Figure 11 which presents a sequence alignment.

In slightly more detail, Figure 1 is a schematic alignment of bovine PDE5, IMAGE clone 298975 and PDE11A1 illustrating the various relevant functional domains shared by PDE11A1 with other phosphodiesterases and the region encompassed by the partial PDE11A1 sequence present in the IMAGE clone 298975.
In slightly more detail, Figure 2 is a Northern blot illustrating the tissue distribution of expression of PDE11A.
In slightly more detail, Figure 2 is a schematic diagram illustrating the strategy used to clone a full length cDNA for PDE11A1, bars are not to scale, CN refers to caudate nucleus.
In slightly more detail, Figure 4A is SEQ ID No.1 which is the nucleotide sequence coding for PDE11A1, ATG translation initiation indicated by double underline.
In slightly more detail, Figure 4B is SEQ ID No.2 which is the protein sequence for PDE11A1.
In slightly more detail, Figure 5A is SEQ ID No.3 which is the nucleotide sequence coding for PDE11A2, ATG translation initiation codon indicated by double underline.
In slightly more detail, Figure 5B is SEQ ID No.4 which is the protein sequence for PDE11A2.
In slightly more detail, Figure 5C is an alignment of the amino termini of PDE11A1 and PDE11A2 illustrating the region of divergence between the 2 splice variants.
In slightly more detail, Figure 6 is a graph showing relative hydrolytic activity of PDE11 against cAMP or cGMP in either PDE11 infected or mock infected *Sf*9 cells.
In slightly more detail, Figure 7 presents Hanes Plots used to calculate Km and Vmax values for PDE11A1 against either cAMP or cGMP.
In slightly more detail, Figure 8 presents *in situ* analysis of PDE11 expression photomicrographs *in situ* hybridisation analysis of various tissues showing expression of PDE11. In more detail, Figure 8 is a photomicrograph showing expression of PDE11 in a variety of tissues, on the left are photomicrographs taken under light field and on the right photomicrographs taken under dark field to allow detection of the silver grains around sites of expression. Panel A illustrates expression of PDE11 in a subset of epithelial cells within the prostate, area 1 expression of PDE11 in a prostatic duct and area 2 a prostatic duct where expression is absent. Panel B illustrates expression of PDE11 in the striatum region of the human brain. Expression is clearly limited to the neuronal nuclei present in grey matter (area 1) whereas white matter (area2) is devoid of PDE11 expression. Panel C shows expression of PDE11 in ganglia cells of the eosophageal sphincter. Panel D illustrates expression of PDE11 in a subset of endothelial cells within vessels of the corpus cavernosum. Arrowheads serve to illustrate precise areas of expression.

In slightly more detail, Figure 9 presents a photograph demonstrating that PDE11 antibodies can detect the PDE11 protein. Crude lysates from insect cells either infected with the PDE11 baculovirus or from uninfected cells were electrophoresed by PAGE. The proteins on the gel were then blotted onto nitro-cellulose membrane. This blot was used to study the relative specificity of the two PDE11 antibodies. Ab-1 was derived from peptide SEQ ID No. 8 and Ab-2 was derived from peptide ID SEQ No. 9.

## EXAMPLES

## EXPERIMENTAL SECTION

### Materials and Methods

### Northern Hybridisation and Probe preparation

Northern blots, obtained from Clontech (Clontech Laboratories, 1020 East Meadow Circle, Palo Alto, California, 94303, USA), were prehybridised for 1 hour in Expresshyb hybridisation solution (Clontech - Clontech Laboratories, 1020 East Meadow Circle, Palo Alto, California, 94303, USA) at 55°C before a radiolabelled PDE11A1 fragment (DNA was labelled using the Megaprime random labelling system (Amersham (Amersham place, Little Chalfont, Bucks, HP7 9NA UK}) strictly following the manufacturers instructions with 50µCi of $^{32}$P-dATP) was added to fresh Expresshyb and hybridised to the blot overnight at 55"C, with gentle shaking. Blots were then washed 3X at room temperature for 10 minutes each in 2XSSC (150mM NaCl, 30mM Na.citrate) followed by 2 washes in 0.2XSSC (15mM NaCl, 3mM Nacitrate) at 55°C for 20 minutes each. Blots were then exposed to autoradiographic film.

### Polymerase Chain Reactions (PCR)

PCRs were performed using standard reagents and conditions. Briefly, all reaction buffers and enzymes were obtained in kit format from either Clontech (Clontech Laboratories, 1020 East Meadow Circle, Palo Alto, California, 94303, USA) (for rapid amplification of cDNA ends (RACE) reactions) or from Life Technologies (3 Fountain Drive, Inchinnan Business Park, Paisley, PA4 9RF UK) for standard PCR. Oligonucleotides were obtained from a commercial supplier (OSWEL DNA services, Lab 5005, Medical And Biological Sciences Building, University of Southampton, Bolderwood, Bassett Crescent East, Southampton, SO16 7PX UK) and used at a concentration of 400nM. Reactions were performed on a MJ Research PTC-200 thermal cycler, using cycling parameters as recommended by the manufacturer of the kit being used.

### Cloning of PCR products

PCR derived DNA fragments were cloned using the TOPO cloning system (Cat No. K3001-0-1) supplied by Invitrogen (De Schelp 12, 9351 NV Leek, The Netherlands), following the methods as outlined in the manufacturers method book and using the reagents as supplied

### Phosphodiesterase assays on crude insect cell lysates

Crude lysates were assayed for phosphodiesterase activity using [$^3$H] -cGMP or [$^3$H] - cAMP (Amersham - Amersham place, Little Chalfont, Bucks, HP7 9NA UK). To 25µl of crude lysate was added 15µl of buffer C (20mM Tris.HCL (pH 7.4), 5mM $MgCl_2.6H_20$) and 25 µl of buffer D (Buffer C + BSA @ 2mg/ml). The reaction was then initiated with 50µl of substrate and the tube incubated with shaking at 30"C for 15 minutes. The tubes were then transferred to a boiling water bath for 2 minutes then onto ice for 10 minutes. 25µl of a 1.5mg/ml aqueous solution of snake venom (*Ophiophagus hannah,* Sigma {Sigma-Aldrich Company Limited, Fancy Road, Poole, Dorset, BH12 4QH UK}, V-0376) was then added mixed and returned to 30°C for a further 10 minutes with shaking. Tubes were then returned to ice for 5 minutes when 500µl of resin slurry was added, thoroughly mixed and allowed to stand on ice for a further 15 minutes. Tubes were then centrifuged for 10 minutes at 2000 rpm after which 150µl of supernatant was removed and added to 2ml of Starscint (Packhard), mixed and subjected to liquid scintillation counting.

Activity of phosphodiesterases present are expressed in pmol/min/ml fraction by means of the following formula:

$$\frac{\text{DPM of sample-DPM of blanks}}{\text{mean DPM of totals-mean DPM of background}} \text{x} \frac{625}{150} \text{x} \frac{\text{50pmols}}{\text{10 mins}} \text{x100}$$

Active crude lysates were then flash frozen in a dry ice ethanol bath and stored at -80°C.

**Purification of Protein from crude lysate**

Crude *Sf*9 lysates containing the expressed protein-FLAG fusion were passed down a FLAG antibody affinity column (Agarose M2 affinity gel beads to which a purified IgG1 monoclonal anti-FLAG antibody had been conjugated by hydrazide linkage) using a Pharmacia FPLC system (LKB.UV-MII) {Pharmacia Biotech, 23 Grosvenor Road, St Albans, Herts, AL1 3AW. UK}. Purified protein was eluted under conditions exactly as specified by the manufacturer of the affinity beads, split into aliquots and stored at -80°C for subsequent analysis.

**Phosphodiesterase assays on affinity purified protein**

Substrate was prepared by adding 14.7µl [$^3$H]-cGMP (Amersham - Amersham place, Little Chalfont, Bucks, HP7 9NA UK) to 20µl Buffer C (as above). In 96 well format 25µl of Buffer D (see above) was added to all wells. 25µl sample was added to all wells followed by 50µl of substrate to initiate the reaction. Samples were incubated for 30 minutes at 30°C before addition of 50µl SPA beads, these were left for 20 minutes before being read on a scintillation counter.

**Western Blotting**

Insect cells were pelleted by centrifugation and resuspended in lysis buffer (50mM Tris.HCL (pH 6.8), 100mM dithiothreitol, 2% SDS, 0.2% bromophenol blue, 20% glycerol), sonicated, placed in a boiling waterbath for 10 minutes and electrophoresed under reducing conditions on SDS PAGE (Novex NuPAGE 4-12% Bis-TRIS reducing gel, Kit NP0000). The proteins were then transferred to a nitro-cellulose membrane using the Novex NuPAGE western transfer system. The western blot was then incubated in PBS/0.1% Tween20/5% dried milk and 4°C overnight. The blot was then incubated for 1 hour in PBS/0.1% Tween20/5% dried milk/1/500 dilution of the primary antibody. Washes were then performed with PBS/Tween20 for 3X 1 minute and 1X 10 minutes prior to a wash in PBS/Tween20/5% dried milk 3X 1 minute and IX 10 minutes. The secondary antibody (Goat anti-rabbit IgG (H+L)- alkaline phosphatase conjugate (Bio-Rad 1706518) was then added to this same solution and incubated for 45 minutes at room temperature with shaking. The blot was then washed 3X 1 minute and 1 X 10 minutes in PBS/Tween20 before detection using 5-bromo-4-chloro-3-indolyl phosphate and Nitro Blue Tetrazolium (BCIP/NBT, Sigma B1911 (Sigma-Aldrich Company Limited, Fancy Road, Poole, Dorset, BH12 4QH UK}).

**Example 1**

**The identification of PDE11A1**

The clone (IMAGE clone id 298975) was obtained from Research Genetics (2130, Memorial Pkwy, SW Huntsville, AL 358801, USA) as a stab culture in L-agar. Bacteria from the stab culture were streaked onto a 37mm L-agar plate in the presence of ampicillin at a concentration of 100mg/ml. After overnight growth at 37°C a single clone was picked, using sterile technique, into 5ml of LB-broth containing ampicillin at a concentration of 100mg/ml and grown at 37°C overnight with shaking (220 rpm). Plasmid DNA was isolated from the bacteria using a standard commercially available miniprep kit (Qiagen™) and strictly following the manufacturers instructions. The isolated DNA was then subjected to full length sequencing, using standard kits and reagents and an ABI (PE Applied Biosystems Incorporated) automated sequencer.

Full length sequencing revealed that IMAGE clone 298975 contains a nucleotide insert of 1779 bp. Database homology searches using the BLAST (Basic Local Alignment Search Tool (Altshul SF (1993) J.Mol. Evol. 36:290-300; Altshul, SF et al (1990) J. Mol. Biol. 215:403-410) were performed and showed that the DNA insert in IMAGE clone 298975 contained the sequence present in the EST (expressed sequence(expressed sequence tag) database, that this sequence was homologous to known PDEs, and that 298975 also contained extensive stretches of additional DNA sequence, 5' to the EST, which are also homologous to known PDEs. Further analysis of this sequence was performed to determine the presence of any open reading frames, ATG translation initiation codons and TGA, TAA, TAG terminator codons. This revealed that clone 298975 contained a putative ORF (open reading frame) extending from an ATG initiator at base pair position + 13 all the way through to the 3' limit of the sequence. There was no indication of a terminator codon at the 3' end of the clone suggesting the cDNA was not complete at the 3' end.

**IMAGE clone 298975 contains a fragment of a potential novel class of PDE**

Detailed bioinformatic analysis comparing the sequence of the insert of 298975 and known PDEs indicate that the sequence of 298975 may encode a fragment of a member of a novel family of PDEs. The reason for this is the relative lack of similarity between 298975 and other PDEs compared to the relative similarity between, for instance, PDE6A and PDE6B. The similarity between 298975 and other PDEs is more comparable to the similarity between, for example PDE5 and PDE6A. This is illustrated in TABLE 1 (shown below) which illustrates the percentage similarity of the peptide sequence between 298975, PDE 6A, PDE6B and PDE5 over the region for which sequence from 298975 was available (see also below).

TABLE 1

| | PDE6A | PDE6B | PDE5 | PDE11 |
|---|---|---|---|---|
| PDE6A | 100 | 77.7 | 39.7 | 34.1 |
| PDE6B | | 100 | 41.1 | 34.1 |
| PDE5 | | | 100 | 35.5 |
| PDE11 | | | | 100 |

Table 1 illustrates the percentage similarity between PDE11 and other PDE gene family members and shows that PDE11 is equally distant to PDE5, PDE6A and PDE6B, in contrast to the PDE6s which have a greater degree of similarity to each other. This suggests that PDE11 constitutes a new PDE gene family rather than be a sub-type of a known family.

For this reason the partial sequence contained within the IMAGE clone 298975 was putatively named, according to current guidelines on PDE nomenclature (Beavo et al. Molecular Pharmacology, 46:399-405), as PDE11A1.

An alignment using the Clustal program (Thompson *et al.* (1994). Nucleic Acids Research, 22:4673-4680.) between the PDE11A1 sequence fragment and bovine PDE5 (PDE5 was selected since functionally this is one of the most well characterised known PDEs) was performed to determine the presence, if any, of the functional domains observed in other PDEs. This is illustrated in FIGURE 1 (panel B), in schematic form, and shows that the sequence fragment of PDE11A1, contained in 298975, overlaps substantially with PDE5 but that it terminates at residue 669 in the PDE5 sequence. This would indicate that substantial 3' sequence for PDE11A1 may be present in addition to that contained within 298975. The presence in PDE11A1 of 2 putative zinc-binding motifs ($HXXXHX_{8-20}D$) supports the hypothesis that PDE11A1 encodes a functional phosphodiesterase enzyme.

**Example 2**

**Isolation of a putative full length cDNA clone for PDE11A1**

To facilitate the isolation of a full length cDNA for PDE11A1 containing the 3' end of the coding region and a terminator codon, the expression of PDE11A1 was determined in a range of tissues using Northern hybridisation. This data (illustrated in FIGURE 2) shows that while the messenger RNA (length 9.5kb), which hybridises to PDE11A1, is present in several tissues, it is particularly highly expressed in the caudate nucleus and putamen regions of the brain. Therefore, caudate nucleus cDNA (Clontech - Clontech Laboratories, 1020 East Meadow Circle, Palo Alto, California, 94303, USA) was used as a template for a PCR based approach (see methods) to generate extra 3' sequence. Initial attempts at 3' RACE were unsuccessful so an alternative strategy was devised to generate further 3' sequence. This was achieved by using an oligonucleotide:

RP1:

5'-TC NCC YTG RTC RTA RAA YTC- 3', SEQ ID No. 5

This was based upon conserved sequences observed in alignments of PDEs 5 and 6 and was used as the 3' primer.

A second oligonucleotide, the 5' primer was also used:

GSP1:

5' -TAC TTC AGA ACA ATC ACA CG- 3', SEQ ID No. 6

This was based upon known PDE11A1 sequence. A PCR carried out using these primers (the PCR was set up using reagents obtained from a PCR Reagent System kit (Life Technologies - 3 Fountain Drive, Inchinnan Business Park, Paisley, PA4 9RF UK) and following the manufacturers instructions, with standard cycling parameters) resulted in the generation of a fragment, which upon cloning into the TOPO vector and full length sequencing proved to overlap with the PDE11A1 sequence derived from IMAGE clone 298975 at it's 5' end and to extend the 3' sequence a further 400bp towards the 3' end of PDE11A1. However, this fragment did not contain a terminator codon indicating that further PDE11A1 at the 3' end remained to be isolated.

To test this hypothesis 3' RACE was carried out on caudate nucleus Marathon Ready™ cDNA (Clontech - Clontech Laboratories, 1020 East Meadow Circle, Palo Alto, California, 94303, USA) with SEQ ID No. 6 (see above) and the 3' primer supplied with the 3'RACE kit (Clontech {Clontech Laboratories, 1020 East Meadow Circle, Palo Alto, California, 94303, USA}, using reagents and methodologies recommended by the manufacturer). These RACE products were used to generate a mini-library of 2000 clones in the TOPO vector (Invitrogen - De Schelp 12, 9351 NV Leek, The Netherlands). This mini-library was screened by hybridisation with a probe derived from the 3' end of the extended fragment derived above. 5 clones were identified which upon sequencing proved to contain further 3' PDE related sequences. These clones also contained an in frame terminator codon, indicating the 3' limit of the putative coding sequence within this cDNA. The procedure used to isolate a full length PDE11A1 cDNA is summarised schematically in FIGURE 3. Analysis of all 3 fragments allowed the assembly of a contiguous sequence of 2557 bp which contains an ORF of 789 residues. The full length cDNA sequence for PDE11A1 is given in FIGURE 4A (SEQ ID No. 1) with the translation of the largest open reading frame within this cDNA given in FIGURE 4B (SEQ ID No. 2).

Like all mammalian phosphodiesterases sequenced to date PDE11A1 contains a conserved catalytic domain sequence of approximately 250 amino acids in the carboxyl-terminal half of the protein that is thought to be essential for catalytic activity. This segment comprises amino acids 499 to 731 in SEQ ID 4 and is also indicated schematically in FIGURE 1 (panel C) and exhibits sequence conservation with the corresponding region of other PDEs.

**Example 3**

**Isolation of a 5' splice variant of PDE11A1**

In order to guard against the possibility that the ATG, translation initiation codon indicated in FIGURE 4A, is not the most 5' ATG in PDE11A1, 5'RACE was performed on caudate nucleus MarathonReady™ cDNA (Clontech - Clontech Laboratories, 1020 East Meadow Circle, Palo Alto, California, 94303, USA) to isolate further upstream sequence. The 5' primer used was the adapter primer supplied in the 5' RACE kit (Clontech - Clontech Laboratories, 1020 East Meadow Circle, Palo Alto, California, 94303, USA) the 3' primer used was the oligonucleotide:

GSP3:

5'- TCT CCA AGG AAA TAC AGT GC- 3' SEQ ID No. 7

The reaction conditions were as specified by the manufacturers instructions. The resulting 5' RACE products were cloned into the TOPO vector (Invitrogen - De Schelp 12, 9351 NV Leek, The Netherlands) and analysed for the presence of PDE11A1 related sequences by hybridisation screening with a probe derived from the 5' end of PDE11A1. This analysis revealed the presence of an upstream 190 bp coding sequence. It is concluded that the ATG indicated in FIGURE 4A may represent the genuine translation initiation codon for PDE11A1. Sequence analysis of several of the 5'RACE clones revealed the presence of a splice variant of PDE11A1, named PDE11A2 which differs in the coding region whereby the terminal 23 codons are replaced, in PDE11A2, by an alternative 25 codons. FIGURE 5C illustrates an alignment of the amino termini of PDE11A1 and A2 for comparison. The full cDNA sequence and translation are given in FIGURES 5A and 5B. PDE11A1 and PDE11A2 also differ in the sequences 5' to the known ATG initiation codons. One interesting observation is that PDE11A2 may not possess the protein kinase A potential phosphorylation site when detected using the prosite program (Bairoch et al., The PROSITE database, its status in 1997. Nucleic Acids Res. 25:217-221(1997)).

**Example 4**

**PDE11A1 shows cyclic nucleotide hydrolytic activity when expressed in the baculovirus system**

PDE11A1 protein was generated using the baculovirus expression system based on *Autographa californica* nuclear polyhedrosis virus (AcNPV) infection of *Spodoptera frugiperda* insect cells (*Sf*9 cells). Briefly, PDE11 was cloned into the donor plasmid pFASTBAC-FLAG which contains a mini-Tn7 transposition element. The recombinant plasmid was transformed into DH10BAC competent cells which contain the parent bacmid bMON14272 (AcNPV infectious DNA) and a helper plasmid. The mini-Tn7 element on the pFASTBAC donor can transpose to the attTn7 attachment site on the bacmid thus introducing PDE11 into the viral genome. Colonies containing recombinant bacmids are identified by disruption of the *lac*Z gene. The PDE11/bacmid construct can then be isolated and infected into insect cells (*Sf*9 cells) resulting in the production of infectious recombinant baculovirus particles and expression of recombinant PDE11A1-FLAG fusion protein.

The phosphodiesterase activity of the crude cell extracts was measured using a modification of the assay procedure described for the cGs-PDE in Martins et al.. J.Biol.Chem., 257:1973-1979 (See also materials and methods). Cells were harvested and extracts prepared 24, 48 and 72 hours after transfection. The results of the assays are presented in FIGURE 6 where the results shown are averages of 3 separate infections. Infection of the insect *Sf*9 cells resulted in the expression of approximately 10-15 fold higher levels of both cAMP and cGMP phosphodiesterase activity than in mock infected cells. These results confirm that PDE11A1 cDNA encodes a phosphodiesterase which is able to hydrolyse both cAMP and cGMP.

The crude lysate material was purified by FPLC using a column containing agarose beads (M2 affinity gel) to which a purified $IgG_1$ monoclonal anti-FLAG antibody had been conjugated by hydrazide linkage (Eastman Kodak). This allows the specific retention on the column of the recombinant material (since this is fused to the FLAG epitope) whilst the endogenous insect proteins are washed off in the eluate. The recombinant material is then washed off under conditions of low pH. This purified material was more suitable for detailed enzymatic and inhibitor studies. The purity of the material is assessed by coomassie staining after sodium dodecyl sulphate-polyacrylamide gel electrophoresis (SDS-PAGE) or through western blotting onto a nitro-cellulose membrane of an unstained SDS-PAGE (containing recombinant PDE11A1) and analysis with the IgG1 monoclonal anti-FLAG epitope antibody. The PDE11A1-FLAG fusion protein is detected due to the interaction between the anti-FLAG antibody and the FLAG epitope which is fused to the PDE11A1 protein.

The phosphodiesterase activity of the purified PDE11A1-FLAG fusion protein was assayed using a commercially available SPA (scintillation proximity assay) kit (Amersham - Amersham place, Little Chalfont, Bucks, HP7 9NA UK) for either cAMP or cGMP. This was used to permit the determination of the Km value for PDE11A1 against cAMP and cGMP by determining the enzyme activity at a range of substrate concentrations allowing the calculation of an approximate Vmax value for the enzyme. The results of these experiments are given in FIGURE 7 and show that the Km for PDE11A1 against cAMP is 400nM and about 4mM against cGMP, indicating that the enzyme has a higher affinity for cAMP over cGMP. Thus, the PDE11 enzyme of the present invention has an affinity for cAMP and cGMP, but wherein it has a higher affinity for cAMP than cGMP. Preferably, the enzyme has an affinity of an order of at least about 5 for cAMP than cGMP, more preferably an affinity of an order of at least about 6 for cAMP than cGMP, more preferably an affinity of an order of at least about 7 for cAMP than cGMP, more preferably an affinity of an order of at least about 8 for cAMP than cGMP, more preferably an affinity of an order of at least about 8 for cAMP than cGMP, more preferably an affinity of an order of about 10 (or more) for cAMP than cGMP.

**Example 5**

***In situ* hybridisation studies with PDE11A1**

This analysis was carried to investigate the potential function of PDE11A1 and as to what therapeutic opportunities may be exploited by inhibiting this enzyme. Accordingly a PDE11A1 riboprobe (see methods) was generated and radiolabelled with 35S. Examples of this sort of analysis are illustrated in FIGURE 8, illustrating expression in endothelial cells of the corpus cavernosum, ganglia in the gut, epithelial cells of the prostate and neuronal nuclei of the striatal region of the brain. These data may serve to illustrate the multitude of opportunities which inhibition of PDE11 activity may represent.

**Example 6**

**Generation of PDE11 antibodies**

Two anti-peptide PDE11 anti-bodies have been generated by a commercial supplier of these reagents (Zeneca CRB - Cambridge Research Biochemicals, Gadbrook Park, Northwich, Cheshire, CW9 7RA. UK).
The first peptide sequence was:

MEDGPSNNASC (SEQ ID No. 8)

and corresponded to residues 01 to 12 of PDE11A1.
The second peptide sequence was:

EDESAPKEVSRYC (SEQ ID No.9)

and corresponded to residues 64-77 of PDE11A1.
These sequences were generated using standard procedures by Zeneca CRB (Cambridge Research Biochemicals, Gadbrook Park, Northwich, Cheshire, CW9 7RA. UK) and injected into rabbits using a standard antigen injection regimen. Anti-bodies were supplied as serum and were characterised by Western blot against recombinantly produced PDE11A1 protein. The data from this experiment is given in FIGURE 9 and shows that both antibodies (Ab-1 and Ab-2) are capable of detecting the recombinant PDE11 protein when present in a background of insect protein present in the crude lysate.

**Example 7**

A cDNA containing the full coding sequence of PDE11A1 is obtained from human caudate nucleus cDNA (Clontech catalogue # 7191-1 {Clontech Laboratories, 1020 East Meadow Circle, Palo Alto, California, 94303, USA}) using the polymerase chain reaction (PCR) and the following oligonucleotides:

5' primer:

TTC GGA TCC GAC ATG GAA GAT GGA CC (SEQ ID No. 10)

and the 3' primer:

GGT GAC TAG TGC TCA ATC TTC AGA TGC (SEQ ID No. 11)

with the PCR reagent system (Life Technologies, Catalogue # 10198-018 (3 Fountain Drive, Inchinnan Business Park, Paisley, PA4 9RF UK}) in a reaction volume of 100 μl or by using conventional hybridisation screening.
The full sequence for PDE11A1 is provided herein. We have also isolated a splice variant of PDE11A1, namely PDE11A2 which differs at the amino terminus as indicated herein. The remainder of the sequence of PDE11A2 is identical to PDE11A1. PDE11A2 can also be obtained from caudate nucleus cDNA using the appropriate primers and a PCR protocol or by hybridisation screening.
The coding sequence of the PDE11A1 cDNA encodes a protein of 789 amino acid residues. The coding sequence of the PDE11A2 cDNA encodes a protein of 791 amino acid residues.
Sequence ID No. 1 is the full sequence for the PDE11A1 cDNA. Sequence ID No. 2 is the translated protein sequence for PDE11A1. Sequence ID No. 3 is the full sequence for the PDE11A2 cDNA. Sequence ID No. 4 is the translated protein sequence for PDE11A2.

**Example 8**

**The Mouse Sequence**

A mouse homologue for PDE11 was found in a clone (IMAGE clone 760844). This homologue was anlaysed according to the protocols layed out above for human PDE11A1. Full length sequecing of the insert within this clone revealed that it contained an insert of 1068 bp of which an internal 900bp corresponded very closely to human PDE11.

The following is a partial nucletide sequence derived from Image clone 760844. The sequence in bold indicates the region of the insert of this clone that has high homology to human PDE11.

## SEQ ID No. 12

ATTTGCACTGTACT**TTCCTTGGAGAGTGCAATAATAGCCTGTGTGTGTTCATACCACCCGGGATGAAGGAAGG**
**CCAACCCCGGCTCATCCCTGCGGGGCCCATCACCCAGGGTACCACCATCTCTGCCTACGTGGCCAAGTCTAGG**
**AAGACGTTGTTGGTAGAGGATATCCTTGGGGATGAGCGATTTCCTCGAGGTACTGGCCTGGAATCAGGAACCC**
**GCATCCAGTCTGTTCTTTGCTTGCCCATTGTCACTGCCATTGGAGACTTGATTGGCATCCTTGAACTGTACAG**
**GCACTGGGACAAAGAGGCCTTCTGCCTCAGCCATCAGGAGGTTGCAACAGCCAATCTTGCTTGGGCTTCCGTA**
**GCAATACACCAGGTGCAGGTGTGTAGAGGTCTCGCCAAACAGACCGAACTGAATGACTTCCTACTCGACGTAT**
**CAAAGACATACTTTGATAACATAGTTGCCATAGACTCTCTACTTGAACACATCATAATATATGCAAAAAATCT**
**AGTGAACGCCGACCGCTGCGCGCTCTTCCAGGTGGACCACAAGAACAAGGAGCTGTACTCGGACCTGTTTGAC**
**ATTGGGGAGGAGAAGGAGGGGAAGCCCATCTTCAAGAAGACCAAGGAGATCAGATTTTCCATTGAGAAAGGGA**
**TTGCTGGTCAAGTGGCAAGAACAGGCGAAGTCTTGAACATTCCCGATGCCTACGCGGACCCTCGCTTTAACAG**
**GGAGGTGGACCTGTACACAGGCTACACCACGAGGAACATTCTGTGTATGCCCATAGTGAGCCGAGGCAGCGTG**
**ATTGGCGTGGTGCAGATGGTGAACAAGATCAGCGGTAGCGCCTTCTCCAAGACAGACGAGAACAACTTCAAGA**
**TGTTTGCTGTCTTCTGCGCACTGGCCTTGCACTGTGCTAA**CGCCAGATGGAAAAGCCTAGCTTCTCTCCCCTG
GGTCAGCTGGGAAGGTTTGCTAGCTTGCCTGCACTGTGGCAAAGACCTGAGGACCTGGAATGGTGACCACTGT
CTGACGTGCACAGTCTTTCCTGCCTCTGTGACACCCGCTTGGGATA

The following is the protein translation of the sequence indicated in bold above, it is 300 residues long and does not contain either an ATG translation initiator codon or a TGA,TAA or TAG terminator codon. We therefore believed that it represented a fragment of the mouse PDE11 cDNA.

## SEQ ID No. 13

FLGECNNSLCVFIPPGMKEGQPRLIPAGPITQGTTISAYVAKSRKTLLVEDILGDERFPRGTGLESGTRIQSV
LCLPIVTAIGDLIGILELYRHWDKEAFCLSHQEVATANLAWASVAIHQVQVCRGLAKQTELNDFLLDVSKTYF
DNIVAIDSLLEHIIIYAKNLVNADRCALFQVDHKNKELYSDLFDIGEEKEGKPIFKKTKEIRFSIEKGIAGQV
ARTGEVLNIPDAYADPRFNREVDLYTGYTTRNILCMPIVSRGSVIGVVQMVNKISGSAFSKTDENNFKMFAVF
CALALHCA

Figure 10 shows the sequence alignment to Human PDE11A1 between residues 126 and 425. This is alignment was generated using the BLAST algorithm of human PDE11A1 protein sequence and mouse PDE11 protein sequence.

Thus, we believed that IMAGE clone 760844 contained a DNA sequence the majority of which is very closely related to human PDE11 and would therefore represent a mouse homologue of the human PDE11 gene. Whilst this cDNA may only contain a fragment of the mouse gene it does represent a variant of the human PDE11 cDNA sequence.

Based on this belief, we then fully sequenced the mouse PDE11 and its coding sequence.

In this respect, the full cDNA sequence of cloned mouse PDE 11 is provided herein as SEQ ID No. 14. The mouse PDE 11 sequence itself is presented as SEQ ID No. 15. The mouse sequence is sometimes referred to as: MMPDE11A3.

Figure 11 shows a sequence comparison between the human PDE11 sequences (HSPDE11A1, HSPDE11A2) and the mouse PDE11 sequence (MPDE11A3). As can be seen, there is a close match between the sequences.

*In situ* hybridisation studies showed that MMPDE11A3 coding sequences are localised in high levels in the striatum of the mouse.

**Example 9**

**The Rat Sequence**

A rat homologue for PDE11 was found, which we have called PDE11A4. We have been able to sequence parts of this homolgue. In this respect, two nucleotide sequences are presented as SEQ ID No. 16 and SEQ ID No. 18. The two respective amino acid sequences are SEQ ID No. 17 and SEQ ID No. 19. Data analysis reveals a very high homology with the human and rat sequences presented herein.

SUMMARY

In summary the present invention provides and the Examples show *inter alia:*

1. Novel amino acids.
2. Novel nucleotide sequences.
3. Assays using said novel sequences.
4. Compounds/compositions identified by use of said assays.
5. Expression systems comprising or expressing said novel sequences.
6. Methods of treatment based on said novel sequences.
7. Pharmaceutical compositions based on said novel sequences.

All publications mentioned in the above specification are herein incorporated by reference. Various modifications and variations of the described methods and system of the present invention will be apparent to those skilled in the art without departing from the scope and spirit of the present invention. Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry and biotechnology or related fields are intended to be within the scope of the following claims.

## Annex to the description

# SEQUENCE LISTINGS

## SEQ ID NO. 1 (Nucleotide sequence coding for PDE11A1)

```
TTCGGCTCCGACATGGAAGATGGACCTTCTAATAATGCGAGCTGCTTCCGAAGGCTGACC 60
GAGTGCTTCCTGAGCCCCAGTTTGACAGATGAAAAAGTGAAGGCATATCTTTCTCTTCAC 120
CCCCAGGTATTAGATGAATTTGTATCTGAAAGTGTTAGTGCAGAGACAGTAGAGAAATGG 180
CTGAAGAGGAAGAACAACAAATCAGAAGATGAATCAGCTCCTAAGGAAGTCAGCAGGTAC 240
CAAGATACGAATATGCAGGGAGTTGTATATGAACTAAACAGCTATATAGAACAACGGTTG 300
GACACAGGAGGAGACAACCAGCTACTCCTCTATGAACTGAGCAGCATCATTAAAATAGCC 360
ACAAAAGCCGATGGATTTGCACTGTATTTCCTTGGAGAGTGCAATAATAGCCTGTGTATA 420
TTCACGCCACCTGGGATAAAGGAAGGAAAACCCCGCCTCATCCCTGCTGGGCCCATCACT 480
CAGGGCACCACCGTCTCTGCTTATGTGGCCAAGTCCAGGAAAACACTGCTAGTAGAAGAC 540
ATCCTTGGAGATGAACGATTTCCAAGAGGTACTGGACTGGAATCAGGGACTCGTATCCAG 600
TCTGTTCTTTGCTTACCAATTGTCACTGCAATTGGTGACTTGATTGGTATTCTCGAGCTG 660
TATCGGCACTGGGGCAAAGAAGCCTTCTGTCTTAGTCACCAGGAGGTTGCAACAGCAAAT 720
CTTGCCTGGGCTTCAGTAGCAATACATCAGGTGCAGGTATGCAGAGGCCTTGCCAAACAG 780
ACAGAATTGAATGACTTCCTACTCGACGTATCAAAAACATATTTTGATAACATAGTTGCA 840
ATAGATTCTCTACTTGAACACATAATGATATATGCAAAAAACCTGGTGAATGCCGATCGT 900
TGTGCACTTTTCCAGGTGGACCATAAGAACAAGGAGTTATATTCAGACCTTTTTGATATT 960
GGAGAGGAAAAGGAAGGAAAACCTGTCTTCAAGAAGACCAAAGAGATAAGATTTTCAATT 1020
GAGAAAGGAATTGCTGGCCAAGTAGCAAGAACAGGGGAAGTCCTGAACATTCCAGATGCC 1080
TATGCAGACCCACGCTTTAACAGAGAAGTAGACTTGTACACAGGCTACACCACGCGGAAC 1140
ATCCTGTGCATGCCCATCGTCAGCCGAGGCAGCGTGATAGGTGTGGTGCAGATGGTCAAC 1200
AAAATCAGTGGCAGTGCCTTCTCTAAAACAGATGAAAACAACTTCAAAATGTTTGCCGTC 1260
TTTTGTGCTTTAGCCTTACACTGTGCTAATATGTATCATAGAATTCGCCACTCAGAGTGC 1320
ATTTACCGGGTAACGATGGAAAAGCTGTCCTACCATAGCATTTGTACTTCAGAAGAGTGG 1380
CAAGGTCTCATGCAATTCACCCTTCCCGTGCGTCTCTGCAAAGAAATTGAATTATTCCAC 1440
TTTGACATTGGTCCTTTTGAAAACATGTGGCCTGGAATTTTTGTCTACATGGTTCATCGG 1500
TCCTGTGGGACATCCTGCTTTGAGCTTGAAAAGTTGTGTCGTTTTATTATGTCTGTGAAG 1560
AAGAACTATCGGCGGGTTCCTTATCACAACTGGAAGCATGCGGTCACTGTAGCACACTGC 1620
ATGTATGCCATACTTCAGAACAATCACACGCTTTTCACAGACCTTGAGCGCAAAGGACTG 1680
CTGATTGCGTGTCTGTGTCATGACCTGGACCACAGGGGCTTCAGTAACAGCTACCTGCAG 1740
AAGTTCGACCACCCTCTGACCGCTCTCTACTCCACTTCCACCATGGAGCAGCACCACTTC 1800
TCCCAGACTGTGTCCATCCTTCAGTTGGAAGGGCACAATATCTTCTCCACTCTGAGCTCC 1860
AGTGAATATGAGCAGGTGCTTGAGATCATCCGCAAAGCCATCATTGCCACAGACCTTGCT 1920
TTATACTTTGGAAACAGGAAGCAGTTGGAAGAGATGTACCAGACCGGATCACTAAACCTT 1980
AATAATCAATCACATAGAGACCGTGTAATTGGTTTGATGATGACTGCCTGTGACCTTTGT 2040
TCTGTGACAAAACCGTGGCCCGTTACAAAATTGACGGCAAATGATATATATGCAGAATTC 2100
TGGGCTGAGGGTGATGAAATGAAGAAATTGGGAATACAGCCTATTCCTATGATGGACAGA 2160
GACAAGAAGGATGAAGTCCCCCAAGGCCAGCTTGGGTTCTACAATGCCGTGGCCATTCCC 2220
TGCTATACAACCCTTACCCAGATCCTCCCTCCCACGGAGCCTCTTCTGAAAGCATGCAGG 2280
GATAATCTCAGTCAGTGGGAGAAGGTGATTCGAGGGGAGGAGACTGCAACCTGGATTTCA 2340
TCCCCATCCGTGGCTCAGAAGGCAGCTGCATCTGAAGATTGAGCACTGGTCACCCTGACA 2400
CGCTGTCCCACCTACAGATCCTCATCTTGCTTCTTTGACATTCTTTTCCTTTTTTGGGGG 2460
GGGTGGGGGGAACCTGCACCTGGTAACTGGGGTGCAAACCTCTTCAAGAAGGTAACATCA 2520
AATAAATAAGTCAAGCAGAAAAAAAAAAAAAAAAAAA                        2557
```

## SEQ ID NO. 2 (Amino acid sequence of PDE11A1)

MEDGPSNNASCFRRLTECFLSPSLTDEKVKAYLSLHPQVLDEFVSESVSAETVEKWLKRK 60
NNKSEDESAPKEVSRYQDTNMQGVVYELNSYIEQRLDTGGDNQLLLYELSSIIKIATKAD 120
GFALYFLGECNNSLCIFTPPGIKEGKPRLIPAGPITQGTTVSAYVAKSRKTLLVEDILGD 180
ERFPRGTGLESGTRIQSVLCLPIVTAIGDLIGILELYRHWGKEAFCLSHQEVATANLAWA 240
SVAIHQVQVCRGLAKQTELNDFLLDVSKTYFDNIVAIDSLLEHIMIYAKNLVNADRCALF 300
QVDHKNKELYSDLFDIGEEKEGKPVFKKTKEIRFSIEKGIAGQVARTGEVLNIPDAYADP 360
RFNREVDLYTGYTTRNILCMPIVSRGSVIGVVQMVNKISGSAFSKTDENNFKMFAVFCAL 420
ALHCANMYHRIRHSECIYRVTMEKLSYHSICTSEEWQGLMQFTLPVRLCKEIELFHFDIG 480
PFENMWPGIFVYMVHRSCGTSCFELEKLCRFIMSVKKNYRRVPYHNWKHAVTVAHCMYAI 540
LQNNHTLFTDLERKGLLIACLCHDLDHRGFSNSYLQKFDHPLTALYSTSTMEQHHFSQTV 600
SILQLEGHNIFSTLSSSEYEQVLEIIRKAIIATDLALYFGNRKQLEEMYQTGSLNLNNQS 660
HRDRVIGLMMTACDLCSVTKPWPVTKLTANDIYAEFWAEGDEMKKLGIQPIPMMDRDKKD 720
EVPQGQLGFYNAVAIPCYTTLTQILPPTEPLLKACRDNLSQWEKVIRGEETATWISSPSV 780
AQKAAASED 789

## SEQ ID NO. 3 (Nucleotide sequence coding for PDE11A2)

ACATAGCTGGGTGCAATGTAAGTGCCTGGCTGAAGTTTGACACGCGAACGGACGGCCCGC 60
TGGAATTCTGTGCTATGAGCCGGAGTAGAAAGAGAGATTTGGACTCTGCAACACCAAGGT 120
AGTCGTTGAAGCCACAGTCGTGAATGGAGACCAGGAGTGAATAGTGGGAGTGAGCAGAAG 180
TCGGAGGATAGGACAGAAGAAGGCAGAGCCATGGAGCACCCTGGAGAGGTGTGACCCGGC 240
AAGATCCTGAGATGGAAGGTAGCACGGCCTGGAGTTCAGAAGCGGAGCCTCAAGAGGGAA 300
GAAGCCAGATGCTCCAGAGAGCAGGTTTGACAGATGAAAAAGTGAAGGCATATCTTTCTC 360
TTCACCCCCAGGTATTAGATGAATTTGTATCTGAAAGTGTTAGTGCAGAGACAGTAGAGA 420
AATGGCTGAAGAGGAAGAACAACAAATCAGAAGATGAATCAGCTCCTAAGGAAGTCAGCA 480
GGTACCAAGATACGAATATGCAGGGAGTTGTATATGAACTAAACAGCTATATAGAACAAC 540
GGTTGGACACAGGAGGAGACAACCAGCTACTCCTCTATGAACTGAGCAGCATCATTAAAA 600
TAGCCACAAAAGCCGATGGATTTGCACTGTATTTCCTTGGAGAGTGCAATAATAGCCTGT 660
GTATATTCACGCCACCTGGGATAAAGGAAGGAAAACCCCGCCTCATCCCTGCTGGGCCCA 720
TCACTCAGGGCACCACCGTCTCTGCTTATGTGGCCAAGTCCAGGAAAACACTGCTAGTAG 780
AAGACATCCTTGGAGATGAACGATTTCCAAGAGGTACTGGACTGGAATCAGGGACTCGTA 840
TCCAGTCTGTTCTTTGCTTACCAATTGTCACTGCAATTGGTGACTTGATTGGTATTCTCG 900
AGCTGTATCGGCACTGGGGCAAAGAAGCCTTCTGTCTTAGTCACCAGGAGGTTGCAACAG 960
CAAATCTTGCCTGGGCTTCAGTAGCAATACATCAGGTGCAGGTATGCAGAGGCCTTGCCA 1020
AACAGACAGAATTGAATGACTTCCTACTCGACGTATCAAAAACATATTTTGATAACATAG 1080
TTGCAATAGATTCTCTACTTGAACACATAATGATATATGCAAAAAACCTGGTGAATGCCG 1140
ATCGTTGTGCACTTTTCCAGGTGGACCATAAGAACAAGGAGTTATATTCAGACCTTTTTG 1200
ATATTGGAGAGGAAAAGGAAGGAAAACCTGTCTTCAAGAAGACCAAAGAGATAAGATTTT 1260
CAATTGAGAAAGGAATTGCTGGCCAAGTAGCAAGAACAGGGGAAGTCCTGAACATTCCAG 1320
ATGCCTATGCAGACCCACGCTTTAACAGAGAAGTAGACTTGTACACAGGCTACACCACGC 1380
GGAACATCCTGTGCATGCCCATCGTCAGCCGAGGCAGCGTGATAGGTGTGGTGCAGATGG 1440
TCAACAAAATCAGTGGCAGTGCCTTCTCTAAAACAGATGAAAACAACTTCAAAATGTTTG 1500
CCGTCTTTTGTGCTTTAGCCTTACACTGTGCTAATATGTATCATAGAATTCGCCACTCAG 1560
AGTGCATTTACCGGGTAACGATGGAAAAGCTGTCCTACCATAGCATTTGTACTTCAGAAG 1620
AGTGGCAAGGTCTCATGCAATTCACCCTTCCCGTGCGTCTCTGCAAAGAAATTGAATTAT 1680
TCCACTTTGACATTGGTCCTTTTGAAAACATGTGGCCTGGAATTTTTGTCTACATGGTTC 1740
ATCGGTCCTGTGGGACATCCTGCTTTGAGCTTGAAAAGTTGTGTCGTTTTATTATGTCTG 1800
TGAAGAAGAACTATCGGCGGGTTCCTTATCACAACTGGAAGCATGCGGTCACTGTAGCAC 1860
ACTGCATGTATGCCATACTTCAGAACAATCACACGCTTTTCACAGACCTTGAGCGCAAAG 1920
GACTGCTGATTGCGTGTCTGTGTCATGACCTGGACCACAGGGGCTTCAGTAACAGCTACC 1980
TGCAGAAGTTCGACCACCCTCTGACCGCTCTCTACTCCACTTCCACCATGGAGCAGCACC 2040
ACTTCTCCCAGACTGTGTCCATCCTTCAGTTGGAAGGGCACAATATCTTCTCCACTCTGA 2100
GCTCCAGTGAATATGAGCAGGTGCTTGAGATCATCCGCAAAGCCATCATTGCCACAGACC 2160
TTGCTTTATACTTTGGAAACAGGAAGCAGTTGGAAGAGATGTACCAGACCGGATCACTAA 2220

```
ACCTTAATAATCAATCACATAGAGACCGTGTAATTGGTTTGATGATGACTGCCTGTGACC 2280
TTTGTTCTGTGACAAAACCGTGGCCCGTTACAAAATTGACGGCAAATGATATATATGCAG 2340
AATTCTGGGCTGAGGGTGATGAAATGAAGAAATTGGGAATACAGCCTATTCCTATGATGG 2400
ACAGAGACAAGAAGGATGAAGTCCCCCAAGGCCAGCTTGGGTTCTACAATGCCGTGGCCA 2460
TTCCCTGCTATACAACCCTTACCCAGATCCTCCCTCCCACGGAGCCTCTTCTGAAAGCAT 2520
GCAGGGATAATCTCAGTCAGTGGGAGAAGGTGATTCGAGGGGAGGAGACTGCAACCTGGA 2580
TTTCATCCCCATCCGTGGCTCAGAAGGCAGCTGCATCTGAAGATTGAGCACTGGTCACCC 2640
TGACACGCTGTCCCACCTACAGATCCTCATCTTGCTTCTTTGACATTCTTTTCCTTTTTT 2700
GGGGGGGGTGGGGGGAACCTGCACCTGGTAACTGGGGTGCAAACCTCTTCAAGAAGGTAA 2760
CATCAAATAAATAAGTCAAGCAGAAAAAAAAAAAAAAA 2799
```

## SEQ ID NO. 4 (Amino acid sequence of PDE11A2)

```
MEGSTAWSSEAEPQEGRSQMLQRAGLTDEKVKAYLSLHPQVLDEFVSESVSAETVEKWLK 60
RKNNKSEDESAPKEVSRYQDTNMQGVVYELNSYIEQRLDTGGDNQLLLYELSSIIKIATK 120
ADGFALYFLGECNNSLCIFTPPGIKEGKPRLIPAGPITQGTTVSAYVAKSRKTLLVEDIL 180
GDERFPRGTGLESGTRIQSVLCLPIVTAIGDLIGILELYRHWGKEAFCLSHQEVATANLA 240
WASVAIHQVQVCRGLAKQTELNDFLLDVSKTYFDNIVAIDSLLEHIMIYAKNLVNADRCA 300
LFQVDHKNKELYSDLFDIGEEKEGKPVFKKTKEIRFSIEKGIAGQVARTGEVLNIPDAYA 360
DPRFNREVDLYTGYTTRNILCMPIVSRGSVIGVVQMVNKISGSAFSKTDENNFKMFAVFC 420
ALALHCANMYHRIRHSECIYRVTMEKLSYHSICTSEEWQGLMQFTLPVRLCKEIELFHFD 480
IGPFENMWPGIFVYMVHRSCGTSCFELEKLCRFIMSVKKNYRRVPYHNWKHAVTVAHCMY 540
AILQNNHTLFTDLERKGLLIACLCHDLDHRGFSNSYLQKFDHPLTALYSTSTMEQHHFSQ 600
TVSILQLEGHNIFSTLSSSEYEQVLEIIRKAIIATDLALYFGNRKQLEEMYQTGSLNLNN 660
QSHRDRVIGLMMTACDLCSVTKPWPVTKLTANDIYAEFWAEGDEMKKLGIQPIPMMDRDK 720
KDEVPQGQLGFYNAVAIPCYTTLTQILPPTEPLLKACRDNLSQWEKVIRGEETATWISSP 780
SVAQKAAASED 791
```

## SEQ ID No. 5

TCNCCYTGRTCRTARAAYTC

## SEQ ID No. 6

TACTTCAGAACAATCACACG

**SEQ ID No. 7**

TCTCCAAGGAAATACAGTGC

**SEQ ID No. 8**

MEDGPSNNASC

**SEQ ID No. 9**

EDESAPKEVSRYC

**SEQ ID No. 10**

TTCGGATCCGACATGGAAGATGGACC

**SEQ ID No. 11**

GGTGACTAGTGCTCAATCTTCAGATGC

**SEQ ID No. 12**

ATTTGCACTGTACTTTCCTTGGAGAGTGCAATAATAGCCTGTGTGTGTTCATACCACCCGGGATGAAGGAAGG
CCAACCCCGGCTCATCCCTGCGGGGCCCATCACCCAGGGTACCACCATCTCTGCCTACGTGGCCAAGTCTAGG
AAGACGTTGTTGGTAGAGGATATCCTTGGGGATGAGCGATTTCCTCGAGGTACTGGCCTGGAATCAGGAACCC
GCATCCAGTCTGTTCTTTGCTTGCCCATTGTCACTGCCATTGGAGACTTGATTGGCATCCTTGAACTGTACAG
GCACTGGGACAAAGAGGCCTTCTGCCTCAGCCATCAGGAGGTTGCAACAGCCAATCTTGCTTGGGCTTCCGTA
GCAATACACCAGGTGCAGGTGTGTAGAGGTCTCGCCAAACAGACCGAACTGAATGACTTCCTACTCGACGTAT
CAAAGACATACTTTGATAACATAGTTGCCATAGACTCTCTACTTGAACACATCATAATATATGCAAAAAATCT
AGTGAACGCCGACCGCTGCGCGCTCTTCCAGGTGGACCACAAGAACAAGGAGCTGTACTCGGACCTGTTTGAC
ATTGGGGAGGAGAAGGAGGGGAAGCCCATCTTCAAGAAGACCAAGGAGATCAGATTTTCCATTGAGAAAGGGA
TTGCTGGTCAAGTGGCAAGAACAGGCGAAGTCTTGAACATTCCCGATGCCTACGCGGACCCTCGCTTTAACAG
GGAGGTGGACCTGTACACAGGCTACACCACGAGGAACATTCTGTGTATGCCCATAGTGAGCCGAGGCAGCGTG
ATTGGCGTGGTGCAGATGGTGAACAAGATCAGCGGTAGCGCCTTCTCCAAGACAGACGAGAACAACTTCAAGA
TGTTTGCTGTCTTCTGCGCACTGGCCTTGCACTGTGCTAACGCCAGATGGAAAAGCCTAGCTTCTCTCCCCTG
GGTCAGCTGGGAAGGTTTGCTAGCTTGCCTGCACTGTGGCAAAGACCTGAGGACCTGGAATGGTGACCACTGT
CTGACGTGCACAGTCTTTCCTGCCTCTGTGACACCCGCTTGGGATA

## SEQ ID No. 13

FLGECNNSLCVFIPPGMKEGQPRLIPAGPITQGTTISAYVAKSRKTLLVEDILGDERFPRGTGLESGTRIQSV
LCLPIVTAIGDLIGILELYRHWDKEAFCLSHQEVATANLAWASVAIHQVQVCRGLAKQTELNDFLLDVSKTYF
DNIVAIDSLLEHIIIYAKNLVNADRCALFQVDHKNKELYSDLFDIGEEKEGKPIFKKTKEIRFSIEKGIAGQV
ARTGEVLNIPDAYADPRFNREVDLYTGYTTRNILCMPIVSRGSVIGVVQMVNKISGSAFSKTDENNFKMFAVF
CALALHCA

## SEQ ID No. 14 (Nucleotide sequence of PDE11A3)

ACGCGTCCGCTCCTCATCTGCCTTCCACCTCCCCGCGCGTCTCCCGAGAAGGGAGGGCGCAGCGGCGGCTGGA
GGAGGAGGAGGCGGCGGCGGCGATGCTGGCGGCGGCGGCGGAGGAGGAGGACAAGAGGCAGCTCCCTTGAGCG
TCCCCCCAGGCAGTAGTCACCGCAGCAGCGGTGGCAGCAGCGGTGGCAGCGGCGGGCGGCGGCGGCTCTTCCT
CTCGCCTGCGATTCAAGGCTTGCTGCTCCCTGCCCGCGCCGGGCCCCGGCCATCTCCGCCGCCGCGGCTTCCC
CTACACCCGGGTGCACGCCGCGCGGACTCCTCGGATTTTCCGGGCGCCGGCGGGGGCTGCCCTGGCCTCGGCC
CCGGCTCTGCCGCCGGTGGCCGAACTCTTTGGCGGCCCCGAGGCGCCGCCTTCCCCCTTGCCACCGTTTGGCC
GCTGCCCTTCGGCTCCGACATGGAAGATGGACCCTCTAACAATGCGAGTTGCTTCCGAAGGCTGACCGAGTGT
TTCCTCAGCCCCAGTTTGACGGATGAAAAGGTGAAGGCCTATCTTTCTCTCCATCCCCAGGTATTAGATGAAT
TTGTTTCTGAAAGTGTTAGTGCAGAGACTGTGGAAAAGTGGCTGAAGAGGAAAACCAACAAAGCAAAAGATGA
ACCATCTCCCAAGGAAGTCAGCAGGTACCAGGATACGAATATGCAGGGAGTCGTGTACGAGCTGAACAGCTAC
ATAGAGCAGCGCCTGGACACGGGCGGGGACAACCACCTGCTCCTCTATGAGCTCAGCAGCATCATCAGGATAG
CCACAAAAGCCGACGGATTTGCACTGTACTTCCTTGGAGAGTGCAATAATAGCCTGTGTGTGTTCATACCACC
CGGGATGAAGGAAGGCCAACCCCGGCTCATCCCTGCAGGGCCCATCACCCAGGGTACCACCATCTCTGCCTAC
GTGGCCAAGTCTAGGAAGACGTTGTTGGTAGAGGATATCCTTGGGGATGAGCGATTTCCTCGAGGTACTGGCC
TGGAATCAGGAACCCGCATCCAGTCTGTTCTTTGCTTGCCCATTGTCACTGCCATTGGAGACTTGATTGGCAT
CCTTGAACTGTACAGGCACTGGGGCAAAGAGGCCTTCTGCCTCAGCCATCAGGAGGTTGCAACAGCCAATCTT
GCTTGGGCTTCCGTAGCAATACACCAGGTGCAGGTGTGTAGAGGTCTCGCCAAACAGACCGAACTGAATGACT
TCCTACTCGACGTATCAAAGACATACTTTGATAACATAGTTGCCATAGACTCTCTACTTGAACACATCATGAT
ATATGCAAAAAATCTAGTGAACGCCGACCGCTGCGCGCTCTTCCAGGTGGACCACAAGAACAAGGAGCTGTAC
TCGGACCTGTTTGACATTGGGGAGGAGAAGGAGGGGAAGCCCATCTTCAAGAAGACCAAGGAGATCAGATTTT
CCATTGAGAAAGGGATTGCTGGTCAAGTGGCAAGAACAGGCGAAGTCTTGAACATTCCCGATGCCTACGCGGA
CCCTCGCTTTAACAGGGAGGTGGACCTGTACACAGGCTACACCACGAGGAACATTCTGTGTATGCCCATAGTG
AGCCGAGGCAGCGTGATTGGCGTGGTGCAGATGGTGAACAAGATCAGCGGTAGCGCCTTCTCCAAGACAGACG
AGAACAACTTCAAGATGTTTGCTGTCTTCTGCGCACTGGCCTTGCACTGTGCTAACATGTACCACAGGATCCG
CCACTCAGAATGCATCTACAGGGTTACCATGGAGAAGCTTTCCTACCACAGCATCTGCACCTCCGAGGAGTGG
CAAGGCCTCATGCGCTTCAACCTACCAGCACGCATCTGCCGGGACATCGAGCTATTCCACTTTGACATTGGTC
CTTTCGAGAACATGTGGCCTGGGATCTTTGTCTACATGATCCATCGGTCTTGTGGGACATCCTGTTTTGAACT
TGAAAAATTGTGCCGTTTTATCATGTCTGTGAAGAAGAACTATCGGCGGGTTCCTTACCACAACTGGAAGCAT

GCAGTCACGGTGGCACACTGCATGTATGCCATACTTCAAAACAACAATGGCCTCTTCACAGACCTCGAGCGCA
AAGGCCTGCTAATTGCGTGTCTGTGCCATGACCTGGACCACAGGGGCTTCAGTAACAGCTACCTGCAGAAGTT
CGACCACCCCCTGGCGGCGCTGTACTCCACCTCCACCATGGAGCAACACCACTTCTCCCAGACGGTGTCCATC
CTTCAGCTGGAAGGGCACAATATCTTCTCCACCCTGAGCTCCAGCGAGTACGAGCAGGTGCTGGAGATCATCC
GCAAAGCCATCATCGCCACCGACCTCGCCCTATACTTTGGGAACAGGAAGCAGTTGGAGGAGATGTACCAGAC
AGGGTCGCTGAACCTCCACAACCAGTCCCATCGAGACCGTGTCATCGGCTTGATGATGACTGCCTGTGATCTT
TGCTCTGTGACCAAACTATGGCCAGTTACAAAATTGACAGCGAATGATATATATGCAGAATTCTGGGCTGAGG
GTGATGAGATGAAGAAGCTGGGCATACAGCCCATTCCTATGATGGACAGAGACAAGCGAGATGAAGTCCCTCA
AGGGCAGCTCGGATTCTACAATGCTGTGGCCATTCCCTGCTATACCACCTTGACGCAGATCCTCCCACCCACA
GAGCCTCTGCTGAAGGCCTGCAGGGATAACCTCAATCAGTGGGAGAAGGTAATTCGCGGGGAAGAGACAGCAA
TGTGGATTTCAGGCCCAGGCCCGGCGCCTAGCAAGAGCACACCTGAGAAGCTGAACGTGAAGGTTGAAGACTG
ATCCTGAAGTGACGTCCTGATGTCTGCCCAGCAACCGACTCAACCTGCTTCTGTGACTTCGTTCTTTTTGTTT
TCAAGGGGTGAAAACCCCCTGTCAGAAGGTACCGTCGCATATCCATGTGAAGCAGACGACTCCCTGCTTGCCG
CACACACCTCGGACAGTGAGCAACCCAGGCTCTGCCGTGTTCAGACGTCGGCTACTCCGTGGCTCCACCTGAC
CTCCGAATGCTATTTGCTCCCAGGCCAGCACTGCACTGTCTGGAGGGGGCAGAGACCACAGGAGAGGTTCTTG
CCTGCATCCTCCCATGAGGGTGTGGCCAGTTCCCTGGTTCTGTGCCATGCTGCTGCTTGGTGGCATTGGTTAG
GAATGGGACACACGCCCCTTGTTGTGAAGTTTACATGTGACCTTCTTATAGGTTAACTGAGTTTGTGGCCTGG
GACACATGTAATGAAGGTCACAGTCCACAGGTGACAGAGAAATCCAAACTGTTGATTACAGGTGCACTACAGG
TATGCTCTTTCAGTCTATCTGGGGGCACATAGGTGAGTCTGCTCCACTCAGAAGGAAGCATACCTCTGCCCTC
ATCCAGGGGACACAGGGTACATCCCAGGCATCGGGGAACTGAAGCTCTCACTTCAAACCATGTCAAAGAATTA
AAACACCTCCCCTCCCCCTCACTGTAGCCTTCGGCAACTGCGCCAATCCCTTTATACAAAGAAAATAAAAGTA
AGGCATATAAATTTAAAAAAAAAAAAAAA

**SEQ ID No. 15 (Amino acid sequence of PDE11A3)**

MEDGPSNNASCFRRLTECFLSPSLTDEKVKAYLSLHPQVLDEFVSESVSAETVEKWLKRKTNKAKDEPSPKEVSRYQDTN
MQGVVYELNSYIEQRLDTGGDNHLLLYELSSIIRIATKADGFALYFLGECNNSLCVFIPPGMKEGQPRLIPAGPITQGTT
ISAYVAKSRKTLLVEDILGDERFPRGTGLESGTRIQSVLCLPIVTAIGDLIGILELYRHWGKEAFCLSHQEVATANLAWA
SVAIHQVQVCRGLAKQTELNDFLLDVSKTYFDNIVAIDSLLEHIMIYAKNLVNADRCALFQVDHKNKELYSDLFDIGEEK
EGKPIFKKTKEIRFSIEKGIAGQVARTGEVLNIPDAYADPRFNREVDLYTGYTTRNILCMPIVSRGSVIGVVQMVNKISG
SAFSKTDENNFKMFAVFCALALHCANMYHRIRHSECIYRVTMEKLSYHSICTSEEWQGLMRFNLPARICRDIELFHFDIG
PFENMWPGIFVYMIHRSCGTSCFELEKLCRFIMSVKKNYRRVPYHNWKHAVTVAHCMYAILQNNNGLFTDLERKGLLIAC
LCHDLDHRGFSNSYLQKFDHPLAALYSTSTMEQHHFSQTVSILQLEGHNIFSTLSSSEYEQVLEIIRKAIIATDLALYFG
NRKQLEEMYQTGSLNLHNQSHRDRVIGLMMTACDLCSVTKLWPVTKLTANDIYAEFWAEGDEMKKLGIQPIPMMDRDKRD
EVPQGQLGFYNAVAIPCYTTLTQILPPTEPLLKACRDNLNQWEKVIRGEETAMWISGPGPAPSKSTPEKLNVKVED

SEQ ID No. 16

Partial sequence obtained from a rat clone representing the first 1250 bp of rat PDE11.

CCTTATGGAAGATGGACCCTCTAACAATGCGAGTTGCTTCCGAAGGCTGACCGAGTGTTTC
CTCAGCCCCAGTTTGACGGATGAAAAGGTGAAGGCCTATCTTTCCCTCCATCCCCAGGTAT
TAGACGAGTTTGTTTCTGAAAGTGTTAGTGCGGAGACCGTGGAGAAGTGGCTGAAGAGGAA
AAACGACAAAGCAGAAGATGAACCATCTCCTAAGGAAGTCAGCAGGTACCAGGACACGAAC

ATGCAGGGAGTCGTGTACGAGCTGAACAGCTACATAGAGCAGCGCCTGGACACCGGCGGGG
ACAACCACCTGCTCCTGTACGAGCTAAACAGTATCATCAGGATAGCCACAAAAGCCGACGG
ATTTGCACTGTACTTCCTTGGAGAGTGCAATAATAGTCTGTGTGTCTTCACACCACCCGGA
ATGAAGGAAGGTCAACCCCGTCTCATCCCCGCAGGGCCCATCACCCAGGGCACCACCATC

SEQ ID No. 17

MEDGPSNNASCFRRLTECFLSPSLTDEKVKAYLSLHPQVLDEFVSESVSAETVEKWLKRKN
DKAEDEPSPKEVSRYQDTNMQGVVYELNSYIEQRLDTGGDNHLLLYELNSIIRIATKADGF
ALYFLGECNNSLCVFTPPGMKEGQPRLIPAGPITQGTT

SEQ ID No. 18

Partial sequence obtained from a rat clone containing rat PDE11.

AATCTCGCTTGGGCTTCCGTAGCAATACACCAGGTGCAGGTGTGCAGAGGTCTCGCCAAGC
AGACCGAACTGAATGACTTCCTGCTCGATGTATCAAAGACATACTTTGATAACATAGTCGC
CATAGACTCTCTACTTGAACACATCATGATATATGCAAAAAATCTAGTGAACGCCGACCGC
TGCGCGCTCTTCCAGGTGGACCACAAGAACAAGGAGCTGTACTCGGACCTGTTTGACATTG
GGGAGGAGAAGGAGGGGAAGCCCGTCTTCAAGAAGACCAAGGAGATCAGATTTTCCATTGA
GAAAGGGATTGCTGGTCAAGTGGCAAGAACGGGAGAAGTCCTGAACATTCCTGATGCCTAC
GCAGACCCGCGCTTTAACAGGGAGGTGGACCTGTACACAGGCTATACCACGCGGAACATTC
TGTGTATGCCCATAGTGAGCCGCGGCAGCGTGATCGGTGTGGTGCAAATGGTTAACAAGAT
CAGCGGCAGCGCCTTCTCCAAGACGGATGAGAACAACTTCAAGATGAAGGGC

SEQ ID No. 19

NLAWASVAIHQVQVCRGLAKQTELNDFLLDVSKTYFDNIVAIDSLLEHIMIYAKNLVNADR
CALFQVDHKNKELYSDLFDIGEEKEGKPVFKKTKEIRFSIEKGIAGQVARTGEVLNIPDAY
ADPRFNREVDLYTGYTTRNILCMPIVSRGSVIGVVQMVNKISGSAFSKTDENNFKM

## Claims

1. An amino acid sequence comprising the sequence presented as Formula I or a variant, homologue, fragment or derivative thereof.

2. An amino acid sequence comprising the sequence presented as Formula I.

**3.** A nucleotide sequence encoding the amino acid sequence as defined in claim 1 or claim 2.

**4.** A nucleotide sequence comprising the sequence presented as SEQ ID No. 1 or SEQ ID No. 3 or SEQ ID No. 14 or a variant, homologue, fragment or derivative thereof.

**5.** A nucleotide sequence comprising the sequence presented as SEQ ID No. 1 or SEQ ID No. 3 or SED ID No. 14.

**6.** A nucleotide sequence that is capable of hybridising to the nucleotide sequence according to any one of claims 3 to 5.

**7.** A nucleotide sequence that is capable of hybridising to the nucleotide sequence according to claim 6.

**8.** A vector comprising the nucleotide sequence according to any one of claims 3 to 7.

**9.** A host cell into which has been incorporated the nucleotide sequence according to any one of claims 3 to 7.

**10.** An assay method for identifying an agent that can affect PDE11 activity or expression, the assay method comprising

contacting an agent with an amino acid according to claim 1 or claim 2 or a nucleotide sequence according to any one of claims 3 to 7; and

measuring the activity or expression of PDE11;

wherein a difference between a) PDE activity or expression in the absence of the agent and b) PDE activity or expression in the presence of the agent is indicative that the agent can affect PDE11 activity or expression.

**11.** An assay method according to claim 10 wherein the assay is to screen for agents useful in the treatment of sexual dysfunction.

**12.** A process comprising the steps of:

(a) performing the assay according to claim 10 or claim 11;

(b) identifying one or more agents that do affect PDE11 activity or expression; and

(c) preparing a quantity of those one or more identified agents.

**13.** A method of affecting *in vivo* PDE11 activity or expression with an agent;

wherein the agent is capable of affecting PDE11 activity or expression in an *in vitro* assay method;

wherein the *in vitro* assay method is the assay method defined in claim 10 or claim 11.

**14.** Use of an agent in the preparation of a pharmaceutical composition for the treatment of a disease or condition associated with PDE11, the agent is capable of having an effect on the activity or expression of PDE when assayed *in vitro* by the assay method according to claim 10 or claim 11.

**15.** An enzyme capable of having an immunological reaction with an antibody raised against PDE11.

**16.** A nucleotide sequence coding for a PDE, wherein the nucleotide sequence is obtainable from NCIMB 40925 or NCIMB 40926 or NCIMB 41007.

**17.** A PDE wherein the PDE is expressable from a nucleotide sequence obtainable from NCIMB 40925 or NCIMB 40926 or NCIMB 41007.

**18.** Use of an agent which has an effect on the activity of PDE11 or the expression thereof in the preparation of a pharmaceutical composition for the treatment of a disease or condition associated with PDE11.

**19.** Use of a PDE11 gene and/or expression product thereof in the preparation of a medicament for the treatment and/or modulation of disturbances associated with an inbalance or disturbance of PDE11.

**20.** Use according to claim 19 wherein the PDE11 and/or expression product thereof is used to screen for agents that can modulate the activity of the PDE11 and/or expression product thereof.

**21.** A PDE11 agonist wherein the PDE11 is as defined in claim 1 or is the nucleotide sequence coding for same.

**22.** A PDE11 antagonist wherein the PDE11 is as defined in claim 1 or is the nucleotide sequence coding for same.

**23.** A recombinant PDE11 enzyme.

**24.** A recombinant nucleotide sequence encoding a PDE11 enzyme.

**25.** A PDE11 enzyme substantially as described herein.

FIGURE 1

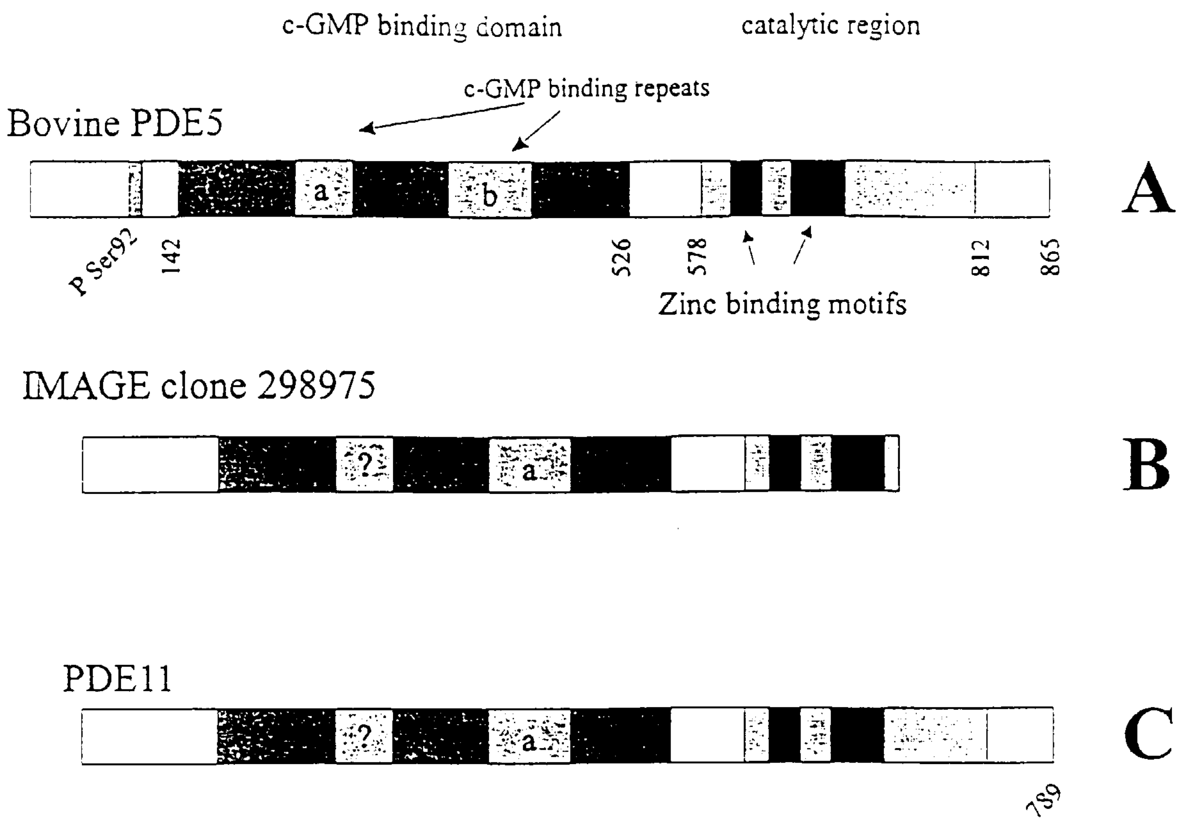
c-GMP binding domain
catalytic region
c-GMP binding repeats
Bovine PDE5
a
b
A
P Ser92
142
526
578
812
865
Zinc binding motifs
IMAGE clone 298975
?
a
B
PDE11
?
a
C
789

FIGURE 2

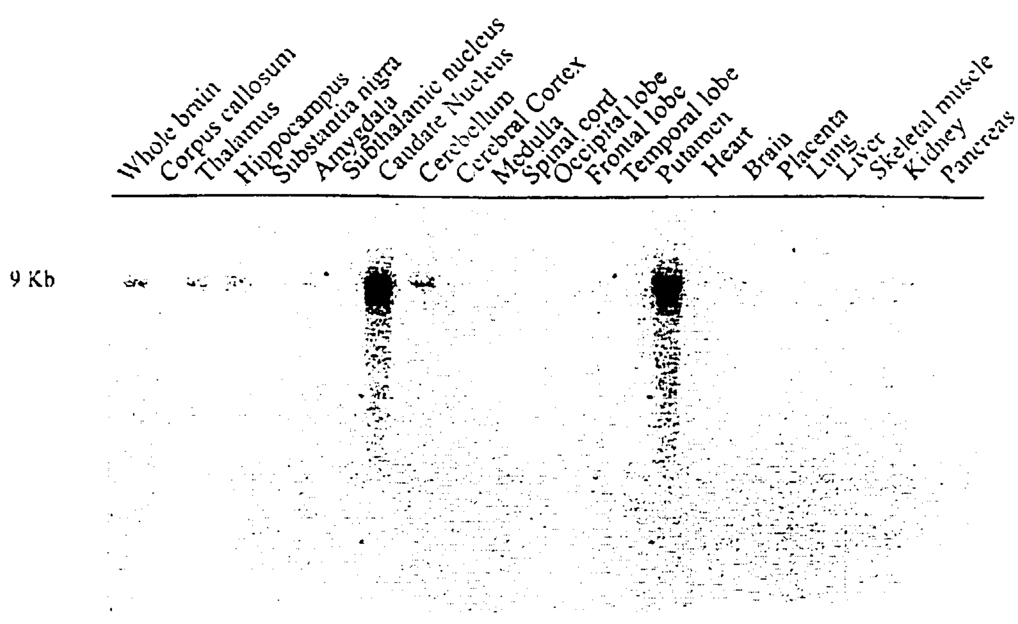
Whole brain
Corpus callosum
Thalamus
Hippocampus
Substantia nigra
Amygdala
Subthalamic nucleus
Caudate Nucleus
Cerebellum
Cerebral Cortex
Medulla
Spinal cord
Occipital lobe
Frontal lobe
Temporal lobe
Putamen
Heart
Brain
Placenta
Lung
Liver
Skeletal muscle
Kidney
Pancreas
9 Kb

FIGURE 3

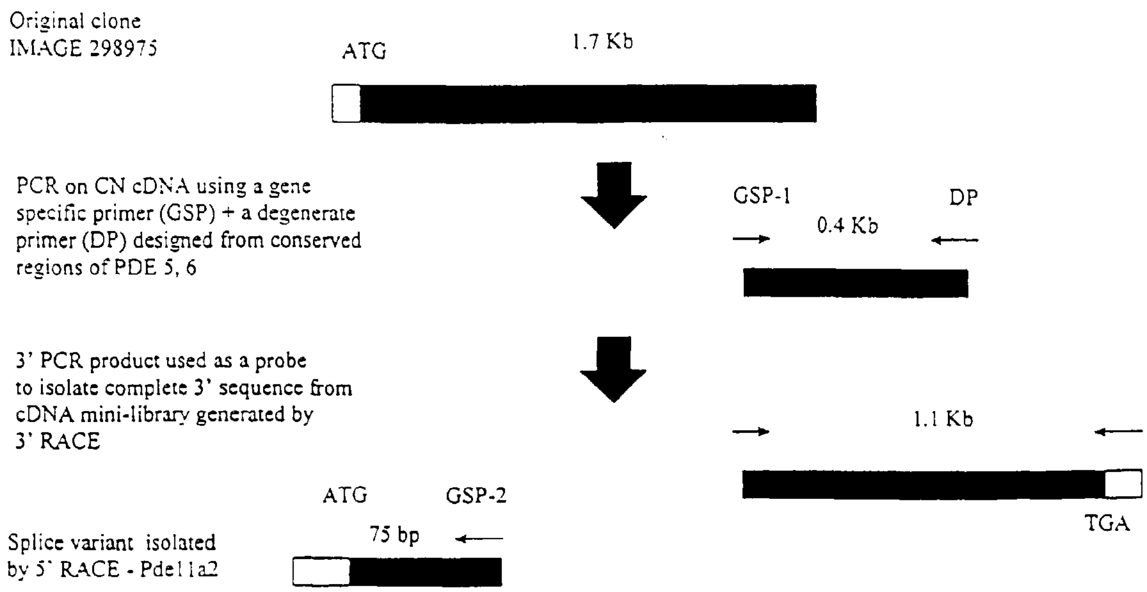
Original clone
IMAGE 298975
ATG
1.7 Kb
PCR on CN cDNA using a gene specific primer (GSP) + a degenerate primer (DP) designed from conserved regions of PDE 5, 6
GSP-1
DP
0.4 Kb
3' PCR product used as a probe to isolate complete 3' sequence from cDNA mini-library generated by 3' RACE
1.1 Kb
TGA
ATG
GSP-2
75 bp
Splice variant isolated by 5' RACE - Pde11a2

# FIGURE 4

Figure 4A
SEQ ID No.1

TTCGGCTCCGACATGGAAGATGGACCTTCTAATAATGCGAGCTGCTTCCGAAGGCTGACC 60
GAGTGCTTCCTGAGCCCCAGTTTGACAGATGAAAAAGTGAAGGCATATCTTTCTCTTCAC 120
CCCCAGGTATTAGATGAATTTGTATCTGAAAGTGTTAGTGCAGAGACAGTAGAGAAATGG 180
CTGAAGAGGAAGAACAACAAATCAGAAGATGAATCAGCTCCTAAGGAAGTCAGCAGGTAC 240
CAAGATACGAATATGCAGGGAGTTGTATATGAACTAAACAGCTATATAGAACAACGGTTG 300
GACACAGGAGGAGACAACCAGCTACTCCTCTATGAACTGAGCAGCATCATTAAAATAGCC 360
ACAAAAGCCGATGGATTTGCACTGTATTTCCTTGGAGAGTGCAATAATAGCCTGTGTATA 420
TTCACGCCACCTGGGATAAAGGAAGGAAAACCCCGCCTCATCCCTGCTGGGCCCATCACT 480
CAGGGCACCACCGTCTCTGCTTATGTGGCCAAGTCCAGGAAAACACTGCTAGTAGAAGAC 540
ATCCTTGGAGATGAACGATTTCCAAGAGGTACTGGACTGGAATCAGGGACTCGTATCCAG 600
TCTGTTCTTTGCTTACCAATTGTCACTGCAATTGGTGACTTGATTGGTATTCTCGAGCTG 660
TATCGGCACTGGGGCAAAGAAGCCTTCTGTCTTAGTCACCAGGAGGTTGCAACAGCAAAT 720
CTTGCCTGGGCTTCAGTAGCAATACATCAGGTGCAGGTATGCAGAGGCCTTGCCAAACAG 780
ACAGAATTGAATGACTTCCTACTCGACGTATCAAAAACATATTTGATAACATAGTTGCA 840
ATAGATTCTCTACTTGAACACATAATGATATATGCAAAAAACCTGGTGAATGCCGATCGT 900
TGTGCACTTTTCCAGGTGGACCATAAGAACAAGGAGTTATATTCAGACCTTTTTGATATT 960
GGAGAGGAAAAGGAAGGAAAACCTGTCTTCAAGAAGACCAAAGAGATAAGATTTTCAATT 1020
GAGAAAGGAATTGCTGGCCAAGTAGCAAGAACAGGGGAAGTCCTGAACATTCCAGATGCC 1080
TATGCAGACCCACGCTTTAACAGAGAAGTAGACTTGTACACAGGCTACACCACGCGGAAC 1140
ATCCTGTGCATGCCCATCGTCAGCCGAGGCAGCGTGATAGGTGTGGTGCAGATGGTCAAC 1200
AAAATCAGTGGCAGTGCCTTCTCTAAAACAGATGAAAACAACTTCAAAATGTTTGCCGTC 1260
TTTTGTGCTTTAGCCTTACACTGTGCTAATATGTATCATAGAATTCGCCACTCAGAGTGC 1320
ATTTACCGGGTAACGATGGAAAAGCTGTCCTACCATAGCATTTGTACTTCAGAAGAGTGG 1380
CAAGGTCTCATGCAATTCACCCTTCCCGTGCGTCTCTGCAAAGAAATTGAATTATTCCAC 1440
TTTGACATTGGTCCTTTTGAAAACATGTGGCCTGGAATTTTTGTCTACATGGTTCATCGG 1500
TCCTGTGGGACATCCTGCTTTGAGCTTGAAAAGTTGTGTCGTTTTATTATGTCTGTGAAG 1560
AAGAACTATCGGCGGGTTCCTTATCACAACTGGAAGCATGCGGTCACTGTAGCACACTGC 1620
ATGTATGCCATACTTCAGAACAATCACACGCTTTTCACAGACCTTGAGCGCAAAGGACTG 1680
CTGATTGCGTGTCTGTGTCATGACCTGGACCACAGGGGCTTCAGTAACAGCTACCTGCAG 1740
AAGTTCGACCACCCTCTGACCGCTCTCTACTCCACTTCCACCATGGAGCAGCACCACTTC 1800
TCCCAGACTGTGTCCATCCTTCAGTTGGAAGGGCACAATATCTTCTCCACTCTGAGCTCC 1860
AGTGAATATGAGCAGGTGCTTGAGATCATCCGCAAAGCCATCATTGCCACAGACCTTGCT 1920
TTATACTTTGGAAACAGGAAGCAGTTGGAAGAGATGTACCAGACCGGATCACTAAACCTT 1980
AATAATCAATCACATAGAGACCGTGTAATTGGTTTGATGATGACTGCCTGTGACCTTTGT 2040
TCTGTGACAAAACCGTGGCCCGTTACAAAATTGACGGCAAATGATATATATGCAGAATTC 2100
TGGGCTGAGGGTGATGAAATGAAGAAATTGGGAATACAGCCTATTCCTATGATGGACAGA 2160
GACAAGAAGGATGAAGTCCCCCAAGGCCAGCTTGGGTTCTACAATGCCGTGGCCATTCCC 2220
TGCTATACAACCCTTACCCAGATCCTCCCTCCCACGGAGCCTCTTCTGAAAGCATGCAGG 2280
GATAATCTCAGTCAGTGGGAGAAGGTGATTCGAGGGGAGGAGACTGCAACCTGGATTTCA 2340
TCCCCATCCGTGGCTCAGAAGGCAGCTGCATCTGAAGATTGAGCACTGGTCACCCTGACA 2400
CGCTGTCCCACCTACAGATCCTCATCTTGCTTCTTTGACATTCTTTTCCTTTTTTGGGGG 2460
GGGTGGGGGGAACCTGCACCTGGTAACTGGGGTGCAAACCTCTTCAAGAAGGTAACATCA 2520
AATAAATAAGTCAAGCAGAAAAAAAAAAAAAAAAA 2557

FIGURE 4 Continued

Figure 4B

SEQ ID No.2

MEDGPSNNASCFRRLTECFLSPSLTDEKVKAYLSLHPQVLDEFVSESVSAETVEKWLKRK 60
NNKSEDESAPKEVSRYQDTNMQGVVYELNSYIEQRLDTGGDNQLLLYELSSIIKIATKAD 120
GFALYFLGECNNSLCIFTPPGIKEGKPRLIPAGPITQGTTVSAYVAKSRKTLLVEDILGD 180
ERFPRGTGLESGTRIQSVLCLPIVTAIGDLIGILELYRHWGKEAFCLSHQEVATANLAWA 240
SVAIHQVQVCRGLAKQTELNDFLLDVSKTYFDNIVAIDSLLEHIMIYAKNLVNADRCALF 300
QVDHKNKELYSDLFDIGEEKEGKPVFKKTKEIRFSIEKGIAGQVARTGEVLNIPDAYADP 360
RFNREVDLYTGYTTRNILCMPIVSRGSVIGVVQMVNKISGSAFSKTDENNFKMFAVFCAL 420
ALHCANMYHRIRHSECIYRVTMEKLSYHSICTSEEWQGLMQFTLPVRLCKEIELFHFDIG 480
PFENMWPGIFVYMVHRSCGTSCFELEKLCRFIMSVKKNYRRVPYHNWKHAVTVAHCMYAI 540
LQNNHTLFTDLERKGLLIACLCHDLDHRGFSNSYLQKFDHPLTALYSTSTMEQHHFSQTV 600
SILQLEGHNIFSTLSSSEYEQVLEIIRKAIIATDLALYFGNRKQLEEMYQTGSLNLNNQS 660
HRDRVIGLMMTACDLCSVTKPWPVTKLTANDIYAEFWAEGDEMKKLGIQPIPMMDRDKKD 720
EVPQGQLGFYNAVAIPCYTTLTQILPPTEPLLKACRDNLSQWEKVIRGEETATWISSPSV 780
AQKAAASED 789

# FIGURE 5

Figure 5A
SEQ ID No.3

ACATAGCTGGGTGCAATGTAAGTGCCTGGCTGAAGTTTGACACGCGAACGGACGGCCCGC 60
TGGAATTCTGTGCTATGAGCCGGAGTAGAAAGAGAGATTTGGACTCTGCAACACCAAGGT 120
AGTCGTTGAAGCCACAGTCGTGAATGGAGACCAGGAGTGAATAGTGGGAGTGAGCAGAAG 180
TCGGAGGATAGGACAGAAGAAGGCAGAGCCATGGAGCACCCTGGAGAGGTGTGACCCGGC 240
AAGATCCTGAGATGGAAGGTAGCACGGCCTGGAGTTCAGAAGCGGAGCCTCAAGAGGGAA 300
GAAGCCAGATGCTCCAGAGAGCAGGTTTGACAGATGAAAAAGTGAAGGCATATCTTTCTC 360
TTCACCCCCAGGTATTAGATGAATTTGTATCTGAAAGTGTTAGTGCAGAGACAGTAGAGA 420
AATGGCTGAAGAGGAAGAACAACAAATCAGAAGATGAATCAGCTCCTAAGGAAGTCAGCA 480
GGTACCAAGATACGAATATGCAGGGAGTTGTATATGAACTAAACAGCTATATAGAACAAC 540
GGTTGGACACAGGAGGAGACAACCAGCTACTCCTCTATGAACTGAGCAGCATCATTAAAA 600
TAGCCACAAAAGCCGATGGATTTGCACTGTATTTCCTTGGAGAGTGCAATAATAGCCTGT 660
GTATATTCACGCCACCTGGGATAAAGGAAGGAAAACCCCGCCTCATCCCTGCTGGGCCCA 720
TCACTCAGGGCACCACCGTCTCTGCTTATGTGGCCAAGTCCAGGAAAACACTGCTAGTAG 780
AAGACATCCTTGGAGATGAACGATTTCCAAGAGGTACTGGACTGGAATCAGGGACTCGTA 840
TCCAGTCTGTTCTTTGCTTACCAATTGTCACTGCAATTGGTGACTTGATTGGTATTCTCG 900
AGCTGTATCGGCACTGGGGCAAAGAAGCCTTCTGTCTTAGTCACCAGGAGGTTGCAACAG 960
CAAATCTTGCCTGGGCTTCAGTAGCAATACATCAGGTGCAGGTATGCAGAGGCCTTGCCA 1020
AACAGACAGAATTGAATGACTTCCTACTCGACGTATCAAAAACATATTTTGATAACATAG 1080
TTGCAATAGATTCTCTACTTGAACACATAATGATATATGCAAAAAACCTGGTGAATGCCG 1140
ATCGTTGTGCACTTTTCCAGGTGGACCATAAGAACAAGGAGTTATATTCAGACCTTTTTG 1200
ATATTGGAGAGGAAAAGGAAGGAAAACCTGTCTTCAAGAAGACCAAAGAGATAAGATTTT 1260
CAATTGAGAAAGGAATTGCTGGCCAAGTAGCAAGAACAGGGGAAGTCCTGAACATTCCAG 1320
ATGCCTATGCAGACCCACGCTTTAACAGAGAAGTAGACTTGTACACAGGCTACACCACGC 1380
GGAACATCCTGTGCATGCCCATCGTCAGCCGAGGCAGCGTGATAGGTGTGGTGCAGATGG 1440
TCAACAAAATCAGTGGCAGTGCCTTCTCTAAAACAGATGAAAACAACTTCAAAATGTTTG 1500
CCGTCTTTTGTGCTTTAGCCTTACACTGTGCTAATATGTATCATAGAATTCGCCACTCAG 1560
AGTGCATTTACCGGGTAACGATGGAAAAGCTGTCCTACCATAGCATTTGTACTTCAGAAG 1620
AGTGGCAAGGTCTCATGCAATTCACCCTTCCCGTGCGTCTCTGCAAAGAAATTGAATTAT 1680
TCCACTTTGACATTGGTCCTTTTGAAAACATGTGGCCTGGAATTTTTGTCTACATGGTTC 1740
ATCGGTCCTGTGGGACATCCTGCTTTGAGCTTGAAAAGTTGTGTCGTTTTATTATGTCTG 1800
TGAAGAAGAACTATCGGCGGGTTCCTTATCACAACTGGAAGCATGCGGTCACTGTAGCAC 1860
ACTGCATGTATGCCATACTTCAGAACAATCACACGCTTTTCACAGACCTTGAGCGCAAAG 1920
GACTGCTGATTGCGTGTCTGTGTCATGACCTGGACCACAGGGGCTTCAGTAACAGCTACC 1980
TGCAGAAGTTCGACCACCCTCTGACCGCTCTCTACTCCACTTCCACCATGGAGCAGCACC 2040
ACTTCTCCCAGACTGTGTCCATCCTTCAGTTGCAAGGGCACAATATCTTCTCCACTCTGA 2100
GCTCCAGTGAATATGAGCAGGTGCTTGAGATCATCCGCAAAGCCATCATTGCCACAGACC 2160
TTGCTTTATACTTTGGAAACAGGAAGCAGTTGGAAGAGATGTACCAGACCGGATCACTAA 2220
ACCTTAATAATCAATCACATAGAGACCGTGTAATTGGTTTGATGATGACTGCCTGTGACC 2280
TTTGTTCTGTGACAAAACCGTGGCCCGTTACAAAATTGACGGCAAATGATATATATGCAG 2340
AATTCTGGGCTGAGGGTGATGAAATGAAGAAATTGGGAATACAGCCTATTCCTATGATGG 2400
ACAGAGACAAGAAGGATGAAGTCCCCCAAGGCCAGCTTGGGTTCTACAATGCCGTGGCCA 2460
TTCCCTGCTATACAACCCTTACCCAGATCCTCCCTCCCACGGAGCCTCTTCTGAAAGCAT 2520
GCAGGGATAATCTCAGTCAGTGGGAGAAGGTGATTCGAGGGGAGGAGACTGCAACCTGGA 2580
TTTCATCCCCATCCGTGGCTCAGAAGGCAGCTGCATCTGAAGATTGAGCACTGGTCACCC 2640
TGACACGCTGTCCCACCTACAGATCCTCATCTTGCTTCTTTGACATTCTTTTCCTTTTTT 2700
GGGGGGGGTGGGGGGAACCTGCACCTGGTAACTGGGGTGCAAACCTCTTCAAGAAGGTAA 2760
CATCAAATAAATAAGTCAAGCAGAAAAAAAAAAAAAAAA 2799

FIGURE 5 Continued

Figure 5B

SEQ ID No.4

MEGSTAWSSEAEPQEGRSQMLQRAGLTDEKVKAYLSLHPQVLDEFVSESVSAETVEKWLK 60

RKNNKSEDESAPKEVSRYQDTNMQGVVYELNSYIEQRLDTGGDNQLLLYELSSIIKIATK 120

ADGFALYFLGECNNSLCIFTPPGIKEGKPRLIPAGPITQGTTVSAYVAKSRKTLLVEDIL 180

GDERFPRGTGLESGTRIQSVLCLPIVTAIGDLIGILELYRHWGKEAFCLSHQEVATANLA 240

WASVAIHQVQVCRGLAKQTELNDFLLDVSKTYFDNIVAIDSLLEHIMIYAKNLVNADRCA 300

LFQVDHKNKELYSDLFDIGEEKEGKPVFKKTKEIRFSIEKGIAGQVARTGEVLNIPDAYA 360

DPRFNREVDLYTGYTTRNILCMPIVSRGSVIGVVQMVNKISGSAFSKTDENNFKMFAVFC 420

ALALHCANMYHRIRHSECIYRVTMEKLSYHSICTSEEWQGLMQFTLPVRLCKEIELFHFD 480

IGPFENMWPGIFVYMVHRSCGTSCFELEKLCRFIMSVKKNYRRVPYHNWKHAVTVAHCMY 540

AILQNNHTLFTDLERKGLLIACLCHDLDHRGFSNSYLQKFDHPLTALYSTSTMEQHHFSQ 600

TVSILQLEGHNIFSTLSSSEYEQVLEIIRKAIIATDLALYFGNRKQLEEMYQTGSLNLNN 660

QSHRDRVIGLMMTACDLCSVTKPWPVTKLTANDIYAEFWAEGDEMKKLGIQPIPMMDRDK 720

KDEVPQGQLGFYNAVAIPCYTTLTQILPPTEPLLKACRDNLSQWEKVIRGEETATWISSP 780

SVAQKAAASED 791

FIGURE 5 Continued

Figure 5C

PDE11A1

MEDGPSNNASCFRRLTECFLSPSLTDEKVKAYLSLHPQVLDEFVSESVSAETVEKWLKRKNNKSEDESAPKEV

+++++++++++++++++++++++++++++++++++++++++++++++++

PDE11A2

MEGSTAWSSEAEPQEGRSQMLQRAGLTDEKVKAYLSLHPQVLDEFVSESVSAETVEKWLKRKNNKSEDESAPKEV

FIGURE 6

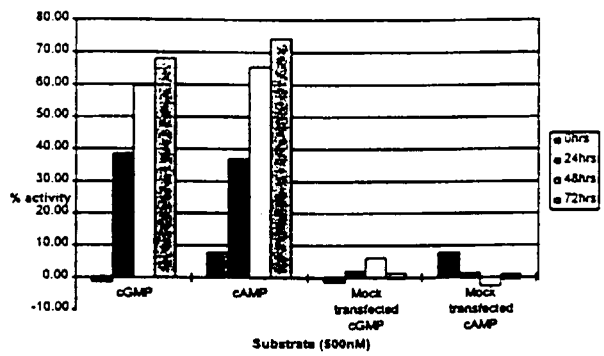
80.00
70.00
60.00
50.00
40.00
30.00
% activity
20.00
10.00
0.00
-10.00
cGMP
cAMP
Mock transfected cGMP
Mock transfected cAMP
Substrate (500nM)
24hrs
48hrs
72hrs

FIGURE 7

7A

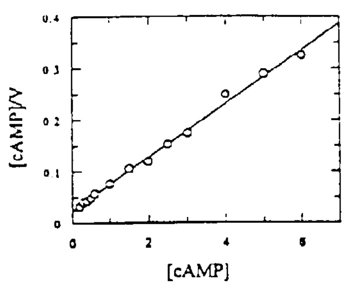


| Variable | Value | Std. Err. |
|---|---|---|
| Intercept | 0.0238 | 0.0029 (Km/Vmax) |
| Slope | 0.0521 | 0.0011 (1/Vmax) |
| Km | 0.456 | |

7B

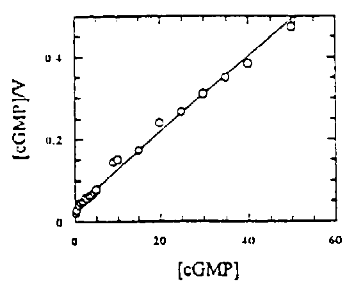


| Variable | Value | Std. Err. |
|---|---|---|
| Intercept | 0.0360 | 0.0038 (Km/Vmax) |
| Slope | 0.0090 | 0.0002 (1/Vmax) |
| Km | 4.0 | |

FIGURE 8

FIGURE 8

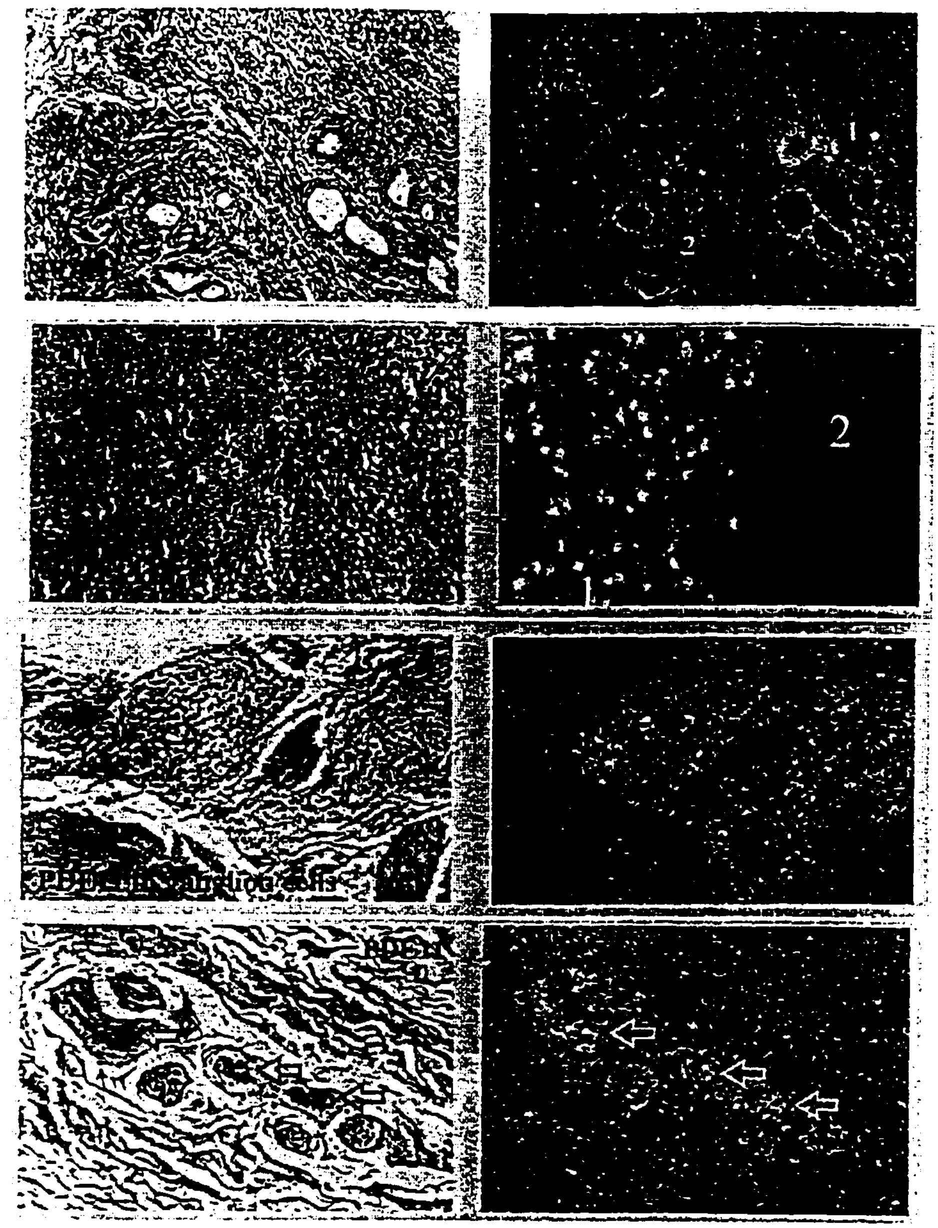

A

B

C

D

FIGURE 9

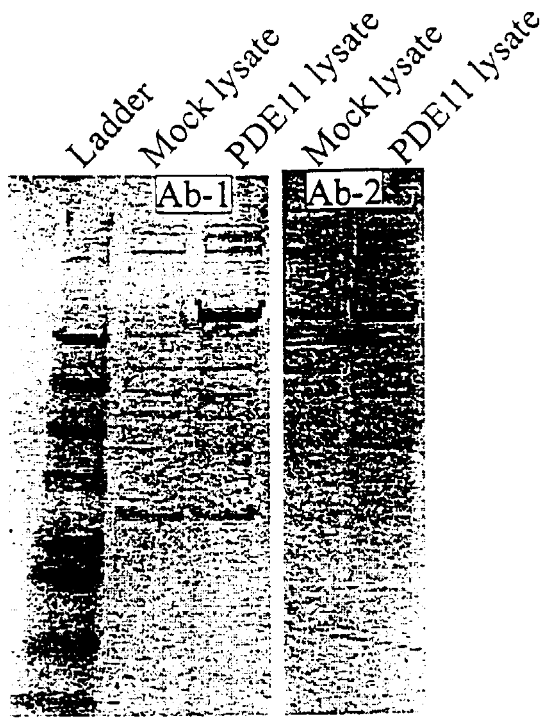
Ladder
Mock lysate
PDE11 lysate
Mock lysate
PDE11 lysate
Ab-1
Ab-2

Figure 10

```
Length = 789
Score = 1527 (709.8 bits), Expect = 1.7e-213, P = 1.7e-213
Identities = 292/300 (97%), Positives = 298/300 (99%)

Query:    1 FLGECNNSLCVFIPPGMKEGQPRLIPAGPITQGTTISAYVAKSRKTLLVEDILGDERFPR 60
            FLGECNNSLC+F PPG+KEG+PRLIPAGPITQGTT+SAYVAKSRKTLLVEDILGDERFPR
Sbjct:  126 FLGECNNSLCIFTPPGIKEGKPRLIPAGPITQGTTVSAYVAKSRKTLLVEDILGDERFPR 185

Query:   61 GTGLESGTRIQSVLCLPIVTAIGDLIGILELYRHWDKEAFCLSHQEVATANLAWASVAIH 120
            GTGLESGTRIQSVLCLPIVTAIGDLIGILELYRHW KEAFCLSHQEVATANLAWASVAIH
Sbjct:  186 GTGLESGTRIQSVLCLPIVTAIGDLIGILELYRHWGKEAFCLSHQEVATANLAWASVAIH 245

Query:  121 QVQVCRGLAKQTELNDFLLDVSKTYFDNIVAIDSLLEHIIIYAKNLVNADRCALFQVDHK 180
            QVQVCRGLAKQTELNDFLLDVSKTYFDNIVAIDSLLEHI+IYAKNLVNADRCALFQVDHK
Sbjct:  246 QVQVCRGLAKQTELNDFLLDVSKTYFDNIVAIDSLLEHIMIYAKNLVNADRCALFQVDHK 305

Query:  181 NKELYSDLFDIGEEKEGKPIFKKTKEIRFSIEKGIAGQVARTGEVLNIPDAYADPRFNRE 240
            NKELYSDLFDIGEEKEGKP+FKKTKEIRFSIEKGIAGQVARTGEVLNIPDAYADPRFNRE
Sbjct:  306 NKELYSDLFDIGEEKEGKPVFKKTKEIRFSIEKGIAGQVARTGEVLNIPDAYADPRFNRE 365

Query:  241 VDLYTGYTTRNILCMPIVSRGSVIGVVQMVNKISGSAFSKTDENNFKMFAVFCALALHCA 300
            VDLYTGYTTRNILCMPIVSRGSVIGVVQMVNKISGSAFSKTDENNFKMFAVFCALALHCA
Sbjct:  366 VDLYTGYTTRNILCMPIVSRGSVIGVVQMVNKISGSAFSKTDENNFKMFAVFCALALHCA 425
```

FIGURE 11

```
HumanPDE11A1_      ME--DGPSNNASCFRRLTECFLSPSLTDEKVKAYLSLHPQVLDEFVSESVSAETVEKWLK
HumanPDE11A2_      MEGSTAWSSEAEPQEGRSQMLQRAGLTDEKVKAYLSLHPQVLDEFVSESVSAETVEKWLK
MousePDE11A3_      ME--DGPSNNASCFRRLTECFLSPSLTDEKVKAYLSLHPQVLDEFVSESVSAETVEKWLK
                   **   . *.:*.  .  :: :  ..***********************************

HumanPDE11A1_      RKNNKSEDESAPKEVSRYQDTNMQGVVYELNSYIEQRLDTGGDNQLLLYELSSIIKIATK
HumanPDE11A2_      RKNNKSEDESAPKEVSRYQDTNMQGVVYELNSYIEQRLDTGGDNQLLLYELSSIIKIATK
MousePDE11A3_      RKTNKAKDEPSPKEVSRYQDTNMQGVVYELNSYIEQRLDTGGDNHLLLYELSSIIRIATK
                   **.**::**.:*********************************:**********:****

HumanPDE11A1_      ADGFALYFLGECNNSLCIFTPPGIKEGKPRLIPAGPITQGTTVSAYVAKSRKTLLVEDIL
HumanPDE11A2_      ADGFALYFLGECNNSLCIFTPPGIKEGKPRLIPAGPITQGTTVSAYVAKSRKTLLVEDIL
MousePDE11A3_      ADGFALYFLGECNNSLCVFIPPGMKEGQPRLIPAGPITQGTTISAYVAKSRKTLLVEDIL
                   *****************:* ***:***:**************:*****************

HumanPDE11A1_      GDERFPRGTGLESGTRIQSVLCLPIVTAIGDLIGILELYRHWGKEAFCLSHQEVATANLA
HumanPDE11A2_      GDERFPRGTGLESGTRIQSVLCLPIVTAIGDLIGILELYRHWGKEAFCLSHQEVATANLA
MousePDE11A3_      GDERFPRGTGLESGTRIQSVLCLPIVTAIGDLIGILELYRHWGKEAFCLSHQEVATANLA
                   ************************************************************

HumanPDE11A1_      WASVAIHQVQVCRGLAKQTELNDFLLDVSKTYFDNIVAIDSLLEHIMIYAKNLVNADRCA
HumanPDE11A2_      WASVAIHQVQVCRGLAKQTELNDFLLDVSKTYFDNIVAIDSLLEHIMIYAKNLVNADRCA
MousePDE11A3_      WASVAIHQVQVCRGLAKQTELNDFLLDVSKTYFDNIVAIDSLLEHIMIYAKNLVNADRCA
                   ************************************************************

HumanPDE11A1_      LFQVDHKNKELYSDLFDIGEEKEGKPVFKKTKEIRFSIEKGIAGQVARTGEVLNIPDAYA
HumanPDE11A2_      LFQVDHKNKELYSDLFDIGEEKEGKPVFKKTKEIRFSIEKGIAGQVARTGEVLNIPDAYA
MousePDE11A3_      LFQVDHKNKELYSDLFDIGEEKEGKPIFKKTKEIRFSIEKGIAGQVARTGEVLNIPDAYA
                   **************************:*********************************

HumanPDE11A1_      DPRFNREVDLYTGYTTRNILCMPIVSRGSVIGVVQMVNKISGSAFSKTDENNFKMFAVFC
HumanPDE11A2_      DPRFNREVDLYTGYTTRNILCMPIVSRGSVIGVVQMVNKISGSAFSKTDENNFKMFAVFC
MousePDE11A3_      DPRFNREVDLYTGYTTRNILCMPIVSRGSVIGVVQMVNKISGSAFSKTDENNFKMFAVFC
                   ************************************************************

HumanPDE11A1_      ALALHCANMYHRIRHSECIYRVTMEKLSYHSICTSEEWQGLMQFTLPVRLCKEIELFHFD
HumanPDE11A2_      ALALHCANMYHRIRHSECIYRVTMEKLSYHSICTSEEWQGLMQFTLPVRLCKEIELFHFD
MousePDE11A3_      ALALHCANMYHRIRHSECIYRVTMEKLSYHSICTSEEWQGLMRFNLPARICRDIELFHFD
                   ******************************************:*.**.*:*::*******

HumanPDE11A1_      IGPFENMWPGIFVYMVHRSCGTSCFELEKLCRFIMSVKKNYRRVPYHNWKHAVTVAHCMY
HumanPDE11A2_      IGPFENMWPGIFVYMVHRSCGTSCFELEKLCRFIMSVKKNYRRVPYHNWKHAVTVAHCMY
MousePDE11A3_      IGPFENMWPGIFVYMIHRSCGTSCFELEKLCRFIMSVKKNYRRVPYHNWKHAVTVAHCMY
                   ***************:********************************************

HumanPDE11A1_      AILQNNHTLFTDLERKGLLIACLCHDLDHRGFSNSYLQKFDHPLTALYSTSTMEQHHFSQ
HumanPDE11A2_      AILQNNHTLFTDLERKGLLIACLCHDLDHRGFSNSYLQKFDHPLTALYSTSTMEQHHFSQ
MousePDE11A3_      AILQNNNGLFTDLERKGLLIACLCHDLDHRGFSNSYLQKFDHPLAALYSTSTMEQHHFSQ
                   ******: ************************************:***************

HumanPDE11A1_      TVSILQLEGHNIFSTLSSSEYEQVLEIIRKAIIATDLALYFGNRKQLEEMYQTGSLNLNN
HumanPDE11A2_      TVSILQLEGHNIFSTLSSSEYEQVLEIIRKAIIATDLALYFGNRKQLEEMYQTGSLNLNN
MousePDE11A3_      TVSILQLEGHNIFSTLSSSEYEQVLEIIRKAIIATDLALYFGNRKQLEEMYQTGSLNLHN
                   **********************************************************:*

HumanPDE11A1_      QSHRDRVIGLMMTACDLCSVTKPWPVTKLTANDIYAEFWAEGDEMKKLGIQPIPMMDRDK
HumanPDE11A2_      QSHRDRVIGLMMTACDLCSVTKPWPVTKLTANDIYAEFWAEGDEMKKLGIQPIPMMDRDK
MousePDE11A3_      QSHRDRVIGLMMTACDLCSVTKLWPVTKLTANDIYAEFWAEGDEMKKLGIQPIPMMDRDK
                   ********************** *************************************

HumanPDE11A1_      KDEVPQGQLGFYNAVAIPCYTTLTQILPPTEPLLKACRDNLSQWEKVIRGEETATWISSP
HumanPDE11A2_      KDEVPQGQLGFYNAVAIPCYTTLTQILPPTEPLLKACRDNLSQWEKVIRGEETATWISSP
MousePDE11A3_      RDEVPQGQLGFYNAVAIPCYTTLTQILPPTEPLLKACRDNLNQWEKVIRGEETAMWISGP
                   :****************************************.************ ***.*
HumanPDE11A1_      SVAQKAAASED-------
HumanPDE11A2_      SVAQKAAASED-------
MousePDE11A3_      GPAPSKSTPEKLNVKVED
                   . * . ::.*.
```

European Patent Office

# PARTIAL EUROPEAN SEARCH REPORT

which under Rule 45 of the European Patent Convention shall be considered, for the purposes of subsequent proceedings, as the European search report

Application Number
EP 99 30 3985

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,X | WO 99 19495 A (ICOS CORPORATION (US); LOUGHNEY KATE (US)) 22 April 1999 (1999-04-22) * the whole document * --- | 1-10, 12-25 | C12N15/55 C12N9/16 G01N33/68 C12Q1/44 C07K16/40 C12N15/11 A61K38/46 A61K35/00 A61K48/00 |
| X | Nucleotide Sequence Database EMBL Entry HS835337; Accession number W04835 8 May 1996 96% identity with Seq.ID:1 nt.1338-1763 96% identity with Seq.ID:3 nt.1583-2008 84% identity with Seq.ID:14 nt.1783-2208 reverse orientation XP002119752 | 1-9, 15-17, 23-25 | |
| Y | * the whole document * --- | 10-14, 18-22 | |
| Y | WO 97 03675 A (LABORATOIRE GLAXO WELLCOME S.A. (FR); DAUGAN ALAIN CLAUDE-MARIE (FR)) 6 February 1997 (1997-02-06) * page 1, line 31 - page 3, line 29 * * page 4, line 17-24 * * page 16, line 10 - page 17, line 27 * * page 18 - page 22; claims * --- -/-- | 10-14, 18-22 | |

TECHNICAL FIELDS SEARCHED (Int.Cl.6)

C12N
G01N
C12Q
C07K
A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 21 October 1999 | Macchia, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 99 30 3985

Remark: Although claim 13 is directed to a method of treatment of the human/animal body (Article 52(4) EPC), the search has been carried out and based on the alleged effects of the compound/composition.

--------------------

Claim(s) searched completely:
4, 5, 15-20, 23-25

Claim(s) searched incompletely:
1-3, 6-14, 21, 22

Reason for the limitation of the search:

Present claims 1, 2 and 3, 6-14, 21, 22 (as far as they refer to claims 1, 2), relate to an extremely large number of possible aminoacid sequences. In fact, Formula I, as it is disclosed on pages 6-15, contains so many variables, that a lack of clarity (and conciseness) within the meaning of Article 84 EPC arises to such an extent as to render a meaningful search of the claims impossible. Consequently, the search has been carried out for those parts of the application which do appear to be clear (and concise), namely those compounds whose nucleotide sequence is disclosed as Seq.ID:1, 3, 14, the corresponding encoded polypeptides, and closely related homologous compounds.

Present claims 21, 22 relate to an extremely large number of possible compounds. Support within the meaning of Article 84 EPC and/or disclosure within the meaning of Article 83 EPC is to be found, however, for only a very small proportion of the compounds claimed. In the present case, the claims so lack support, and the application so lacks disclosure, that a meaningful search over the whole of the claimed scope is impossible. Consequently, the search has been carried out for those parts of the claims which appear to be supported and disclosed, namely those parts relating to peptides derived from the disclosed polypeptides, specific antibodies and antisense molecules.

European Patent Office

# PARTIAL EUROPEAN SEARCH REPORT

Application Number
EP 99 30 3985

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | WO 94 28902 A (PFIZER LTD (GB) PFIZER INC (US) PFIZER RES & DEV CO NV/SA (IE); ELLIS) 22 December 1994 (1994-12-22)<br>* page 2, paragraph 2 *<br>* page 7, paragraph 3 *<br>* page 8, paragraph 2 - page 10, line 4 *<br>* page 13 - page 17; claims *<br>--- | 10-14, 18-22 | |
| A,D | WO 97 35989 A (INCYTE PHARMA INC (US) AU-YOUNG J; COCKS B.G; COLEMAN R; SEILHAMER J.J) 2 October 1997 (1997-10-02)<br>* abstract *<br>* page 21, line 27 - page 23, line 18 *<br>* page 24, line 29 - page 27, line 1 *<br>* page 43 - page 44; claims *<br>--- | 10-14, 18-22 | TECHNICAL FIELDS SEARCHED (Int.Cl.6) |
| X | Nucleotide Sequence Database EMBL<br>Entry AA973152; Accession number AA973152<br>21 May 1998<br>98% identity with Seq.ID:1 nt.2186-2539<br>98% identity with Seq.ID:3 nt.2431-2784<br>reverse orientation<br>XP002119753<br>* the whole document *<br>--- | 4,6-9 | |
| X | Nucleotide Sequence Database EMBL<br>Entry MM1276640<br>Accession number AA386789<br>25 June 1997<br>86% identity with Seq.ID:1 nt.360-833<br>86% identity with Seq.ID:3 nt.605-1078<br>99% identity with Seq.ID:14 nt.805-1278<br>XP002119754<br>* the whole document *<br>---<br>-/-- | 4,6-9 | |

EPO FORM 1503 03.82 (P04C10)

European Patent Office

# PARTIAL EUROPEAN SEARCH REPORT

Application Number
EP 99 30 3985

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | Nucleotide Sequence Database EMBL<br>Entry RS7341; Accession number H32734<br>6 April 1998<br>84% identity with Seq.ID:1 nt.853-1254<br>84% identity with Seq.ID:3 nt.1098-1499<br>94% identity with Seq.ID:14 nt.1298-1699<br>XP002119755<br>* the whole document *<br>--- | 6-9 | |
| A | BEAVO J.A.: "Cyclic Nucleotide Phosphodiesterases: functional implications of multiple isoforms"<br>PHYSIOLOGICAL REVIEWS,<br>vol. 75, no. 4,<br>1 October 1995 (1995-10-01), pages 725-748, XP002034532<br>ISSN: 0031-9333<br>* page 741, right-hand column, paragraph 4 - page 743 *<br>--- | 10-14, 18-22 | TECHNICAL FIELDS SEARCHED (Int.Cl.6) |
| A | LOUGHNEY K. AND FERGUSON K.: "Identification and quantification of PDE isoenzymes and subtypes by molecular biological methods"<br>PHOSPHODIESTERASE INHIBITORS,1996, pages 1-19, XP002105161<br>----- | | |

EPO FORM 1503 03.82 (P04C10)

## ANNEX TO THE EUROPEAN SEARCH REPORT ON EUROPEAN PATENT APPLICATION NO. EP 99 30 3985

This annex lists the patent family members relating to the patent documents cited in the above-mentioned European search report.
The members are as contained in the European Patent Office EDP file on
The European Patent Office is in no way liable for these particulars which are merely given for the purpose of information.

21-10-1999

| Patent document cited in search report | Publication date | Patent family member(s) | Publication date |
|---|---|---|---|
| WO 9919495 A | 22-04-1999 | US 5932465 A | 03-08-1999 |
| | | AU 1097499 A | 03-05-1999 |
| | | EP 0944725 A | 29-09-1999 |
| | | NO 992916 A | 06-08-1999 |
| WO 9703675 A | 06-02-1997 | AU 704955 B | 13-05-1999 |
| | | AU 6419196 A | 18-02-1997 |
| | | BR 9609758 A | 26-01-1999 |
| | | CA 2226784 A | 06-02-1997 |
| | | CN 1195290 A | 07-10-1998 |
| | | CZ 9800033 A | 13-05-1998 |
| | | EP 0839040 A | 06-05-1998 |
| | | HU 9900065 A | 28-05-1999 |
| | | JP 11509221 T | 17-08-1999 |
| | | NO 980153 A | 10-03-1998 |
| | | PL 324495 A | 25-05-1998 |
| | | SK 3998 A | 08-07-1998 |
| WO 9428902 A | 22-12-1994 | AT 163852 T | 15-03-1998 |
| | | AU 676571 B | 13-03-1997 |
| | | AU 6797394 A | 03-01-1995 |
| | | CA 2163446 A,C | 22-12-1994 |
| | | CN 1124926 A | 19-06-1996 |
| | | CZ 9503242 A | 17-07-1996 |
| | | DE 69408981 D | 16-04-1998 |
| | | DE 69408981 T | 02-07-1998 |
| | | DK 702555 T | 06-04-1998 |
| | | EP 0702555 A | 27-03-1996 |
| | | ES 2113656 T | 01-05-1998 |
| | | FI 955911 A | 08-12-1995 |
| | | GR 3026520 T | 31-07-1998 |
| | | IL 109873 A | 27-12-1998 |
| | | IL 121836 A | 27-12-1998 |
| | | JP 2925034 B | 26-07-1999 |
| | | JP 9503996 T | 22-04-1997 |
| | | LV 12269 A | 20-05-1999 |
| | | NO 954757 A | 24-11-1995 |
| | | NZ 266463 A | 24-03-1997 |
| | | PL 311948 A | 18-03-1996 |
| | | ZA 9404018 A | 08-12-1995 |
| WO 9735989 A | 02-10-1997 | AU 2542797 A | 17-10-1997 |
| | | CA 2248675 A | 02-10-1997 |
| | | EP 0907742 A | 14-04-1999 |

EPO FORM P0459

For more details about this annex : see Official Journal of the European Patent Office, No. 12/82

SEQUENCE LISTING

<110> Pfizer Limited
Pfizer Incorporated

<120> Phosphodiesterase Enzymes

<130> P004205EP CTH

<140> EP 99303985.8
<141> 1999-05-21

<150> GB 9811500.9
<151> 1998-05-28

<150> GB 9823882.7
<151> 1998-10-30

<150> GB 9826777.6
<151> 1998-12-04

<150> GB 9908247.1
<151> 1999-04-09

<150> GB 9910801.1
<151> 1999-05-10

<160> 47

<170> PatentIn Ver. 2.1

<210> 1
<211> 2554
<212> DNA
<213> Homo sapiens

<400> 1

ttcggctccg acatggaaga tggaccttct aataatgcga gctgcttccg aaggctgacc 60
gagtgcttcc tgagccccag tttgacagat gaaaaagtga aggcatatct ttctcttcac 120
ccccaggtat tagatgaatt tgtatctgaa agtgttagtg cagagacagt agagaaatgg 180
ctgaagagga agaacaacaa atcagaagat gaatcagctc ctaaggaagt cagcaggtac 240
caagatacga atatgcaggg agttgtatat gaactaaaca gctatataga acaacggttg 300
gacacaggag gagacaacca gctactcctc tatgaactga gcagcatcat taaaatagcc 360
acaaaagccg atggatttgc actgtatttc cttggagagt gcaataatag cctgtgtata 420
ttcacgccac ctgggataaa ggaaggaaaa ccccgcctca tccctgctgg gcccatcact 480
cagggcacca ccgtctctgc ttatgtggcc aagtccagga aaacactgct agtagaagac 540
atccttggag atgaacgatt tccaagaggt actggactgg aatcagggac tcgtatccag 600
tctgttcttt gcttaccaat tgtcactgca attggtgact tgattggtat tctcgagctg 660
tatcggcact ggggcaaaga agccttctgt cttagtcacc aggaggttgc aacagcaaat 720
cttgcctggg cttcagtagc aatacatcag gtgcaggtat gcagaggcct tgccaaacag 780
acagaattga atgacttcct actcgacgta tcaaaaacat attttgataa catagttgca 840
atagattctc tacttgaaca cataatgata tatgcaaaaa acctggtgaa tgccgatcgt 900
tgtgcacttt tccaggtgga ccataagaac aaggagttat attcagacct tttgatattt 960
ggagaggaaa aggaaggaaa acctgtcttc aagaagacca aagagataag attttcaatt 1020
gagaaaggaa ttgctggcca agtagcaaga acaggggaag tcctgaacat tccagatgcc 1080
tatgcagacc cacgctttaa cagagaagta gacttgtaca caggctacac cacgcggaac 1140
atcctgtgca tgcccatcgt cagccgaggc agcgtgatag gtgtggtgca gatggtcaac 1200
aaaatcagtg gcagtgcctt ctctaaaaca gatgaaaaca acttcaaaat gtttgccgtc 1260
ttttgtgctt tagccttaca ctgtgctaat atgtatcata gaattcgcca ctcagagtgc 1320
atttaccggg taacgatgga aaagctgtcc taccatagca tttgtacttc agaagagtgg 1380
caaggtctca tgcaattcac ccttcccgtg cgtctctgca aagaaattga attattccac 1440
tttgacattg gtccttttga aaacatgtgg cctggaattt ttgtctacat ggttcatcgg 1500
tcctgtggga catcctgctt tgagcttgaa aagttgtgtc gttttattat gtctgtgaag 1560
aagaactatc ggcgggttcc ttatcacaac tggaagcatg cggtcactgt agcacactgc 1620
atgtatgcca tacttcagaa caatcacacg cttttcacag accttgagcg caaaggactg 1680
ctgattgcgt gtctgtgtca tgacctggac cacaggggct tcagtaacag ctacctgcag 1740
aagttcgacc accctctgac cgctctctac tccacttcca ccatggagca gcaccacttc 1800
tcccagactg tgtccatcct tcagttggaa gggcacaata tcttctccac tctgagctcc 1860
agtgaatatg agcaggtgct tgagatcatc cgcaaagcca tcattgccac agaccttgct 1920
ttatactttg gaaacaggaa gcagttggaa gagatgtacc agaccggatc actaaacctt 1980
aataatcaat cacatagaga ccgtgtaatt ggtttgatga tgactgcctg tgacctttgt 2040
tctgtgacaa aaccgtggcc cgttacaaaa ttgacggcaa atgatatata tgcagaattc 2100
tgggctgagg gtgatgaaat gaagaaattg ggaatacagc ctattcctat gatggacaga 2160
gacaagaagg atgaagtccc ccaaggccag cttgggttct acaatgccgt ggccattccc 2220

```
tgctatacaa cccttaccca gatcctccct cccacggagc ctcttctgaa agcatgcagg 2280
gataatctca gtcagtggga gaaggtgatt cgaggggagg agactgcaac ctggatttca 2340
tccccatccg tggctcagaa ggcagctgca tctgaagatt gagcactggt caccctgaca 2400
cgctgtccca cctacagatc ctcatcttgc ttctttgaca ttcttttcct tttttggggg 2460
gggtgggggg aacctgcacc tggtaactgg ggtgcaaacc tcttcaagaa ggtaacatca 2520
aataaataag tcaagcagaa aaaaaaaaaa aaaa                             2554
```

<210> 2
<211> 789
<212> PRT
<213> Homo sapiens

<400> 2

```
Met Glu Asp Gly Pro Ser Asn Asn Ala Ser Cys Phe Arg Arg Leu Thr
 1               5                  10                  15

Glu Cys Phe Leu Ser Pro Ser Leu Thr Asp Glu Lys Val Lys Ala Tyr
             20                  25                  30

Leu Ser Leu His Pro Gln Val Leu Asp Glu Phe Val Ser Glu Ser Val
         35                  40                  45

Ser Ala Glu Thr Val Glu Lys Trp Leu Lys Arg Lys Asn Asn Lys Ser
     50                  55                  60

Glu Asp Glu Ser Ala Pro Lys Glu Val Ser Arg Tyr Gln Asp Thr Asn
65                  70                  75                  80

Met Gln Gly Val Val Tyr Glu Leu Asn Ser Tyr Ile Glu Gln Arg Leu
                85                  90                  95

Asp Thr Gly Gly Asp Asn Gln Leu Leu Leu Tyr Glu Leu Ser Ser Ile
            100                 105                 110

Ile Lys Ile Ala Thr Lys Ala Asp Gly Phe Ala Leu Tyr Phe Leu Gly
        115                 120                 125
```

Glu Cys Asn Asn Ser Leu Cys Ile Phe Thr Pro Pro Gly Ile Lys Glu
130 135 140

Gly Lys Pro Arg Leu Ile Pro Ala Gly Pro Ile Thr Gln Gly Thr Thr
145 150 155 160

Val Ser Ala Tyr Val Ala Lys Ser Arg Lys Thr Leu Leu Val Glu Asp
165 170 175

Ile Leu Gly Asp Glu Arg Phe Pro Arg Gly Thr Gly Leu Glu Ser Gly
180 185 190

Thr Arg Ile Gln Ser Val Leu Cys Leu Pro Ile Val Thr Ala Ile Gly
195 200 205

Asp Leu Ile Gly Ile Leu Glu Leu Tyr Arg His Trp Gly Lys Glu Ala
210 215 220

Phe Cys Leu Ser His Gln Glu Val Ala Thr Ala Asn Leu Ala Trp Ala
225 230 235 240

Ser Val Ala Ile His Gln Val Gln Val Cys Arg Gly Leu Ala Lys Gln
245 250 255

Thr Glu Leu Asn Asp Phe Leu Leu Asp Val Ser Lys Thr Tyr Phe Asp
260 265 270

Asn Ile Val Ala Ile Asp Ser Leu Leu Glu His Ile Met Ile Tyr Ala
275 280 285

Lys Asn Leu Val Asn Ala Asp Arg Cys Ala Leu Phe Gln Val Asp His
290 295 300

Lys Asn Lys Glu Leu Tyr Ser Asp Leu Phe Asp Ile Gly Glu Glu Lys
305 310 315 320

Glu Gly Lys Pro Val Phe Lys Lys Thr Lys Glu Ile Arg Phe Ser Ile
325 330 335

```
Glu Lys Gly Ile Ala Gly Gln Val Ala Arg Thr Gly Glu Val Leu Asn
            340                 345                 350

Ile Pro Asp Ala Tyr Ala Asp Pro Arg Phe Asn Arg Glu Val Asp Leu
        355                 360                 365

Tyr Thr Gly Tyr Thr Thr Arg Asn Ile Leu Cys Met Pro Ile Val Ser
    370                 375                 380

Arg Gly Ser Val Ile Gly Val Val Gln Met Val Asn Lys Ile Ser Gly
385                 390                 395                 400

Ser Ala Phe Ser Lys Thr Asp Glu Asn Asn Phe Lys Met Phe Ala Val
                405                 410                 415

Phe Cys Ala Leu Ala Leu His Cys Ala Asn Met Tyr His Arg Ile Arg
            420                 425                 430

His Ser Glu Cys Ile Tyr Arg Val Thr Met Glu Lys Leu Ser Tyr His
        435                 440                 445

Ser Ile Cys Thr Ser Glu Glu Trp Gln Gly Leu Met Gln Phe Thr Leu
    450                 455                 460

Pro Val Arg Leu Cys Lys Glu Ile Glu Leu Phe His Phe Asp Ile Gly
465                 470                 475                 480

Pro Phe Glu Asn Met Trp Pro Gly Ile Phe Val Tyr Met Val His Arg
                485                 490                 495

Ser Cys Gly Thr Ser Cys Phe Glu Leu Glu Lys Leu Cys Arg Phe Ile
            500                 505                 510

Met Ser Val Lys Lys Asn Tyr Arg Arg Val Pro Tyr His Asn Trp Lys
        515                 520                 525

His Ala Val Thr Val Ala His Cys Met Tyr Ala Ile Leu Gln Asn Asn
    530                 535                 540
```

His Thr Leu Phe Thr Asp Leu Glu Arg Lys Gly Leu Leu Ile Ala Cys
545 550 555 560

Leu Cys His Asp Leu Asp His Arg Gly Phe Ser Asn Ser Tyr Leu Gln
565 570 575

Lys Phe Asp His Pro Leu Thr Ala Leu Tyr Ser Thr Ser Thr Met Glu
580 585 590

Gln His His Phe Ser Gln Thr Val Ser Ile Leu Gln Leu Glu Gly His
595 600 605

Asn Ile Phe Ser Thr Leu Ser Ser Ser Glu Tyr Glu Gln Val Leu Glu
610 615 620

Ile Ile Arg Lys Ala Ile Ile Ala Thr Asp Leu Ala Leu Tyr Phe Gly
625 630 635 640

Asn Arg Lys Gln Leu Glu Glu Met Tyr Gln Thr Gly Ser Leu Asn Leu
645 650 655

Asn Asn Gln Ser His Arg Asp Arg Val Ile Gly Leu Met Met Thr Ala
660 665 670

Cys Asp Leu Cys Ser Val Thr Lys Pro Trp Pro Val Thr Lys Leu Thr
675 680 685

Ala Asn Asp Ile Tyr Ala Glu Phe Trp Ala Glu Gly Asp Glu Met Lys
690 695 700

Lys Leu Gly Ile Gln Pro Ile Pro Met Met Asp Arg Asp Lys Lys Asp
705 710 715 720

Glu Val Pro Gln Gly Gln Leu Gly Phe Tyr Asn Ala Val Ala Ile Pro
725 730 735

Cys Tyr Thr Thr Leu Thr Gln Ile Leu Pro Pro Thr Glu Pro Leu Leu
740 745 750

Lys Ala Cys Arg Asp Asn Leu Ser Gln Trp Glu Lys Val Ile Arg Gly
755 760 765

Glu Glu Thr Ala Thr Trp Ile Ser Ser Pro Ser Val Ala Gln Lys Ala
770 775 780

Ala Ala Ser Glu Asp
785

<210> 3
<211> 2798
<212> DNA
<213> Homo sapiens

<400> 3
acatagctgg gtgcaatgta agtgcctggc tgaagtttga cacgcgaacg gacggcccgc 60
tggaattctg tgctatgagc cggagtagaa agagagattt ggactctgca acaccaaggt 120
agtcgttgaa gccacagtcg tgaatggaga ccaggagtga atagtgggag tgagcagaag 180
tcggaggata ggacagaaga aggcagagcc atggagcacc ctggagaggt gtgacccggc 240
aagatcctga gatggaaggt agcacggcct ggagttcaga agcggagcct caagagggaa 300
gaagccagat gctccagaga gcaggtttga cagatgaaaa agtgaaggca tatctttctc 360
ttcacccccca ggtattagat gaatttgtat ctgaaagtgt tagtgcagag acagtagaga 420
aatggctgaa gaggaagaac aacaaatcag aagatgaatc agctcctaag gaagtcagca 480
ggtaccaaga tacgaatatg cagggagttg tatatgaact aaacagctat atagaacaac 540
ggttggacac aggaggagac aaccagctac tcctctatga actgagcagc atcattaaaa 600
tagccacaaa agccgatgga tttgcactgt atttccttgg agagtgcaat aatagcctgt 660
gtatattcac gccacctggg ataaaggaag gaaaaccccg cctcatccct gctgggccca 720
tcactcaggg caccaccgtc tctgcttatg tggccaagtc caggaaaaca ctgctagtag 780
aagacatcct tggagatgaa cgatttccaa gaggtactgg actggaatca gggactcgta 840
tccagtctgt tctttgctta ccaattgtca ctgcaattgg tgacttgatt ggtattctcg 900
agctgtatcg gcactggggc aaagaagcct tctgtcttag tcaccaggag gttgcaacag 960
caaatcttgc ctgggcttca gtagcaatac atcaggtgca ggtatgcaga ggccttgcca 1020
aacagacaga attgaatgac ttcctactcg acgtatcaaa aacatatttt gataacatag 1080
ttgcaataga ttctctactt gaacacataa tgatatatgc aaaaaacctg gtgaatgccg 1140
atcgttgtgc acttttccag gtggaccata agaacaagga gttatattca gacctttttg 1200
atattggaga ggaaaaggaa ggaaaacctg tcttcaagaa gaccaaagag ataagatttt 1260

caattgagaa aggaattgct ggccaagtag caagaacagg ggaagtcctg aacattccag 1320
atgcctatgc agacccacgc tttaacagag aagtagactt gtacacaggc tacaccacgc 1380
ggaacatcct gtgcatgccc atcgtcagcc gaggcagcgt gataggtgtg gtgcagatgg 1440
tcaacaaaat cagtggcagt gccttctcta aaacagatga aaacaacttc aaaatgtttg 1500
ccgtcttttg tgctttagcc ttacactgtg ctaatatgta tcatagaatt cgccactcag 1560
agtgcattta ccgggtaacg atggaaaagc tgtcctacca tagcatttgt acttcagaag 1620
agtggcaagg tctcatgcaa ttcacccttc ccgtgcgtct ctgcaaagaa attgaattat 1680
tccactttga cattggtcct tttgaaaaca tgtggcctgg aatttttgtc tacatggttc 1740
atcggtcctg tgggacatcc tgctttgagc ttgaaaagtt gtgtcgtttt attatgtctg 1800
tgaagaagaa ctatcggcgg gttccttatc acaactggaa gcatgcggtc actgtagcac 1860
actgcatgta tgccatactt cagaacaatc acacgctttt cacagacctt gagcgcaaag 1920
gactgctgat tgcgtgtctg tgtcatgacc tggaccacag gggcttcagt aacagctacc 1980
tgcagaagtt cgaccaccct ctgaccgctc tctactccac ttccaccatg gagcagcacc 2040
acttctccca gactgtgtcc atccttcagt tggaagggca caatatcttc tccactctga 2100
gctccagtga atatgagcag gtgcttgaga tcatccgcaa agccatcatt gccacagacc 2160
ttgctttata ctttggaaac aggaagcagt tggaagagat gtaccagacc ggatcactaa 2220
accttaataa tcaatcacat agagaccgtg taattggttt gatgatgact gcctgtgacc 2280
tttgttctgt gacaaaaccg tggcccgtta caaaattgac ggcaaatgat atatatgcag 2340
aattctgggc tgagggtgat gaaatgaaga aattgggaat acagcctatt cctatgatgg 2400
acagagacaa gaaggatgaa gtcccccaag gccagcttgg gttctacaat gccgtggcca 2460
ttccctgcta tacaaccctt acccagatcc tccctcccac ggagcctctt ctgaaagcat 2520
gcagggataa tctcagtcag tgggagaagg tgattcgagg ggaggagact gcaacctgga 2580
tttcatcccc atccgtggct cagaaggcag ctgcatctga agattgagca ctggtcaccc 2640
tgacacgctg tcccacctac agatcctcat cttgcttctt tgacattctt ttcctttttt 2700
gggggggggtg gggggaacct gcacctggta actggggtgc aaacctcttc aagaaggtaa 2760
catcaaataa ataagtcaag cagaaaaaaa aaaaaaaa 2798

<210> 4
<211> 791
<212> PRT
<213> Homo sapiens

<400> 4
Met Glu Gly Ser Thr Ala Trp Ser Ser Glu Ala Glu Pro Gln Glu Gly
1 5 10 15

Arg Ser Gln Met Leu Gln Arg Ala Gly Leu Thr Asp Glu Lys Val Lys
20 25 30

Ala Tyr Leu Ser Leu His Pro Gln Val Leu Asp Glu Phe Val Ser Glu
35 40 45

Ser Val Ser Ala Glu Thr Val Glu Lys Trp Leu Lys Arg Lys Asn Asn
50 55 60

Lys Ser Glu Asp Glu Ser Ala Pro Lys Glu Val Ser Arg Tyr Gln Asp
65 70 75 80

Thr Asn Met Gln Gly Val Val Tyr Glu Leu Asn Ser Tyr Ile Glu Gln
85 90 95

Arg Leu Asp Thr Gly Gly Asp Asn Gln Leu Leu Leu Tyr Glu Leu Ser
100 105 110

Ser Ile Ile Lys Ile Ala Thr Lys Ala Asp Gly Phe Ala Leu Tyr Phe
115 120 125

Leu Gly Glu Cys Asn Asn Ser Leu Cys Ile Phe Thr Pro Pro Gly Ile
130 135 140

Lys Glu Gly Lys Pro Arg Leu Ile Pro Ala Gly Pro Ile Thr Gln Gly
145 150 155 160

Thr Thr Val Ser Ala Tyr Val Ala Lys Ser Arg Lys Thr Leu Leu Val
165 170 175

Glu Asp Ile Leu Gly Asp Glu Arg Phe Pro Arg Gly Thr Gly Leu Glu
180 185 190

Ser Gly Thr Arg Ile Gln Ser Val Leu Cys Leu Pro Ile Val Thr Ala
195 200 205

Ile Gly Asp Leu Ile Gly Ile Leu Glu Leu Tyr Arg His Trp Gly Lys
210 215 220

Glu Ala Phe Cys Leu Ser His Gln Glu Val Ala Thr Ala Asn Leu Ala
225 230 235 240

Trp Ala Ser Val Ala Ile His Gln Val Gln Val Cys Arg Gly Leu Ala
245 250 255

Lys Gln Thr Glu Leu Asn Asp Phe Leu Leu Asp Val Ser Lys Thr Tyr
260 265 270

Phe Asp Asn Ile Val Ala Ile Asp Ser Leu Leu Glu His Ile Met Ile
275 280 285

Tyr Ala Lys Asn Leu Val Asn Ala Asp Arg Cys Ala Leu Phe Gln Val
290 295 300

Asp His Lys Asn Lys Glu Leu Tyr Ser Asp Leu Phe Asp Ile Gly Glu
305 310 315 320

Glu Lys Glu Gly Lys Pro Val Phe Lys Lys Thr Lys Glu Ile Arg Phe
325 330 335

Ser Ile Glu Lys Gly Ile Ala Gly Gln Val Ala Arg Thr Gly Glu Val
340 345 350

Leu Asn Ile Pro Asp Ala Tyr Ala Asp Pro Arg Phe Asn Arg Glu Val
355 360 365

Asp Leu Tyr Thr Gly Tyr Thr Thr Arg Asn Ile Leu Cys Met Pro Ile
370 375 380

Val Ser Arg Gly Ser Val Ile Gly Val Val Gln Met Val Asn Lys Ile
385 390 395 400

Ser Gly Ser Ala Phe Ser Lys Thr Asp Glu Asn Asn Phe Lys Met Phe
405 410 415

Ala Val Phe Cys Ala Leu Ala Leu His Cys Ala Asn Met Tyr His Arg
420 425 430

Ile Arg His Ser Glu Cys Ile Tyr Arg Val Thr Met Glu Lys Leu Ser
435 440 445

Tyr His Ser Ile Cys Thr Ser Glu Glu Trp Gln Gly Leu Met Gln Phe
    450                 455                 460

Thr Leu Pro Val Arg Leu Cys Lys Glu Ile Glu Leu Phe His Phe Asp
465                 470                 475                 480

Ile Gly Pro Phe Glu Asn Met Trp Pro Gly Ile Phe Val Tyr Met Val
                485                 490                 495

His Arg Ser Cys Gly Thr Ser Cys Phe Glu Leu Glu Lys Leu Cys Arg
            500                 505                 510

Phe Ile Met Ser Val Lys Lys Asn Tyr Arg Arg Val Pro Tyr His Asn
        515                 520                 525

Trp Lys His Ala Val Thr Val Ala His Cys Met Tyr Ala Ile Leu Gln
    530                 535                 540

Asn Asn His Thr Leu Phe Thr Asp Leu Glu Arg Lys Gly Leu Leu Ile
545                 550                 555                 560

Ala Cys Leu Cys His Asp Leu Asp His Arg Gly Phe Ser Asn Ser Tyr
                565                 570                 575

Leu Gln Lys Phe Asp His Pro Leu Thr Ala Leu Tyr Ser Thr Ser Thr
            580                 585                 590

Met Glu Gln His His Phe Ser Gln Thr Val Ser Ile Leu Gln Leu Glu
        595                 600                 605

Gly His Asn Ile Phe Ser Thr Leu Ser Ser Ser Glu Tyr Glu Gln Val
    610                 615                 620

Leu Glu Ile Ile Arg Lys Ala Ile Ile Ala Thr Asp Leu Ala Leu Tyr
625                 630                 635                 640

Phe Gly Asn Arg Lys Gln Leu Glu Glu Met Tyr Gln Thr Gly Ser Leu
                645                 650                 655

Asn Leu Asn Asn Gln Ser His Arg Asp Arg Val Ile Gly Leu Met Met
660 665 670

Thr Ala Cys Asp Leu Cys Ser Val Thr Lys Pro Trp Pro Val Thr Lys
675 680 685

Leu Thr Ala Asn Asp Ile Tyr Ala Glu Phe Trp Ala Glu Gly Asp Glu
690 695 700

Met Lys Lys Leu Gly Ile Gln Pro Ile Pro Met Met Asp Arg Asp Lys
705 710 715 720

Lys Asp Glu Val Pro Gln Gly Gln Leu Gly Phe Tyr Asn Ala Val Ala
725 730 735

Ile Pro Cys Tyr Thr Thr Leu Thr Gln Ile Leu Pro Pro Thr Glu Pro
740 745 750

Leu Leu Lys Ala Cys Arg Asp Asn Leu Ser Gln Trp Glu Lys Val Ile
755 760 765

Arg Gly Glu Glu Thr Ala Thr Trp Ile Ser Ser Pro Ser Val Ala Gln
770 775 780

Lys Ala Ala Ala Ser Glu Asp
785 790

<210> 5
<211> 20
<212> DNA
<213> Artificial Sequence

<220>
<221> misc_feature
<222> (3)
<223> n is a or g or c or t

<220>
<223> Description of Artificial Sequence: Primer

<400> 5
tcnccytgrt crtaraaytc 20

<210> 6
<211> 20
<212> DNA
<213> Artificial Sequence

<220>
<223> Description of Artificial Sequence: Primer

<400> 6
tacttcagaa caatcacacg 20

<210> 7
<211> 20
<212> DNA
<213> Artificial Sequence

<220>
<223> Description of Artificial Sequence: Primer

<400> 7
tctccaagga aatacagtgc 20

<210> 8
<211> 11
<212> PRT
<213> Artificial Sequence

<220>
<223> Description of Artificial Sequence: Synthetic sequence

<400> 8

Met Glu Asp Gly Pro Ser Asn Asn Ala Ser Cys
1 5 10

<210> 9
<211> 13
<212> PRT
<213> Artificial Sequence

<220>
<223> Description of Artificial Sequence: Synthetic sequence

<400> 9

Glu Asp Glu Ser Ala Pro Lys Glu Val Ser Arg Tyr Cys
1 5 10

<210> 10
<211> 26
<212> DNA
<213> Artificial Sequence

<220>
<223> Description of Artificial Sequence: Primer

<400> 10

ttcggatccg acatggaaga tggacc 26

<210> 11
<211> 27
<212> DNA
<213> Artificial Sequence

<220>
<223> Description of Artificial Sequence: Primer

<400> 11
ggtgactagt gctcaatctt cagatgc 27

<210> 12
<211> 1068
<212> DNA
<213> Mus sp.

<400> 12
atttgcactg tactttcctt ggagagtgca ataatagcct gtgtgtgttc ataccacccg 60
ggatgaagga aggccaaccc cggctcatcc ctgcggggcc catcacccag ggtaccacca 120
tctctgccta cgtggccaag tctaggaaga cgttgttggt agaggatatc cttggggatg 180
agcgatttcc tcgaggtact ggcctggaat caggaacccg catccagtct gttctttgct 240
tgcccattgt cactgccatt ggagacttga ttggcatcct tgaactgtac aggcactggg 300
acaaagaggc cttctgcctc agccatcagg aggttgcaac agccaatctt gcttgggctt 360
ccgtagcaat acaccaggtg caggtgtgta gaggtctcgc caaacagacc gaactgaatg 420
acttcctact cgacgtatca aagacatact ttgataacat agttgccata gactctctac 480
ttgaacacat cataatatat gcaaaaaatc tagtgaacgc cgaccgctgc gcgctcttcc 540
aggtggacca caagaacaag gagctgtact cggacctgtt tgacattggg gaggagaagg 600
aggggaagcc catcttcaag aagaccaagg agatcagatt ttccattgag aaagggattg 660
ctggtcaagt ggcaagaaca ggcgaagtct tgaacattcc cgatgcctac gcggaccctc 720
gctttaacag ggaggtggac ctgtacacag gctacaccac gaggaacatt ctgtgtatgc 780
ccatagtgag ccgaggcagc gtgattggcg tggtgcagat ggtgaacaag atcagcggta 840
gcgccttctc caagacagac gagaacaact tcaagatgtt tgctgtcttc tgcgcactgg 900
ccttgcactg tgctaacgcc agatggaaaa gcctagcttc tctcccctgg gtcagctggg 960
aaggtttgct agcttgcctg cactgtggca aagacctgag gacctggaat ggtgaccact 1020
gtctgacgtg cacagtcttt cctgcctctg tgacacccgc ttgggata 1068

<210> 13
<211> 300
<212> PRT
<213> Mus sp.

<400> 13

Phe Leu Gly Glu Cys Asn Asn Ser Leu Cys Val Phe Ile Pro Pro Gly
1               5                   10                  15

Met Lys Glu Gly Gln Pro Arg Leu Ile Pro Ala Gly Pro Ile Thr Gln
            20                  25                  30

Gly Thr Thr Ile Ser Ala Tyr Val Ala Lys Ser Arg Lys Thr Leu Leu
        35                  40                  45

Val Glu Asp Ile Leu Gly Asp Glu Arg Phe Pro Arg Gly Thr Gly Leu
    50                  55                  60

Glu Ser Gly Thr Arg Ile Gln Ser Val Leu Cys Leu Pro Ile Val Thr
65                  70                  75                  80

Ala Ile Gly Asp Leu Ile Gly Ile Leu Glu Leu Tyr Arg His Trp Asp
                85                  90                  95

Lys Glu Ala Phe Cys Leu Ser His Gln Glu Val Ala Thr Ala Asn Leu
            100                 105                 110

Ala Trp Ala Ser Val Ala Ile His Gln Val Gln Val Cys Arg Gly Leu
        115                 120                 125

Ala Lys Gln Thr Glu Leu Asn Asp Phe Leu Leu Asp Val Ser Lys Thr
    130                 135                 140

Tyr Phe Asp Asn Ile Val Ala Ile Asp Ser Leu Leu Glu His Ile Ile
145                 150                 155                 160

Ile Tyr Ala Lys Asn Leu Val Asn Ala Asp Arg Cys Ala Leu Phe Gln
                165                 170                 175

Val Asp His Lys Asn Lys Glu Leu Tyr Ser Asp Leu Phe Asp Ile Gly
            180                 185                 190

Glu Glu Lys Glu Gly Lys Pro Ile Phe Lys Lys Thr Lys Glu Ile Arg
195 200 205

Phe Ser Ile Glu Lys Gly Ile Ala Gly Gln Val Ala Arg Thr Gly Glu
210 215 220

Val Leu Asn Ile Pro Asp Ala Tyr Ala Asp Pro Arg Phe Asn Arg Glu
225 230 235 240

Val Asp Leu Tyr Thr Gly Tyr Thr Thr Arg Asn Ile Leu Cys Met Pro
245 250 255

Ile Val Ser Arg Gly Ser Val Ile Gly Val Val Gln Met Val Asn Lys
260 265 270

Ile Ser Gly Ser Ala Phe Ser Lys Thr Asp Glu Asn Asn Phe Lys Met
275 280 285

Phe Ala Val Phe Cys Ala Leu Ala Leu His Cys Ala
290 295 300

<210> 14
<211> 3606
<212> DNA
<213> Mus sp.

<400> 14
acgcgtccgc tcctcatctg ccttccacct ccccgcgcgt ctcccgagaa gggagggcgc 60
agcggcggct ggaggaggag gaggcggcgg cggcgatgct ggcggcggcg gcggaggagg 120
aggacaagag gcagctccct tgagcgtccc cccaggcagt agtcaccgca gcagcggtgg 180
cagcagcggt ggcagcggcg ggcggcggcg gctcttcctc tcgcctgcga ttcaaggctt 240
gctgctccct gcccgcgccg ggccccggcc atctccgccg ccgcggcttc ccctacaccc 300
gggtgcacgc cgcgcggact cctcggattt tccgggcgcc ggcgggggct gccctggcct 360
cggccccggc tctgccgccg gtggccgaac tctttggcgg ccccgaggcg ccgccttccc 420
ccttgccacc gtttggccgc tgcccttcgg ctccgacatg gaagatggac cctctaacaa 480
tgcgagttgc ttccgaaggc tgaccgagtg tttcctcagc cccagtttga cggatgaaaa 540

ggtgaaggcc tatctttctc tccatcccca ggtattagat gaatttgttt ctgaaagtgt 600
tagtgcagag actgtggaaa agtggctgaa gaggaaaacc aacaaagcaa aagatgaacc 660
atctcccaag gaagtcagca ggtaccagga tacgaatatg cagggagtcg tgtacgagct 720
gaacagctac atagagcagc gcctggacac gggcggggac aaccacctgc tcctctatga 780
gctcagcagc atcatcagga tagccacaaa agccgacgga tttgcactgt acttccttgg 840
agagtgcaat aatagcctgt gtgtgttcat accacccggg atgaaggaag gccaaccccg 900
gctcatccct gcagggccca tcacccaggg taccaccatc tctgcctacg tggccaagtc 960
taggaagacg ttgttggtag aggatatcct tggggatgag cgatttcctc gaggtactgg 1020
cctggaatca ggaacccgca tccagtctgt tctttgcttg cccattgtca ctgccattgg 1080
agacttgatt ggcatccttg aactgtacag gcactggggc aaagaggcct tctgcctcag 1140
ccatcaggag gttgcaacag ccaatcttgc ttgggcttcc gtagcaatac accaggtgca 1200
ggtgtgtaga ggtctcgcca aacagaccga actgaatgac ttcctactcg acgtatcaaa 1260
gacatacttt gataacatag ttgccataga ctctctactt gaacacatca tgatatatgc 1320
aaaaaatcta gtgaacgccg accgctgcgc gctcttccag gtggaccaca agaacaagga 1380
gctgtactcg gacctgtttg acattgggga ggagaaggag gggaagccca tcttcaagaa 1440
gaccaaggag atcagatttt ccattgagaa agggattgct ggtcaagtgg caagaacagg 1500
cgaagtcttg aacattcccg atgcctacgc ggaccctcgc tttaacaggg aggtggacct 1560
gtacacaggc tacaccacga ggaacattct gtgtatgccc atagtgagcc gaggcagcgt 1620
gattggcgtg gtgcagatgg tgaacaagat cagcggtagc gccttctcca agacagacga 1680
gaacaacttc aagatgtttg ctgtcttctg cgcactggcc ttgcactgtg ctaacatgta 1740
ccacaggatc cgccactcag aatgcatcta cagggttacc atggagaagc tttcctacca 1800
cagcatctgc acctccgagg agtggcaagg cctcatgcgc ttcaacctac cagcacgcat 1860
ctgccgggac atcgagctat tccactttga cattggtcct ttcgagaaca tgtggcctgg 1920
gatctttgtc tacatgatcc atcggtcttg tgggacatcc tgttttgaac ttgaaaaatt 1980
gtgccgtttt atcatgtctg tgaagaagaa ctatcggcgg gttccttacc acaactggaa 2040
gcatgcagtc acggtggcac actgcatgta tgccatactt caaaacaaca atggcctctt 2100
cacagacctc gagcgcaaag gcctgctaat tgcgtgtctg tgccatgacc tggaccacag 2160
gggcttcagt aacagctacc tgcagaagtt cgaccacccc ctggcggcgc tgtactccac 2220
ctccaccatg gagcaacacc acttctccca gacggtgtcc atccttcagc tggaagggca 2280
caatatcttc tccaccctga gctccagcga gtacgagcag gtgctggaga tcatccgcaa 2340
agccatcatc gccaccgacc tcgccctata ctttgggaac aggaagcagt tggaggagat 2400
gtaccagaca gggtcgctga acctccacaa ccagtcccat cgagaccgtg tcatcggctt 2460
gatgatgact gcctgtgatc tttgctctgt gaccaaacta tggccagtta caaaattgac 2520
agcgaatgat atatatgcag aattctgggc tgagggtgat gagatgaaga agctgggcat 2580
acagcccatt cctatgatgg acagagacaa gcgagatgaa gtccctcaag ggcagctcgg 2640
attctacaat gctgtggcca ttccctgcta taccaccttg acgcagatcc tcccacccac 2700
agagcctctg ctgaaggcct gcagggataa cctcaatcag tgggagaagg taattcgcgg 2760
ggaagagaca gcaatgtgga tttcaggccc aggcccggcg cctagcaaga gcacacctga 2820

gaagctgaac gtgaaggttg aagactgatc ctgaagtgac gtcctgatgt ctgcccagca 2880
accgactcaa cctgcttctg tgacttcgtt ctttttgttt tcaaggggtg aaaacccccct 2940
gtcagaaggt accgtcgcat atccatgtga agcagacgac tccctgcttg ccgcacacac 3000
ctcggacagt gagcaaccca ggctctgccg tgttcagacg tcggctactc cgtggctcca 3060
cctgacctcc gaatgctatt tgctcccagg ccagcactgc actgtctgga gggggcagag 3120
accacaggag aggttcttgc ctgcatcctc ccatgagggt gtggccagtt ccctggttct 3180
gtgccatgct gctgcttggt ggcattggtt aggaatggga cacacgcccc ttgttgtgaa 3240
gtttacatgt gaccttctta taggttaact gagtttgtgg cctgggacac atgtaatgaa 3300
ggtcacagtc cacaggtgac agagaaatcc aaactgttga ttacaggtgc actacaggta 3360
tgctctttca gtctatctgg gggcacatag gtgagtctgc tccactcaga aggaagcata 3420
cctctgccct catccagggg acacagggta catcccaggc atcggggaac tgaagctctc 3480
acttcaaacc atgtcaaaga attaaaacac ctcccctccc cctcactgta gccttcggca 3540
actgcgccaa tccctttata caaagaaaat aaaagtaagg catataaatt taaaaaaaaa 3600
aaaaaa 3606

<210> 15
<211> 796
<212> PRT
<213> Mus sp.

<400> 15
Met Glu Asp Gly Pro Ser Asn Asn Ala Ser Cys Phe Arg Arg Leu Thr
1 5 10 15

Glu Cys Phe Leu Ser Pro Ser Leu Thr Asp Glu Lys Val Lys Ala Tyr
20 25 30

Leu Ser Leu His Pro Gln Val Leu Asp Glu Phe Val Ser Glu Ser Val
35 40 45

Ser Ala Glu Thr Val Glu Lys Trp Leu Lys Arg Lys Thr Asn Lys Ala
50 55 60

Lys Asp Glu Pro Ser Pro Lys Glu Val Ser Arg Tyr Gln Asp Thr Asn
65 70 75 80

Met Gln Gly Val Val Tyr Glu Leu Asn Ser Tyr Ile Glu Gln Arg Leu
85 90 95

```
Asp Thr Gly Gly Asp Asn His Leu Leu Leu Tyr Glu Leu Ser Ser Ile
            100                 105                 110

Ile Arg Ile Ala Thr Lys Ala Asp Gly Phe Ala Leu Tyr Phe Leu Gly
        115                 120                 125

Glu Cys Asn Asn Ser Leu Cys Val Phe Ile Pro Pro Gly Met Lys Glu
    130                 135                 140

Gly Gln Pro Arg Leu Ile Pro Ala Gly Pro Ile Thr Gln Gly Thr Thr
145                 150                 155                 160

Ile Ser Ala Tyr Val Ala Lys Ser Arg Lys Thr Leu Leu Val Glu Asp
                165                 170                 175

Ile Leu Gly Asp Glu Arg Phe Pro Arg Gly Thr Gly Leu Glu Ser Gly
            180                 185                 190

Thr Arg Ile Gln Ser Val Leu Cys Leu Pro Ile Val Thr Ala Ile Gly
        195                 200                 205

Asp Leu Ile Gly Ile Leu Glu Leu Tyr Arg His Trp Gly Lys Glu Ala
    210                 215                 220

Phe Cys Leu Ser His Gln Glu Val Ala Thr Ala Asn Leu Ala Trp Ala
225                 230                 235                 240

Ser Val Ala Ile His Gln Val Gln Val Cys Arg Gly Leu Ala Lys Gln
                245                 250                 255

Thr Glu Leu Asn Asp Phe Leu Leu Asp Val Ser Lys Thr Tyr Phe Asp
            260                 265                 270

Asn Ile Val Ala Ile Asp Ser Leu Leu Glu His Ile Met Ile Tyr Ala
        275                 280                 285

Lys Asn Leu Val Asn Ala Asp Arg Cys Ala Leu Phe Gln Val Asp His
    290                 295                 300
```

Lys Asn Lys Glu Leu Tyr Ser Asp Leu Phe Asp Ile Gly Glu Glu Lys
305 310 315 320

Glu Gly Lys Pro Ile Phe Lys Lys Thr Lys Glu Ile Arg Phe Ser Ile
325 330 335

Glu Lys Gly Ile Ala Gly Gln Val Ala Arg Thr Gly Glu Val Leu Asn
340 345 350

Ile Pro Asp Ala Tyr Ala Asp Pro Arg Phe Asn Arg Glu Val Asp Leu
355 360 365

Tyr Thr Gly Tyr Thr Thr Arg Asn Ile Leu Cys Met Pro Ile Val Ser
370 375 380

Arg Gly Ser Val Ile Gly Val Val Gln Met Val Asn Lys Ile Ser Gly
385 390 395 400

Ser Ala Phe Ser Lys Thr Asp Glu Asn Asn Phe Lys Met Phe Ala Val
405 410 415

Phe Cys Ala Leu Ala Leu His Cys Ala Asn Met Tyr His Arg Ile Arg
420 425 430

His Ser Glu Cys Ile Tyr Arg Val Thr Met Glu Lys Leu Ser Tyr His
435 440 445

Ser Ile Cys Thr Ser Glu Glu Trp Gln Gly Leu Met Arg Phe Asn Leu
450 455 460

Pro Ala Arg Ile Cys Arg Asp Ile Glu Leu Phe His Phe Asp Ile Gly
465 470 475 480

Pro Phe Glu Asn Met Trp Pro Gly Ile Phe Val Tyr Met Ile His Arg
485 490 495

Ser Cys Gly Thr Ser Cys Phe Glu Leu Glu Lys Leu Cys Arg Phe Ile
500 505 510

Met Ser Val Lys Lys Asn Tyr Arg Arg Val Pro Tyr His Asn Trp Lys
515 520 525

His Ala Val Thr Val Ala His Cys Met Tyr Ala Ile Leu Gln Asn Asn
530 535 540

Asn Gly Leu Phe Thr Asp Leu Glu Arg Lys Gly Leu Leu Ile Ala Cys
545 550 555 560

Leu Cys His Asp Leu Asp His Arg Gly Phe Ser Asn Ser Tyr Leu Gln
565 570 575

Lys Phe Asp His Pro Leu Ala Ala Leu Tyr Ser Thr Ser Thr Met Glu
580 585 590

Gln His His Phe Ser Gln Thr Val Ser Ile Leu Gln Leu Glu Gly His
595 600 605

Asn Ile Phe Ser Thr Leu Ser Ser Ser Glu Tyr Glu Gln Val Leu Glu
610 615 620

Ile Ile Arg Lys Ala Ile Ile Ala Thr Asp Leu Ala Leu Tyr Phe Gly
625 630 635 640

Asn Arg Lys Gln Leu Glu Glu Met Tyr Gln Thr Gly Ser Leu Asn Leu
645 650 655

His Asn Gln Ser His Arg Asp Arg Val Ile Gly Leu Met Met Thr Ala
660 665 670

Cys Asp Leu Cys Ser Val Thr Lys Leu Trp Pro Val Thr Lys Leu Thr
675 680 685

Ala Asn Asp Ile Tyr Ala Glu Phe Trp Ala Glu Gly Asp Glu Met Lys
690 695 700

Lys Leu Gly Ile Gln Pro Ile Pro Met Met Asp Arg Asp Lys Arg Asp
705 710 715 720

Glu Val Pro Gln Gly Gln Leu Gly Phe Tyr Asn Ala Val Ala Ile Pro
725 730 735

Cys Tyr Thr Thr Leu Thr Gln Ile Leu Pro Pro Thr Glu Pro Leu Leu
740 745 750

Lys Ala Cys Arg Asp Asn Leu Asn Gln Trp Glu Lys Val Ile Arg Gly
755 760 765

Glu Glu Thr Ala Met Trp Ile Ser Gly Pro Gly Pro Ala Pro Ser Lys
770 775 780

Ser Thr Pro Glu Lys Leu Asn Val Lys Val Glu Asp
785 790 795

<210> 16
<211> 487
<212> DNA
<213> Rattus sp.

<400> 16
ccttatggaa gatggaccct ctaacaatgc gagttgcttc cgaaggctga ccgagtgttt 60
cctcagcccc agtttgacgg atgaaaaggt gaaggcctat ctttccctcc atccccaggt 120
attagacgag tttgtttctg aaagtgttag tgcggagacc gtggagaagt ggctgaagag 180
gaaaaacgac aaagcagaag atgaaccatc tcctaaggaa gtcagcaggt accaggacac 240
gaacatgcag ggagtcgtgt acgagctgaa cagctacata gagcagcgcc tggacaccgg 300
cggggacaac cacctgctcc tgtacgagct aaacagtatc atcaggatag ccacaaaagc 360
cgacggattt gcactgtact tccttggaga gtgcaataat agtctgtgtg tcttcacacc 420
acccggaatg aaggaaggtc aaccccgtct catccccgca gggcccatca cccagggcac 480
caccatc 487

<210> 17
<211> 160
<212> PRT
<213> Rattus sp.

<400> 17

```
Met Glu Asp Gly Pro Ser Asn Asn Ala Ser Cys Phe Arg Arg Leu Thr
  1               5                  10                  15

Glu Cys Phe Leu Ser Pro Ser Leu Thr Asp Glu Lys Val Lys Ala Tyr
             20                  25                  30

Leu Ser Leu His Pro Gln Val Leu Asp Glu Phe Val Ser Glu Ser Val
         35                  40                  45

Ser Ala Glu Thr Val Glu Lys Trp Leu Lys Arg Lys Asn Asp Lys Ala
     50                  55                  60

Glu Asp Glu Pro Ser Pro Lys Glu Val Ser Arg Tyr Gln Asp Thr Asn
 65                  70                  75                  80

Met Gln Gly Val Val Tyr Glu Leu Asn Ser Tyr Ile Glu Gln Arg Leu
                 85                  90                  95

Asp Thr Gly Gly Asp Asn His Leu Leu Leu Tyr Glu Leu Asn Ser Ile
            100                 105                 110

Ile Arg Ile Ala Thr Lys Ala Asp Gly Phe Ala Leu Tyr Phe Leu Gly
        115                 120                 125

Glu Cys Asn Asn Ser Leu Cys Val Phe Thr Pro Pro Gly Met Lys Glu
    130                 135                 140

Gly Gln Pro Arg Leu Ile Pro Ala Gly Pro Ile Thr Gln Gly Thr Thr
145                 150                 155                 160
```

<210> 18
<211> 540
<212> DNA
<213> Rattus sp.

<400> 18
aatctcgctt gggcttccgt agcaatacac caggtgcagg tgtgcagagg tctcgccaag 60
cagaccgaac tgaatgactt cctgctcgat gtatcaaaga catactttga taacatagtc 120
gccatagact ctctacttga acacatcatg atatatgcaa aaaatctagt gaacgccgac 180
cgctgcgcgc tcttccaggt ggaccacaag aacaaggagc tgtactcgga cctgtttgac 240
attggggagg agaaggaggg gaagcccgtc ttcaagaaga ccaaggagat cagattttcc 300
attgagaaag ggattgctgg tcaagtggca agaacgggag aagtcctgaa cattcctgat 360
gcctacgcag acccgcgctt taacagggag gtggacctgt acacaggcta taccacgcgg 420
aacattctgt gtatgcccat agtgagccgc ggcagcgtga tcggtgtggt gcaaatggtt 480
aacaagatca gcggcagcgc cttctccaag acggatgaga acaacttcaa gatgaagggc 540

<210> 19
<211> 178
<212> PRT
<213> Rattus sp.

<400> 19
Asn Leu Ala Trp Ala Ser Val Ala Ile His Gln Val Gln Val Cys Arg
1 5 10 15

Gly Leu Ala Lys Gln Thr Glu Leu Asn Asp Phe Leu Leu Asp Val Ser
20 25 30

Lys Thr Tyr Phe Asp Asn Ile Val Ala Ile Asp Ser Leu Leu Glu His
35 40 45

Ile Met Ile Tyr Ala Lys Asn Leu Val Asn Ala Asp Arg Cys Ala Leu
50 55 60

Phe Gln Val Asp His Lys Asn Lys Glu Leu Tyr Ser Asp Leu Phe Asp
65 70 75 80

Ile Gly Glu Glu Lys Glu Gly Lys Pro Val Phe Lys Lys Thr Lys Glu
85 90 95

Ile Arg Phe Ser Ile Glu Lys Gly Ile Ala Gly Gln Val Ala Arg Thr
100 105 110

Gly Glu Val Leu Asn Ile Pro Asp Ala Tyr Ala Asp Pro Arg Phe Asn
115 120 125

Arg Glu Val Asp Leu Tyr Thr Gly Tyr Thr Thr Arg Asn Ile Leu Cys
130 135 140

Met Pro Ile Val Ser Arg Gly Ser Val Ile Gly Val Val Gln Met Val
145 150 155 160

Asn Lys Ile Ser Gly Ser Ala Phe Ser Lys Thr Asp Glu Asn Asn Phe
165 170 175

Lys Met

<210> 20
<211> 37
<212> PRT
<213> Artificial Sequence

<220>
<223> Z1 in Formula I and Formula II

<220>
<223> Description of Artificial Sequence: Synthetic sequence

<400> 20
Leu Thr Asp Glu Lys Val Lys Ala Tyr Leu Ser Leu His Pro Gln Val
1 5 10 15

Leu Asp Glu Phe Val Ser Glu Ser Val Ser Ala Glu Thr Val Glu Lys
20 25 30

Trp Leu Lys Arg Lys
35

<210> 21
<211> 33
<212> PRT
<213> Artificial Sequence

<220>
<223> Z4 in Formula I and Formula II

<220>
<223> Description of Artificial Sequence: Synthetic
sequence

<400> 21
Pro Lys Glu Val Ser Arg Tyr Gln Asp Thr Asn Met Gln Gly Val Val
1 5 10 15

Tyr Glu Leu Asn Ser Tyr Ile Glu Gln Arg Leu Asp Thr Gly Gly Asp
20 25 30

Asn

<210> 22
<211> 10
<212> PRT
<213> Artificial Sequence

<220>
<223> Z5 in Formula I and Formula II

<220>
<223> Description of Artificial Sequence: Synthetic
sequence

<400> 22
Leu Leu Leu Tyr Glu Leu Ser Ser Ile Ile
1 5 10

<210> 23
<211> 21
<212> PRT
<213> Artificial Sequence

<220>
<223> Z6 in Formula I and Formula II

<220>
<223> Description of Artificial Sequence: Synthetic sequence

<400> 23
Ile Ala Thr Lys Ala Asp Gly Phe Ala Leu Tyr Phe Leu Gly Glu Cys
1 5 10 15

Asn Asn Ser Leu Cys
20

<210> 24
<211> 14
<212> PRT
<213> Artificial Sequence

<220>
<223> Z10 in Formula I and Formula II

<220>
<223> Description of Artificial Sequence: Synthetic sequence

<400> 24

Pro Arg Leu Ile Pro Ala Gly Pro Ile Thr Gln Gly Thr Thr
1               5                   10

<210> 25
<211> 163
<212> PRT
<213> Artificial Sequence

<220>
<223> Z11 in Formula I and Formula II

<220>
<223> Description of Artificial Sequence: Synthetic sequence

<400> 25

Ser Ala Tyr Val Ala Lys Ser Arg Lys Thr Leu Leu Val Glu Asp Ile
1               5                   10                  15

Leu Gly Asp Glu Arg Phe Pro Arg Gly Thr Gly Leu Glu Ser Gly Thr
            20                  25                  30

Arg Ile Gln Ser Val Leu Cys Leu Pro Ile Val Thr Ala Ile Gly Asp
        35                  40                  45

Leu Ile Gly Ile Leu Glu Leu Tyr Arg His Trp Gly Lys Glu Ala Phe
    50                  55                  60

Cys Leu Ser His Gln Glu Val Ala Thr Ala Asn Leu Ala Trp Ala Ser
65                  70                  75                  80

Val Ala Ile His Gln Val Gln Val Cys Arg Gly Leu Ala Lys Gln Thr
                85                  90                  95

Glu Leu Asn Asp Phe Leu Leu Asp Val Ser Lys Thr Tyr Phe Asp Asn
            100                 105                 110

Ile Val Ala Ile Asp Ser Leu Leu Glu His Ile Met Ile Tyr Ala Lys
        115                 120                 125

Asn Leu Val Asn Ala Asp Arg Cys Ala Leu Phe Gln Val Asp His Lys
    130                 135                 140

Asn Lys Glu Leu Tyr Ser Asp Leu Phe Asp Ile Gly Glu Glu Lys Glu
145                 150                 155                 160

Gly Lys Pro

<210> 26
<211> 135
<212> PRT
<213> Artificial Sequence

<220>
<223> Z12 in Formula I and Formula II

<220>
<223> Description of Artificial Sequence: Synthetic sequence

<400> 26
Phe Lys Lys Thr Lys Glu Ile Arg Phe Ser Ile Glu Lys Gly Ile Ala
  1               5                  10                  15

Gly Gln Val Ala Arg Thr Gly Glu Val Leu Asn Ile Pro Asp Ala Tyr
            20                  25                  30

Ala Asp Pro Arg Phe Asn Arg Glu Val Asp Leu Tyr Thr Gly Tyr Thr
        35                  40                  45

Thr Arg Asn Ile Leu Cys Met Pro Ile Val Ser Arg Gly Ser Val Ile
    50                  55                  60

Gly Val Val Gln Met Val Asn Lys Ile Ser Gly Ser Ala Phe Ser Lys
65 70 75 80

Thr Asp Glu Asn Asn Phe Lys Met Phe Ala Val Phe Cys Ala Leu Ala
85 90 95

Leu His Cys Ala Asn Met Tyr His Arg Ile Arg His Ser Glu Cys Ile
100 105 110

Tyr Arg Val Thr Met Glu Lys Leu Ser Tyr His Ser Ile Cys Thr Ser
115 120 125

Glu Glu Trp Gln Gly Leu Met
130 135

<210> 27
<211> 22
<212> PRT
<213> Artificial Sequence

<220>
<223> Z17 in Formula I and Formula II

<220>
<223> Description of Artificial Sequence: Synthetic sequence

<400> 27
Ile Glu Leu Phe His Phe Asp Ile Gly Pro Phe Glu Asn Met Trp Pro
1 5 10 15

Gly Ile Phe Val Tyr Met
20

<210> 28
<211> 50
<212> PRT
<213> Artificial Sequence

<220>
<223> Z18 in Formula I and Formula II

<220>
<223> Description of Artificial Sequence: Synthetic sequence

<400> 28
His Arg Ser Cys Gly Thr Ser Cys Phe Glu Leu Glu Lys Leu Cys Arg
1 5 10 15

Phe Ile Met Ser Val Lys Lys Asn Tyr Arg Arg Val Pro Tyr His Asn
20 25 30

Trp Lys His Ala Val Thr Val Ala His Cys Met Tyr Ala Ile Leu Gln
35 40 45

Asn Asn
50

<210> 29
<211> 36
<212> PRT
<213> Artificial Sequence

<220>
<223> Z19 in Formula I and Formula II

<220>
<223> Description of Artificial Sequence: Synthetic sequence

<400> 29

Leu Phe Thr Asp Leu Glu Arg Lys Gly Leu Leu Ile Ala Cys Leu Cys
1 5 10 15

His Asp Leu Asp His Arg Gly Phe Ser Asn Ser Tyr Leu Gln Lys Phe
20 25 30

Asp His Pro Leu
35

<210> 30

<211> 72

<212> PRT

<213> Artificial Sequence

<220>

<223> Z20 in Formula I and Formula II

<220>

<223> Description of Artificial Sequence: Synthetic sequence

<400> 30

Ala Leu Tyr Ser Thr Ser Thr Met Glu Gln His His Phe Ser Gln Thr
1 5 10 15

Val Ser Ile Leu Gln Leu Glu Gly His Asn Ile Phe Ser Thr Leu Ser
20 25 30

Ser Ser Glu Tyr Glu Gln Val Leu Glu Ile Ile Arg Lys Ala Ile Ile
35 40 45

Ala Thr Asp Leu Ala Leu Tyr Phe Gly Asn Arg Gln Leu Glu Glu Met
50 55 60

Tyr Gln Thr Gly Ser Leu Asn Leu
65 70

<210> 31
<211> 23
<212> PRT
<213> Artificial Sequence

<220>
<223> Z21 in Formula I and Formula II

<220>
<223> Description of Artificial Sequence: Synthetic sequence

<400> 31
Asn Gln Ser His Arg Asp Arg Val Ile Gly Leu Met Met Thr Ala Cys
1 5 10 15

Asp Leu Cys Ser Val Thr Lys
20

<210> 32
<211> 37
<212> PRT
<213> Artificial Sequence

<220>
<223> Z22 in Formula I and Formula II

<220>
<223> Description of Artificial Sequence: Synthetic sequence

<400> 32
Trp Pro Val Thr Lys Leu Thr Ala Asn Asp Ile Tyr Ala Glu Phe Trp
1 5 10 15

Ala Glu Gly Asp Glu Met Lys Lys Leu Gly Ile Gln Pro Ile Pro Met
20 25 30

Met Asp Arg Asp Lys
35

<210> 33
<211> 40
<212> PRT
<213> Artificial Sequence

<220>
<223> Z23 in Formula I and Formula II

<220>
<223> Description of Artificial Sequence: Synthetic sequence

<400> 33
Asp Glu Val Pro Gln Gly Gln Leu Gly Phe Tyr Asn Ala Val Ala Ile
1 5 10 15

Pro Cys Tyr Thr Thr Leu Thr Gln Ile Leu Pro Pro Thr Glu Pro Leu
20 25 30

Leu Lys Ala Cys Arg Asp Asn Leu
35 40

<210> 34
<211> 12
<212> PRT
<213> Artificial Sequence

<220>
<223> Z24 in Formula I and Formula II

<220>
<223> Description of Artificial Sequence: Synthetic sequence

<400> 34

```
Gln Trp Glu Lys Val Ile Arg Gly Glu Glu Thr Ala
  1               5                  10
```

<210> 35
<211> 727
<212> PRT
<213> Artificial Sequence

<220>
<223> Description of Artificial Sequence: Synthetic sequence

<400> 35

```
Leu Thr Asp Glu Lys Val Lys Ala Tyr Leu Ser Leu His Pro Gln Val
  1               5                  10                  15

Leu Asp Glu Phe Val Ser Glu Ser Val Ser Ala Glu Thr Val Glu Lys
             20                  25                  30

Trp Leu Lys Arg Lys Asn Lys Asp Glu Pro Lys Glu Val Ser Arg Tyr
         35                  40                  45

Gln Asp Thr Asn Met Gln Gly Val Val Tyr Glu Leu Asn Ser Tyr Ile
     50                  55                  60

Glu Gln Arg Leu Asp Thr Gly Gly Asp Asn Leu Leu Leu Tyr Glu Leu
 65                  70                  75                  80

Ser Ser Ile Ile Ile Ala Thr Lys Ala Asp Gly Phe Ala Leu Tyr Phe
                 85                  90                  95
```

```
Leu Gly Glu Cys Asn Asn Ser Leu Cys Phe Pro Pro Gly Lys Glu Gly
            100                 105                 110

Pro Arg Leu Ile Pro Ala Gly Pro Ile Thr Gln Gly Thr Thr Ser Ala
        115                 120                 125

Tyr Val Ala Lys Ser Arg Lys Thr Leu Leu Val Glu Asp Ile Leu Gly
    130                 135                 140

Asp Glu Arg Phe Pro Arg Gly Thr Gly Leu Glu Ser Gly Thr Arg Ile
145                 150                 155                 160

Gln Ser Val Leu Cys Leu Pro Ile Val Thr Ala Ile Gly Asp Leu Ile
                165                 170                 175

Gly Ile Leu Glu Leu Tyr Arg His Trp Gly Lys Glu Ala Phe Cys Leu
            180                 185                 190

Ser His Gln Glu Val Ala Thr Ala Asn Leu Ala Trp Ala Ser Val Ala
        195                 200                 205

Ile His Gln Val Gln Val Cys Arg Gly Leu Ala Lys Gln Thr Glu Leu
    210                 215                 220

Asn Asp Phe Leu Leu Asp Val Ser Lys Thr Tyr Phe Asp Asn Ile Val
225                 230                 235                 240

Ala Ile Asp Ser Leu Leu Glu His Ile Met Ile Tyr Ala Lys Asn Leu
                245                 250                 255

Val Asn Ala Asp Arg Cys Ala Leu Phe Gln Val Asp His Lys Asn Lys
            260                 265                 270

Glu Leu Tyr Ser Asp Leu Phe Asp Ile Gly Glu Glu Lys Glu Gly Lys
        275                 280                 285

Pro Phe Lys Lys Thr Lys Glu Ile Arg Phe Ser Ile Glu Lys Gly Ile
    290                 295                 300
```

Ala Gly Gln Val Ala Arg Thr Gly Glu Val Leu Asn Ile Pro Asp Ala
305 310 315 320

Tyr Ala Asp Pro Arg Phe Asn Arg Glu Val Asp Leu Tyr Thr Gly Tyr
325 330 335

Thr Thr Arg Asn Ile Leu Cys Met Pro Ile Val Ser Arg Gly Ser Val
340 345 350

Ile Gly Val Val Gln Met Val Asn Lys Ile Ser Gly Ser Ala Phe Ser
355 360 365

Lys Thr Asp Glu Asn Asn Phe Lys Met Phe Ala Val Phe Cys Ala Leu
370 375 380

Ala Leu His Cys Ala Asn Met Tyr His Arg Ile Arg His Ser Glu Cys
385 390 395 400

Ile Tyr Arg Val Thr Met Glu Lys Leu Ser Tyr His Ser Ile Cys Thr
405 410 415

Ser Glu Glu Trp Gln Gly Leu Met Phe Leu Pro Arg Cys Ile Glu Leu
420 425 430

Phe His Phe Asp Ile Gly Pro Phe Glu Asn Met Trp Pro Gly Ile Phe
435 440 445

Val Tyr Met His Arg Ser Cys Gly Thr Ser Cys Phe Glu Leu Glu Lys
450 455 460

Leu Cys Arg Phe Ile Met Ser Val Lys Lys Asn Tyr Arg Arg Val Pro
465 470 475 480

Tyr His Asn Trp Lys His Ala Val Thr Val Ala His Cys Met Tyr Ala
485 490 495

Ile Leu Gln Asn Asn Leu Phe Thr Asp Leu Glu Arg Lys Gly Leu Leu
500 505 510

Ile Ala Cys Leu Cys His Asp Leu Asp His Arg Gly Phe Ser Asn Ser
515 520 525

Tyr Leu Gln Lys Phe Asp His Pro Leu Ala Leu Tyr Ser Thr Ser Thr
530 535 540

Met Glu Gln His His Phe Ser Gln Thr Val Ser Ile Leu Gln Leu Glu
545 550 555 560

Gly His Asn Ile Phe Ser Thr Leu Ser Ser Ser Glu Tyr Glu Gln Val
565 570 575

Leu Glu Ile Ile Arg Lys Ala Ile Ile Ala Thr Asp Leu Ala Leu Tyr
580 585 590

Phe Gly Asn Arg Gln Leu Glu Glu Met Tyr Gln Thr Gly Ser Leu Asn
595 600 605

Leu Asn Gln Ser His Arg Asp Arg Val Ile Gly Leu Met Met Thr Ala
610 615 620

Cys Asp Leu Cys Ser Val Thr Lys Trp Pro Val Thr Lys Leu Thr Ala
625 630 635 640

Asn Asp Ile Tyr Ala Glu Phe Trp Ala Glu Gly Asp Glu Met Lys Lys
645 650 655

Leu Gly Ile Gln Pro Ile Pro Met Met Asp Arg Asp Lys Asp Glu Val
660 665 670

Pro Gln Gly Gln Leu Gly Phe Tyr Asn Ala Val Ala Ile Pro Cys Tyr
675 680 685

Thr Thr Leu Thr Gln Ile Leu Pro Pro Thr Glu Pro Leu Leu Lys Ala
690 695 700

Cys Arg Asp Asn Leu Gln Trp Glu Lys Val Ile Arg Gly Glu Glu Thr
705 710 715 720

Ala Trp Ile Ser Pro Ala Glu
725

<210> 36
<211> 749
<212> PRT
<213> Artificial Sequence

<220>
<223> Description of Artificial Sequence: Synthetic sequence

<220>
<221> SITE
<222> (38)
<223> Xaa=Asn or Thr

<220>
<221> SITE
<222> (41)
<223> Xaa=Ser or Ala

<220>
<221> SITE
<222> (42)
<223> Xaa=Glu or Lys

<220>
<221> SITE
<222> (45)
<223> Xaa=Ser or Pro

<220>
<221> SITE
<222> (46)
<223> Xaa=Ala or Ser

<220>
<221> SITE
<222> (80)
<223> Xaa=Gln or His

<220>
<221> SITE
<222> (91)
<223> Xaa=Lys or Arg

<220>
<221> SITE
<222> (113)
<223> Xaa=Ile or Val

<220>
<221> SITE
<222> (115)
<223> Xaa=Thr or Ile

<220>
<221> SITE
<222> (119)
<223> Xaa=Ile or Met

<220>
<221> SITE
<222> (123)
<223> Xaa=Lys or Gln

<220>
<221> SITE
<222> (138)
<223> Xaa=Val or Ile

<220>
<221> SITE
<222> (302)
<223> Xaa=Val or Ile

<220>
<221> SITE
<222> (438)
<223> Xaa=Gln or Arg

<220>
<221> SITE
<222> (440)
<223> Xaa=Thr or Asn

<220>
<221> SITE
<222> (443)
<223> Xaa=Val or Ala

<220>
<221> SITE
<222> (445)
<223> Xaa=Leu or Ile

<220>
<221> SITE
<222> (447)
<223> Xaa=Lys or Arg

<220>
<221> SITE
<222> (448)
<223> Xaa=Glu or Asp

<220>
<221> SITE
<222> (471)
<223> Xaa=Val or Ile

<220>
<221> SITE
<222> (522)
<223> Xaa=His or Asn

<220>
<221> SITE
<222> (523)
<223> Xaa=Thr or Gly

<220>
<221> SITE
<222> (560)
<223> Xaa=Thr or Ala

<220>
<221> SITE
<222> (634)
<223> Xaa=Asn or His

<220>
<221> SITE
<222> (658)
<223> Xaa=Pro or Leu

<220>
<221> SITE
<222> (696)
<223> Xaa=Lys or Arg

<220>
<221> SITE
<222> (737)
<223> Xaa=Ser or Asn

<400> 36
```
Leu Thr Asp Glu Lys Val Lys Ala Tyr Leu Ser Leu His Pro Gln Val
 1               5                  10                  15

Leu Asp Glu Phe Val Ser Glu Ser Val Ser Ala Glu Thr Val Glu Lys
            20                  25                  30

Trp Leu Lys Arg Lys Xaa Asn Lys Xaa Xaa Asp Glu Xaa Xaa Pro Lys
        35                  40                  45

Glu Val Ser Arg Tyr Gln Asp Thr Asn Met Gln Gly Val Val Tyr Glu
    50                  55                  60

Leu Asn Ser Tyr Ile Glu Gln Arg Leu Asp Thr Gly Gly Asp Asn Xaa
65                  70                  75                  80

Leu Leu Leu Tyr Glu Leu Ser Ser Ile Ile Xaa Ile Ala Thr Lys Ala
                85                  90                  95

Asp Gly Phe Ala Leu Tyr Phe Leu Gly Glu Cys Asn Asn Ser Leu Cys
            100                 105                 110

Xaa Phe Xaa Pro Pro Gly Xaa Lys Glu Gly Xaa Pro Arg Leu Ile Pro
        115                 120                 125

Ala Gly Pro Ile Thr Gln Gly Thr Thr Xaa Ser Ala Tyr Val Ala Lys
    130                 135                 140

Ser Arg Lys Thr Leu Leu Val Glu Asp Ile Leu Gly Asp Glu Arg Phe
145                 150                 155                 160

Pro Arg Gly Thr Gly Leu Glu Ser Gly Thr Arg Ile Gln Ser Val Leu
                165                 170                 175
```

```
Cys Leu Pro Ile Val Thr Ala Ile Gly Asp Leu Ile Gly Ile Leu Glu
            180                 185                 190

Leu Tyr Arg His Trp Gly Lys Glu Ala Phe Cys Leu Ser His Gln Glu
        195                 200                 205

Val Ala Thr Ala Asn Leu Ala Trp Ala Ser Val Ala Ile His Gln Val
    210                 215                 220

Gln Val Cys Arg Gly Leu Ala Lys Gln Thr Glu Leu Asn Asp Phe Leu
225                 230                 235                 240

Leu Asp Val Ser Lys Thr Tyr Phe Asp Asn Ile Val Ala Ile Asp Ser
                245                 250                 255

Leu Leu Glu His Ile Met Ile Tyr Ala Lys Asn Leu Val Asn Ala Asp
            260                 265                 270

Arg Cys Ala Leu Phe Gln Val Asp His Lys Asn Lys Glu Leu Tyr Ser
        275                 280                 285

Asp Leu Phe Asp Ile Gly Glu Glu Lys Glu Gly Lys Pro Xaa Phe Lys
    290                 295                 300

Lys Thr Lys Glu Ile Arg Phe Ser Ile Glu Lys Gly Ile Ala Gly Gln
305                 310                 315                 320

Val Ala Arg Thr Gly Glu Val Leu Asn Ile Pro Asp Ala Tyr Ala Asp
                325                 330                 335

Pro Arg Phe Asn Arg Glu Val Asp Leu Tyr Thr Gly Tyr Thr Thr Arg
            340                 345                 350

Asn Ile Leu Cys Met Pro Ile Val Ser Arg Gly Ser Val Ile Gly Val
        355                 360                 365

Val Gln Met Val Asn Lys Ile Ser Gly Ser Ala Phe Ser Lys Thr Asp
    370                 375                 380
```

Glu Asn Asn Phe Lys Met Phe Ala Val Phe Cys Ala Leu Ala Leu His
385 390 395 400

Cys Ala Asn Met Tyr His Arg Ile Arg His Ser Glu Cys Ile Tyr Arg
405 410 415

Val Thr Met Glu Lys Leu Ser Tyr His Ser Ile Cys Thr Ser Glu Glu
420 425 430

Trp Gln Gly Leu Met Xaa Phe Xaa Leu Pro Xaa Arg Xaa Cys Xaa Xaa
435 440 445

Ile Glu Leu Phe His Phe Asp Ile Gly Pro Phe Glu Asn Met Trp Pro
450 455 460

Gly Ile Phe Val Tyr Met Xaa His Arg Ser Cys Gly Thr Ser Cys Phe
465 470 475 480

Glu Leu Glu Lys Leu Cys Arg Phe Ile Met Ser Val Lys Lys Asn Tyr
485 490 495

Arg Arg Val Pro Tyr His Asn Trp Lys His Ala Val Thr Val Ala His
500 505 510

Cys Met Tyr Ala Ile Leu Gln Asn Asn Xaa Xaa Leu Phe Thr Asp Leu
515 520 525

Glu Arg Lys Gly Leu Leu Ile Ala Cys Leu Cys His Asp Leu Asp His
530 535 540

Arg Gly Phe Ser Asn Ser Tyr Leu Gln Lys Phe Asp His Pro Leu Xaa
545 550 555 560

Ala Leu Tyr Ser Thr Ser Thr Met Glu Gln His His Phe Ser Gln Thr
565 570 575

Val Ser Ile Leu Gln Leu Glu Gly His Asn Ile Phe Ser Thr Leu Ser
580 585 590

Ser Ser Glu Tyr Glu Gln Val Leu Glu Ile Ile Arg Lys Ala Ile Ile
595 600 605

Ala Thr Asp Leu Ala Leu Tyr Phe Gly Asn Arg Lys Gln Leu Glu Glu
610 615 620

Met Tyr Gln Thr Gly Ser Leu Asn Leu Xaa Asn Gln Ser His Arg Asp
625 630 635 640

Arg Val Ile Gly Leu Met Met Thr Ala Cys Asp Leu Cys Ser Val Thr
645 650 655

Lys Xaa Trp Pro Val Thr Lys Leu Thr Ala Asn Asp Ile Tyr Ala Glu
660 665 670

Phe Trp Ala Glu Gly Asp Glu Met Lys Lys Leu Gly Ile Gln Pro Ile
675 680 685

Pro Met Met Asp Arg Asp Lys Xaa Asp Glu Val Pro Gln Gly Gln Leu
690 695 700

Gly Phe Tyr Asn Ala Val Ala Ile Pro Cys Tyr Thr Thr Leu Thr Gln
705 710 715 720

Ile Leu Pro Pro Thr Glu Pro Leu Leu Lys Ala Cys Arg Asp Asn Leu
725 730 735

Xaa Gln Trp Glu Lys Val Ile Arg Gly Glu Glu Thr Ala
740 745

<210> 37
<211> 750
<212> PRT
<213> Artificial Sequence

<220>
<223> Description of Artificial Sequence: Synthetic sequence

<220>
<221> SITE
<222> (1)
<223> Xaa=Ser or Gly

<220>
<221> SITE
<222> (39)
<223> Xaa=Asn or Thr

<220>
<221> SITE
<222> (42)
<223> Xaa=Ser or Ala

<220>
<221> SITE
<222> (43)
<223> Xaa=Glu or Lys

<220>
<221> SITE
<222> (46)
<223> Xaa=Ser or Pro

<220>
<221> SITE
<222> (47)
<223> Xaa=Ala or Ser

<220>
<221> SITE
<222> (81)
<223> Xaa=Gln or His

<220>
<221> SITE
<222> (92)
<223> Xaa=Lys or Arg

<220>
<221> SITE
<222> (114)
<223> Xaa=Ile or Val

<220>
<221> SITE
<222> (116)
<223> Xaa=Thr or Ile

<220>
<221> SITE
<222> (120)
<223> Xaa=Ile or Met

<220>
<221> SITE
<222> (124)
<223> Xaa=Lys or Gln

<220>
<221> SITE
<222> (139)
<223> Xaa=Val or Ile

<220>
<221> SITE
<222> (303)
<223> Xaa=Val or Ile

<220>
<221> SITE
<222> (439)
<223> Xaa=Gln or Arg

<220>
<221> SITE
<222> (441)
<223> Xaa=Thr or Asn

<220>
<221> SITE
<222> (444)
<223> Xaa=Val or Ala

<220>
<221> SITE
<222> (446)
<223> Xaa=Leu or Ile

<220>
<221> SITE
<222> (448)
<223> Xaa=Lys or Arg

<220>
<221> SITE
<222> (449)
<223> Xaa=Glu or Asp

<220>
<221> SITE
<222> (472)
<223> Xaa=Val or Ile

<220>
<221> SITE
<222> (523)
<223> Xaa=His or Asn

<220>
<221> SITE
<222> (524)
<223> Xaa=Thr or Gly

<220>
<221> SITE
<222> (561)
<223> Xaa=Thr or Ala

<220>
<221> SITE
<222> (635)
<223> Xaa=Asn or His

<220>
<221> SITE
<222> (659)
<223> Xaa=Pro or Leu

<220>
<221> SITE
<222> (697)
<223> Xaa=Lys or Arg

<220>
<221> SITE
<222> (738)
<223> Xaa=Ser or Asn

<400> 37
Xaa Leu Thr Asp Glu Lys Val Lys Ala Tyr Leu Ser Leu His Pro Gln
1 5 10 15

Val Leu Asp Glu Phe Val Ser Glu Ser Val Ser Ala Glu Thr Val Glu
20 25 30

Lys Trp Leu Lys Arg Lys Xaa Asn Lys Xaa Xaa Asp Glu Xaa Xaa Pro
35 40 45

Lys Glu Val Ser Arg Tyr Gln Asp Thr Asn Met Gln Gly Val Val Tyr
50 55 60

Glu Leu Asn Ser Tyr Ile Glu Gln Arg Leu Asp Thr Gly Gly Asp Asn
65 70 75 80

Xaa Leu Leu Leu Tyr Glu Leu Ser Ser Ile Ile Xaa Ile Ala Thr Lys
85 90 95

Ala Asp Gly Phe Ala Leu Tyr Phe Leu Gly Glu Cys Asn Asn Ser Leu
100 105 110

Cys Xaa Phe Xaa Pro Pro Gly Xaa Lys Glu Gly Xaa Pro Arg Leu Ile
115 120 125

Pro Ala Gly Pro Ile Thr Gln Gly Thr Thr Xaa Ser Ala Tyr Val Ala
130 135 140

Lys Ser Arg Lys Thr Leu Leu Val Glu Asp Ile Leu Gly Asp Glu Arg
145 150 155 160

Phe Pro Arg Gly Thr Gly Leu Glu Ser Gly Thr Arg Ile Gln Ser Val
165 170 175

Leu Cys Leu Pro Ile Val Thr Ala Ile Gly Asp Leu Ile Gly Ile Leu
180 185 190

Glu Leu Tyr Arg His Trp Gly Lys Glu Ala Phe Cys Leu Ser His Gln
195 200 205

Glu Val Ala Thr Ala Asn Leu Ala Trp Ala Ser Val Ala Ile His Gln
210 215 220

Val Gln Val Cys Arg Gly Leu Ala Lys Gln Thr Glu Leu Asn Asp Phe
225 230 235 240

Leu Leu Asp Val Ser Lys Thr Tyr Phe Asp Asn Ile Val Ala Ile Asp
245 250 255

Ser Leu Leu Glu His Ile Met Ile Tyr Ala Lys Asn Leu Val Asn Ala
260 265 270

Asp Arg Cys Ala Leu Phe Gln Val Asp His Lys Asn Lys Glu Leu Tyr
275 280 285

Ser Asp Leu Phe Asp Ile Gly Glu Glu Lys Glu Gly Lys Pro Xaa Phe
290 295 300

Lys Lys Thr Lys Glu Ile Arg Phe Ser Ile Glu Lys Gly Ile Ala Gly
305 310 315 320

Gln Val Ala Arg Thr Gly Glu Val Leu Asn Ile Pro Asp Ala Tyr Ala
325 330 335

Asp Pro Arg Phe Asn Arg Glu Val Asp Leu Tyr Thr Gly Tyr Thr Thr
340 345 350

Arg Asn Ile Leu Cys Met Pro Ile Val Ser Arg Gly Ser Val Ile Gly
355 360 365

Val Val Gln Met Val Asn Lys Ile Ser Gly Ser Ala Phe Ser Lys Thr
370 375 380

Asp Glu Asn Asn Phe Lys Met Phe Ala Val Phe Cys Ala Leu Ala Leu
385 390 395 400

His Cys Ala Asn Met Tyr His Arg Ile Arg His Ser Glu Cys Ile Tyr
405 410 415

Arg Val Thr Met Glu Lys Leu Ser Tyr His Ser Ile Cys Thr Ser Glu
420 425 430

Glu Trp Gln Gly Leu Met Xaa Phe Xaa Leu Pro Xaa Arg Xaa Cys Xaa
435 440 445

Xaa Ile Glu Leu Phe His Phe Asp Ile Gly Pro Phe Glu Asn Met Trp
450 455 460

Pro Gly Ile Phe Val Tyr Met Xaa His Arg Ser Cys Gly Thr Ser Cys
465 470 475 480

Phe Glu Leu Glu Lys Leu Cys Arg Phe Ile Met Ser Val Lys Lys Asn
485 490 495

Tyr Arg Arg Val Pro Tyr His Asn Trp Lys His Ala Val Thr Val Ala
500 505 510

His Cys Met Tyr Ala Ile Leu Gln Asn Asn Xaa Xaa Leu Phe Thr Asp
515 520 525

Leu Glu Arg Lys Gly Leu Leu Ile Ala Cys Leu Cys His Asp Leu Asp
530 535 540

His Arg Gly Phe Ser Asn Ser Tyr Leu Gln Lys Phe Asp His Pro Leu
545 550 555 560

Xaa Ala Leu Tyr Ser Thr Ser Thr Met Glu Gln His His Phe Ser Gln
565 570 575

Thr Val Ser Ile Leu Gln Leu Glu Gly His Asn Ile Phe Ser Thr Leu
580 585 590

Ser Ser Ser Glu Tyr Glu Gln Val Leu Glu Ile Ile Arg Lys Ala Ile
595 600 605

Ile Ala Thr Asp Leu Ala Leu Tyr Phe Gly Asn Arg Lys Gln Leu Glu
610 615 620

Glu Met Tyr Gln Thr Gly Ser Leu Asn Leu Xaa Asn Gln Ser His Arg
625 630 635 640

Asp Arg Val Ile Gly Leu Met Met Thr Ala Cys Asp Leu Cys Ser Val
645 650 655

Thr Lys Xaa Trp Pro Val Thr Lys Leu Thr Ala Asn Asp Ile Tyr Ala
660 665 670

Glu Phe Trp Ala Glu Gly Asp Glu Met Lys Lys Leu Gly Ile Gln Pro
675 680 685

Ile Pro Met Met Asp Arg Asp Lys Xaa Asp Glu Val Pro Gln Gly Gln
690 695 700

Leu Gly Phe Tyr Asn Ala Val Ala Ile Pro Cys Tyr Thr Thr Leu Thr
705 710 715 720

Gln Ile Leu Pro Pro Thr Glu Pro Leu Leu Lys Ala Cys Arg Asp Asn
725 730 735

Leu Xaa Gln Trp Glu Lys Val Ile Arg Gly Glu Glu Thr Ala
740 745 750

<210> 38
<211> 754
<212> PRT
<213> Artificial Sequence

<220>
<223> Description of Artificial Sequence: Synthetic
sequence

<220>
<221> SITE
<222> (1)
<223> Xaa=Ser or Gly

<220>
<221> SITE
<222> (39)
<223> Xaa=Asn or Thr

<220>
<221> SITE
<222> (42)
<223> Xaa=Ser or Ala

<220>
<221> SITE
<222> (43)
<223> Xaa=Glu or Lys

<220>
<221> SITE
<222> (46)
<223> Xaa=Ser or Pro

<220>
<221> SITE
<222> (47)
<223> Xaa=Ala or Ser

<220>
<221> SITE
<222> (81)
<223> Xaa=Gln or His

<220>
<221> SITE
<222> (92)
<223> Xaa=Lys or Arg

<220>
<221> SITE
<222> (114)
<223> Xaa=Ile or Val

<220>
<221> SITE
<222> (116)
<223> Xaa=Thr or Ile

<220>
<221> SITE
<222> (120)
<223> Xaa=Ile or Met

<220>
<221> SITE
<222> (124)
<223> Xaa=Lys or Gln

<220>
<221> SITE
<222> (139)
<223> Xaa=Val or Ile

<220>
<221> SITE
<222> (303)
<223> Xaa=Val or Ile

<220>
<221> SITE
<222> (439)
<223> Xaa=Gln or Arg

<220>
<221> SITE
<222> (441)
<223> Xaa=Thr or Asn

<220>
<221> SITE
<222> (444)
<223> Xaa=Val or Ala

<220>
<221> SITE
<222> (446)
<223> Xaa=Leu or Ile

<220>
<221> SITE
<222> (448)
<223> Xaa=Lys or Arg

<220>
<221> SITE
<222> (449)
<223> Xaa=Glu or Asp

<220>
<221> SITE
<222> (472)
<223> Xaa=Val or Ile

<220>
<221> SITE
<222> (523)
<223> Xaa=His or Asn

<220>
<221> SITE
<222> (524)
<223> Xaa=Thr or Gly

<220>
<221> SITE
<222> (561)
<223> Xaa=Thr or Ala

<220>
<221> SITE
<222> (635)
<223> Xaa=Asn or His

<220>
<221> SITE
<222> (659)
<223> Xaa=Pro or Leu

<220>
<221> SITE
<222> (697)
<223> Xaa=Lys or Arg

<220>
<221> SITE
<222> (738)
<223> Xaa=Ser or Asn

<220>
<221> SITE
<222> (751)
<223> Xaa=Thr or Met

<400> 38
```
Xaa Leu Thr Asp Glu Lys Val Lys Ala Tyr Leu Ser Leu His Pro Gln
 1               5                  10                  15
```

```
Val Leu Asp Glu Phe Val Ser Glu Ser Val Ser Ala Glu Thr Val Glu
            20                  25                  30

Lys Trp Leu Lys Arg Lys Xaa Asn Lys Xaa Xaa Asp Glu Xaa Xaa Pro
        35                  40                  45

Lys Glu Val Ser Arg Tyr Gln Asp Thr Asn Met Gln Gly Val Val Tyr
    50                  55                  60

Glu Leu Asn Ser Tyr Ile Glu Gln Arg Leu Asp Thr Gly Gly Asp Asn
65                  70                  75                  80

Xaa Leu Leu Leu Tyr Glu Leu Ser Ser Ile Ile Xaa Ile Ala Thr Lys
                85                  90                  95

Ala Asp Gly Phe Ala Leu Tyr Phe Leu Gly Glu Cys Asn Asn Ser Leu
            100                 105                 110

Cys Xaa Phe Xaa Pro Pro Gly Xaa Lys Glu Gly Xaa Pro Arg Leu Ile
        115                 120                 125

Pro Ala Gly Pro Ile Thr Gln Gly Thr Thr Xaa Ser Ala Tyr Val Ala
    130                 135                 140

Lys Ser Arg Lys Thr Leu Leu Val Glu Asp Ile Leu Gly Asp Glu Arg
145                 150                 155                 160

Phe Pro Arg Gly Thr Gly Leu Glu Ser Gly Thr Arg Ile Gln Ser Val
                165                 170                 175

Leu Cys Leu Pro Ile Val Thr Ala Ile Gly Asp Leu Ile Gly Ile Leu
            180                 185                 190

Glu Leu Tyr Arg His Trp Gly Lys Glu Ala Phe Cys Leu Ser His Gln
        195                 200                 205

Glu Val Ala Thr Ala Asn Leu Ala Trp Ala Ser Val Ala Ile His Gln
    210                 215                 220
```

Val Gln Val Cys Arg Gly Leu Ala Lys Gln Thr Glu Leu Asn Asp Phe
225                 230                 235                 240

Leu Leu Asp Val Ser Lys Thr Tyr Phe Asp Asn Ile Val Ala Ile Asp
                245                 250                 255

Ser Leu Leu Glu His Ile Met Ile Tyr Ala Lys Asn Leu Val Asn Ala
            260                 265                 270

Asp Arg Cys Ala Leu Phe Gln Val Asp His Lys Asn Lys Glu Leu Tyr
        275                 280                 285

Ser Asp Leu Phe Asp Ile Gly Glu Glu Lys Glu Gly Lys Pro Xaa Phe
    290                 295                 300

Lys Lys Thr Lys Glu Ile Arg Phe Ser Ile Glu Lys Gly Ile Ala Gly
305                 310                 315                 320

Gln Val Ala Arg Thr Gly Glu Val Leu Asn Ile Pro Asp Ala Tyr Ala
                325                 330                 335

Asp Pro Arg Phe Asn Arg Glu Val Asp Leu Tyr Thr Gly Tyr Thr Thr
            340                 345                 350

Arg Asn Ile Leu Cys Met Pro Ile Val Ser Arg Gly Ser Val Ile Gly
        355                 360                 365

Val Val Gln Met Val Asn Lys Ile Ser Gly Ser Ala Phe Ser Lys Thr
    370                 375                 380

Asp Glu Asn Asn Phe Lys Met Phe Ala Val Phe Cys Ala Leu Ala Leu
385                 390                 395                 400

His Cys Ala Asn Met Tyr His Arg Ile Arg His Ser Glu Cys Ile Tyr
                405                 410                 415

Arg Val Thr Met Glu Lys Leu Ser Tyr His Ser Ile Cys Thr Ser Glu
            420                 425                 430

Glu Trp Gln Gly Leu Met Xaa Phe Xaa Leu Pro Xaa Arg Xaa Cys Xaa
435 440 445

Xaa Ile Glu Leu Phe His Phe Asp Ile Gly Pro Phe Glu Asn Met Trp
450 455 460

Pro Gly Ile Phe Val Tyr Met Xaa His Arg Ser Cys Gly Thr Ser Cys
465 470 475 480

Phe Glu Leu Glu Lys Leu Cys Arg Phe Ile Met Ser Val Lys Lys Asn
485 490 495

Tyr Arg Arg Val Pro Tyr His Asn Trp Lys His Ala Val Thr Val Ala
500 505 510

His Cys Met Tyr Ala Ile Leu Gln Asn Asn Xaa Xaa Leu Phe Thr Asp
515 520 525

Leu Glu Arg Lys Gly Leu Leu Ile Ala Cys Leu Cys His Asp Leu Asp
530 535 540

His Arg Gly Phe Ser Asn Ser Tyr Leu Gln Lys Phe Asp His Pro Leu
545 550 555 560

Xaa Ala Leu Tyr Ser Thr Ser Thr Met Glu Gln His His Phe Ser Gln
565 570 575

Thr Val Ser Ile Leu Gln Leu Glu Gly His Asn Ile Phe Ser Thr Leu
580 585 590

Ser Ser Ser Glu Tyr Glu Gln Val Leu Glu Ile Ile Arg Lys Ala Ile
595 600 605

Ile Ala Thr Asp Leu Ala Leu Tyr Phe Gly Asn Arg Lys Gln Leu Glu
610 615 620

Glu Met Tyr Gln Thr Gly Ser Leu Asn Leu Xaa Asn Gln Ser His Arg
625 630 635 640

Asp Arg Val Ile Gly Leu Met Met Thr Ala Cys Asp Leu Cys Ser Val
645 650 655

Thr Lys Xaa Trp Pro Val Thr Lys Leu Thr Ala Asn Asp Ile Tyr Ala
660 665 670

Glu Phe Trp Ala Glu Gly Asp Glu Met Lys Lys Leu Gly Ile Gln Pro
675 680 685

Ile Pro Met Met Asp Arg Asp Lys Xaa Asp Glu Val Pro Gln Gly Gln
690 695 700

Leu Gly Phe Tyr Asn Ala Val Ala Ile Pro Cys Tyr Thr Thr Leu Thr
705 710 715 720

Gln Ile Leu Pro Pro Thr Glu Pro Leu Leu Lys Ala Cys Arg Asp Asn
725 730 735

Leu Xaa Gln Trp Glu Lys Val Ile Arg Gly Glu Glu Thr Ala Xaa Trp
740 745 750

Ile Ser

<210> 39
<211> 759
<212> PRT
<213> Artificial Sequence

<220>
<223> Description of Artificial Sequence: Synthetic sequence

<220>
<221> SITE
<222> (1)
<223> Xaa=Ser or Gly

<220>
<221> SITE
<222> (39)
<223> Xaa=Asn or Thr

<220>
<221> SITE
<222> (42)
<223> Xaa=Ser or Ala

<220>
<221> SITE
<222> (43)
<223> Xaa=Glu or Lys

<220>
<221> SITE
<222> (46)
<223> Xaa=Ser or Pro

<220>
<221> SITE
<222> (47)
<223> Xaa=Ala or Ser

<220>
<221> SITE
<222> (81)
<223> Xaa=Gln or His

<220>
<221> SITE
<222> (92)
<223> Xaa=Lys or Arg

<220>
<221> SITE
<222> (114)
<223> Xaa=Ile or Val

<220>
<221> SITE
<222> (116)
<223> Xaa=Thr or Ile

<220>
<221> SITE
<222> (120)
<223> Xaa=Ile or Met

<220>
<221> SITE
<222> (124)
<223> Xaa=Lys or Gln

<220>
<221> SITE
<222> (139)
<223> Xaa=Val or Ile

<220>
<221> SITE
<222> (303)
<223> Xaa=Val or Ile

<220>
<221> SITE
<222> (439)
<223> Xaa=Gln or Arg

<220>
<221> SITE
<222> (441)
<223> Xaa=Thr or Asn

<220>
<221> SITE
<222> (444)
<223> Xaa=Val or Ala

<220>
<221> SITE
<222> (446)
<223> Xaa=Leu or Ile

<220>
<221> SITE
<222> (448)
<223> Xaa=Lys or Arg

<220>
<221> SITE
<222> (449)
<223> Xaa=Glu or Asp

<220>
<221> SITE
<222> (472)
<223> Xaa=Val or Ile

<220>
<221> SITE
<222> (523)
<223> Xaa=His or Asn

<220>
<221> SITE
<222> (524)
<223> Xaa=Thr or Gly

<220>
<221> SITE
<222> (561)
<223> Xaa=Thr or Ala

<220>
<221> SITE
<222> (635)
<223> Xaa=Asn or His

<220>
<221> SITE
<222> (659)
<223> Xaa=Pro or Leu

<220>
<221> SITE
<222> (697)
<223> Xaa=Lys or Arg

<220>
<221> SITE
<222> (738)
<223> Xaa=Ser or Asn

<220>
<221> SITE
<222> (751)
<223> Xaa=Thr or Met

<220>
<221> SITE
<222> (755)
<223> Xaa=Ser or Gly

<220>
<221> SITE
<222> (757)
<223> Xaa=Ser or Gly

<220>
<221> SITE
<222> (758)
<223> Xaa=Val or Pro

<400> 39

```
Xaa Leu Thr Asp Glu Lys Val Lys Ala Tyr Leu Ser Leu His Pro Gln
  1               5                  10                  15

Val Leu Asp Glu Phe Val Ser Glu Ser Val Ser Ala Glu Thr Val Glu
             20                  25                  30

Lys Trp Leu Lys Arg Lys Xaa Asn Lys Xaa Xaa Asp Glu Xaa Xaa Pro
         35                  40                  45

Lys Glu Val Ser Arg Tyr Gln Asp Thr Asn Met Gln Gly Val Val Tyr
     50                  55                  60

Glu Leu Asn Ser Tyr Ile Glu Gln Arg Leu Asp Thr Gly Gly Asp Asn
 65                  70                  75                  80

Xaa Leu Leu Leu Tyr Glu Leu Ser Ser Ile Ile Xaa Ile Ala Thr Lys
                 85                  90                  95

Ala Asp Gly Phe Ala Leu Tyr Phe Leu Gly Glu Cys Asn Asn Ser Leu
             100                 105                 110
```

Cys Xaa Phe Xaa Pro Pro Gly Xaa Lys Glu Gly Xaa Pro Arg Leu Ile
        115                 120                 125

Pro Ala Gly Pro Ile Thr Gln Gly Thr Thr Xaa Ser Ala Tyr Val Ala
    130                 135                 140

Lys Ser Arg Lys Thr Leu Leu Val Glu Asp Ile Leu Gly Asp Glu Arg
145                 150                 155                 160

Phe Pro Arg Gly Thr Gly Leu Glu Ser Gly Thr Arg Ile Gln Ser Val
                165                 170                 175

Leu Cys Leu Pro Ile Val Thr Ala Ile Gly Asp Leu Ile Gly Ile Leu
            180                 185                 190

Glu Leu Tyr Arg His Trp Gly Lys Glu Ala Phe Cys Leu Ser His Gln
        195                 200                 205

Glu Val Ala Thr Ala Asn Leu Ala Trp Ala Ser Val Ala Ile His Gln
    210                 215                 220

Val Gln Val Cys Arg Gly Leu Ala Lys Gln Thr Glu Leu Asn Asp Phe
225                 230                 235                 240

Leu Leu Asp Val Ser Lys Thr Tyr Phe Asp Asn Ile Val Ala Ile Asp
                245                 250                 255

Ser Leu Leu Glu His Ile Met Ile Tyr Ala Lys Asn Leu Val Asn Ala
            260                 265                 270

Asp Arg Cys Ala Leu Phe Gln Val Asp His Lys Asn Lys Glu Leu Tyr
        275                 280                 285

Ser Asp Leu Phe Asp Ile Gly Glu Glu Lys Glu Gly Lys Pro Xaa Phe
    290                 295                 300

Lys Lys Thr Lys Glu Ile Arg Phe Ser Ile Glu Lys Gly Ile Ala Gly
305                 310                 315                 320

```
Gln Val Ala Arg Thr Gly Glu Val Leu Asn Ile Pro Asp Ala Tyr Ala
                325                 330                 335

Asp Pro Arg Phe Asn Arg Glu Val Asp Leu Tyr Thr Gly Tyr Thr Thr
            340                 345                 350

Arg Asn Ile Leu Cys Met Pro Ile Val Ser Arg Gly Ser Val Ile Gly
        355                 360                 365

Val Val Gln Met Val Asn Lys Ile Ser Gly Ser Ala Phe Ser Lys Thr
    370                 375                 380

Asp Glu Asn Asn Phe Lys Met Phe Ala Val Phe Cys Ala Leu Ala Leu
385                 390                 395                 400

His Cys Ala Asn Met Tyr His Arg Ile Arg His Ser Glu Cys Ile Tyr
                405                 410                 415

Arg Val Thr Met Glu Lys Leu Ser Tyr His Ser Ile Cys Thr Ser Glu
            420                 425                 430

Glu Trp Gln Gly Leu Met Xaa Phe Xaa Leu Pro Xaa Arg Xaa Cys Xaa
        435                 440                 445

Xaa Ile Glu Leu Phe His Phe Asp Ile Gly Pro Phe Glu Asn Met Trp
    450                 455                 460

Pro Gly Ile Phe Val Tyr Met Xaa His Arg Ser Cys Gly Thr Ser Cys
465                 470                 475                 480

Phe Glu Leu Glu Lys Leu Cys Arg Phe Ile Met Ser Val Lys Lys Asn
                485                 490                 495

Tyr Arg Arg Val Pro Tyr His Asn Trp Lys His Ala Val Thr Val Ala
            500                 505                 510

His Cys Met Tyr Ala Ile Leu Gln Asn Asn Xaa Xaa Leu Phe Thr Asp
        515                 520                 525
```

```
Leu Glu Arg Lys Gly Leu Leu Ile Ala Cys Leu Cys His Asp Leu Asp
    530                 535                 540

His Arg Gly Phe Ser Asn Ser Tyr Leu Gln Lys Phe Asp His Pro Leu
545                 550                 555                 560

Xaa Ala Leu Tyr Ser Thr Ser Thr Met Glu Gln His His Phe Ser Gln
                565                 570                 575

Thr Val Ser Ile Leu Gln Leu Glu Gly His Asn Ile Phe Ser Thr Leu
            580                 585                 590

Ser Ser Ser Glu Tyr Glu Gln Val Leu Glu Ile Ile Arg Lys Ala Ile
        595                 600                 605

Ile Ala Thr Asp Leu Ala Leu Tyr Phe Gly Asn Arg Lys Gln Leu Glu
    610                 615                 620

Glu Met Tyr Gln Thr Gly Ser Leu Asn Leu Xaa Asn Gln Ser His Arg
625                 630                 635                 640

Asp Arg Val Ile Gly Leu Met Met Thr Ala Cys Asp Leu Cys Ser Val
                645                 650                 655

Thr Lys Xaa Trp Pro Val Thr Lys Leu Thr Ala Asn Asp Ile Tyr Ala
            660                 665                 670

Glu Phe Trp Ala Glu Gly Asp Glu Met Lys Lys Leu Gly Ile Gln Pro
        675                 680                 685

Ile Pro Met Met Asp Arg Asp Lys Xaa Asp Glu Val Pro Gln Gly Gln
    690                 695                 700

Leu Gly Phe Tyr Asn Ala Val Ala Ile Pro Cys Tyr Thr Thr Leu Thr
705                 710                 715                 720

Gln Ile Leu Pro Pro Thr Glu Pro Leu Leu Lys Ala Cys Arg Asp Asn
                725                 730                 735
```

```
Leu Xaa Gln Trp Glu Lys Val Ile Arg Gly Glu Glu Thr Ala Xaa Trp
            740                 745                 750

Ile Ser Xaa Pro Xaa Xaa Ala
        755
```

<210> 40
<211> 767
<212> PRT
<213> Artificial Sequence

<220>
<223> Description of Artificial Sequence: Synthetic sequence

<220>
<221> SITE
<222> (1)
<223> Xaa=Ser or Gly

<220>
<221> SITE
<222> (39)
<223> Xaa=Asn or Thr

<220>
<221> SITE
<222> (42)
<223> Xaa=Ser or Ala

<220>
<221> SITE
<222> (43)
<223> Xaa=Glu or Lys

<220>
<221> SITE
<222> (46)
<223> Xaa=Ser or Pro

<220>
<221> SITE
<222> (47)
<223> Xaa=Ala or Ser

<220>
<221> SITE
<222> (81)
<223> Xaa=Gln or His

<220>
<221> SITE
<222> (92)
<223> Xaa=Lys or Arg

<220>
<221> SITE
<222> (114)
<223> Xaa=Ile or Val

<220>
<221> SITE
<222> (116)
<223> Xaa=Thr or Ile

<220>
<221> SITE
<222> (120)
<223> Xaa=Ile or Met

<220>
<221> SITE
<222> (124)
<223> Xaa=Lys or Gln

<220>
<221> SITE
<222> (139)
<223> Xaa=Val or Ile

<220>
<221> SITE
<222> (303)
<223> Xaa=Val or Ile

<220>
<221> SITE
<222> (439)
<223> Xaa=Gln or Arg

<220>
<221> SITE
<222> (441)
<223> Xaa=Thr or Asn

<220>
<221> SITE
<222> (444)
<223> Xaa=Val or Ala

<220>
<221> SITE
<222> (446)
<223> Xaa=Leu or Ile

<220>
<221> SITE
<222> (448)
<223> Xaa=Lys or Arg

<220>
<221> SITE
<222> (449)
<223> Xaa=Glu or Asp

<220>
<221> SITE
<222> (472)
<223> Xaa=Val or Ile

<220>
<221> SITE
<222> (523)
<223> Xaa=His or Asn

<220>
<221> SITE
<222> (524)
<223> Xaa=Thr or Gly

<220>
<221> SITE
<222> (561)
<223> Xaa=Thr or Ala

<220>
<221> SITE
<222> (635)
<223> Xaa=Asn or His

<220>
<221> SITE
<222> (659)
<223> Xaa=Pro or Leu

<220>
<221> SITE
<222> (697)
<223> Xaa=Lys or Arg

<220>
<221> SITE
<222> (738)
<223> Xaa=Ser or Asn

<220>
<221> SITE
<222> (751)
<223> Xaa=Thr or Met

<220>
<221> SITE
<222> (755)
<223> Xaa=Ser or Gly

<220>
<221> SITE
<222> (757)
<223> Xaa=Ser or Gly

<220>
<221> SITE
<222> (758)
<223> Xaa=Val or Pro

<220>
<221> SITE
<222> (760)
<223> Xaa=Gln or Pro

<220>
<221> SITE
<222> (761)
<223> Xaa=Lys or Ser

<220>
<221> SITE
<222> (762)
<223> Xaa=Ala or Lys

<220>
<221> SITE
<222> (763)
<223> Xaa=Ala or Ser

<220>
<221> SITE
<222> (764)
<223> Xaa=Ala or Thr

<220>
<221> SITE
<222> (765)
<223> Xaa=Ser or Pro

<220>
<221> SITE
<222> (767)
<223> Xaa=Asp or Lys

<400> 40
Xaa Leu Thr Asp Glu Lys Val Lys Ala Tyr Leu Ser Leu His Pro Gln
1 5 10 15

Val Leu Asp Glu Phe Val Ser Glu Ser Val Ser Ala Glu Thr Val Glu
20 25 30

Lys Trp Leu Lys Arg Lys Xaa Asn Lys Xaa Xaa Asp Glu Xaa Xaa Pro
35 40 45

Lys Glu Val Ser Arg Tyr Gln Asp Thr Asn Met Gln Gly Val Val Tyr
50 55 60

Glu Leu Asn Ser Tyr Ile Glu Gln Arg Leu Asp Thr Gly Gly Asp Asn
65 70 75 80

Xaa Leu Leu Leu Tyr Glu Leu Ser Ser Ile Ile Xaa Ile Ala Thr Lys
85 90 95

Ala Asp Gly Phe Ala Leu Tyr Phe Leu Gly Glu Cys Asn Asn Ser Leu
100 105 110

Cys Xaa Phe Xaa Pro Pro Gly Xaa Lys Glu Gly Xaa Pro Arg Leu Ile
115 120 125

Pro Ala Gly Pro Ile Thr Gln Gly Thr Thr Xaa Ser Ala Tyr Val Ala
130 135 140

Lys Ser Arg Lys Thr Leu Leu Val Glu Asp Ile Leu Gly Asp Glu Arg
145 150 155 160

Phe Pro Arg Gly Thr Gly Leu Glu Ser Gly Thr Arg Ile Gln Ser Val
165 170 175

Leu Cys Leu Pro Ile Val Thr Ala Ile Gly Asp Leu Ile Gly Ile Leu
180 185 190

Glu Leu Tyr Arg His Trp Gly Lys Glu Ala Phe Cys Leu Ser His Gln
195 200 205

Glu Val Ala Thr Ala Asn Leu Ala Trp Ala Ser Val Ala Ile His Gln
210 215 220

Val Gln Val Cys Arg Gly Leu Ala Lys Gln Thr Glu Leu Asn Asp Phe
225 230 235 240

Leu Leu Asp Val Ser Lys Thr Tyr Phe Asp Asn Ile Val Ala Ile Asp
245 250 255

Ser Leu Leu Glu His Ile Met Ile Tyr Ala Lys Asn Leu Val Asn Ala
260 265 270

Asp Arg Cys Ala Leu Phe Gln Val Asp His Lys Asn Lys Glu Leu Tyr
275 280 285

Ser Asp Leu Phe Asp Ile Gly Glu Glu Lys Glu Gly Lys Pro Xaa Phe
290 295 300

Lys Lys Thr Lys Glu Ile Arg Phe Ser Ile Glu Lys Gly Ile Ala Gly
305 310 315 320

Gln Val Ala Arg Thr Gly Glu Val Leu Asn Ile Pro Asp Ala Tyr Ala
325 330 335

Asp Pro Arg Phe Asn Arg Glu Val Asp Leu Tyr Thr Gly Tyr Thr Thr
340 345 350

Arg Asn Ile Leu Cys Met Pro Ile Val Ser Arg Gly Ser Val Ile Gly
355 360 365

Val Val Gln Met Val Asn Lys Ile Ser Gly Ser Ala Phe Ser Lys Thr
370 375 380

Asp Glu Asn Asn Phe Lys Met Phe Ala Val Phe Cys Ala Leu Ala Leu
385 390 395 400

His Cys Ala Asn Met Tyr His Arg Ile Arg His Ser Glu Cys Ile Tyr
405 410 415

Arg Val Thr Met Glu Lys Leu Ser Tyr His Ser Ile Cys Thr Ser Glu
420 425 430

Glu Trp Gln Gly Leu Met Xaa Phe Xaa Leu Pro Xaa Arg Xaa Cys Xaa
435 440 445

Xaa Ile Glu Leu Phe His Phe Asp Ile Gly Pro Phe Glu Asn Met Trp
450 455 460

Pro Gly Ile Phe Val Tyr Met Xaa His Arg Ser Cys Gly Thr Ser Cys
465 470 475 480

Phe Glu Leu Glu Lys Leu Cys Arg Phe Ile Met Ser Val Lys Lys Asn
485 490 495

Tyr Arg Arg Val Pro Tyr His Asn Trp Lys His Ala Val Thr Val Ala
500 505 510

His Cys Met Tyr Ala Ile Leu Gln Asn Asn Xaa Xaa Leu Phe Thr Asp
515 520 525

Leu Glu Arg Lys Gly Leu Leu Ile Ala Cys Leu Cys His Asp Leu Asp
530 535 540

His Arg Gly Phe Ser Asn Ser Tyr Leu Gln Lys Phe Asp His Pro Leu
545 550 555 560

Xaa Ala Leu Tyr Ser Thr Ser Thr Met Glu Gln His His Phe Ser Gln
565 570 575

Thr Val Ser Ile Leu Gln Leu Glu Gly His Asn Ile Phe Ser Thr Leu
580 585 590

Ser Ser Ser Glu Tyr Glu Gln Val Leu Glu Ile Ile Arg Lys Ala Ile
595 600 605

Ile Ala Thr Asp Leu Ala Leu Tyr Phe Gly Asn Arg Lys Gln Leu Glu
610 615 620

Glu Met Tyr Gln Thr Gly Ser Leu Asn Leu Xaa Asn Gln Ser His Arg
625 630 635 640

Asp Arg Val Ile Gly Leu Met Met Thr Ala Cys Asp Leu Cys Ser Val
645 650 655

Thr Lys Xaa Trp Pro Val Thr Lys Leu Thr Ala Asn Asp Ile Tyr Ala
660 665 670

Glu Phe Trp Ala Glu Gly Asp Glu Met Lys Lys Leu Gly Ile Gln Pro
675 680 685

Ile Pro Met Met Asp Arg Asp Lys Xaa Asp Glu Val Pro Gln Gly Gln
690 695 700

Leu Gly Phe Tyr Asn Ala Val Ala Ile Pro Cys Tyr Thr Thr Leu Thr
705 710 715 720

Gln Ile Leu Pro Pro Thr Glu Pro Leu Leu Lys Ala Cys Arg Asp Asn
725 730 735

Leu Xaa Gln Trp Glu Lys Val Ile Arg Gly Glu Glu Thr Ala Xaa Trp
740 745 750

Ile Ser Xaa Pro Xaa Xaa Ala Xaa Xaa Xaa Xaa Xaa Xaa Glu Xaa
755 760 765

<210> 41
<211> 8
<212> PRT
<213> Artificial Sequence

<220>
<223> Description of Artificial Sequence: Synthetic sequence

<220>
<221> SITE
<222> (4)
<223> Xaa=Asn or Thr

<220>
<221> SITE
<222> (7)
<223> Xaa=Ser or Ala

<220>
<221> SITE
<222> (8)
<223> Xaa=Glu or Lys

<400> 41
Lys Arg Lys Xaa Asn Lys Xaa Xaa
  1               5

<210> 42
<211> 8
<212> PRT
<213> Artificial Sequence

<220>
<223> Description of Artificial Sequence: Synthetic
      sequence

<220>
<221> SITE
<222> (4)
<223> Xaa=Asn or Thr

<220>
<221> SITE
<222> (7)
<223> Xaa=Ser or Ala

<220>
<221> SITE
<222> (8)
<223> Xaa=Glu or Lys

<400> 42

Lys Arg Lys Xaa Asp Lys Xaa Xaa
1 5

<210> 43
<211> 11
<212> PRT
<213> Artificial Sequence

<220>
<223> Description of Artificial Sequence: Synthetic sequence

<220>
<221> SITE
<222> (11)
<223> Xaa=Lys or Arg

<400> 43

Leu Leu Leu Tyr Glu Leu Ser Ser Ile Ile Xaa
1 5 10

<210> 44
<211> 12
<212> PRT
<213> Artificial Sequence

<220>
<223> Description of Artificial Sequence: Synthetic sequence

<220>
<221> SITE
<222> (12)
<223> Xaa=Lys or Arg

<400> 44
Leu Leu Leu Tyr Glu Leu Asn Ser Ser Ile Ile Xaa
1 5 10

<210> 45
<211> 73
<212> PRT
<213> Homo sapiens

<400> 45
Met Glu Asp Gly Pro Ser Asn Asn Ala Ser Cys Phe Arg Arg Leu Thr
1 5 10 15

Glu Cys Phe Leu Ser Pro Ser Leu Thr Asp Glu Lys Val Lys Ala Tyr
20 25 30

Leu Ser Leu His Pro Gln Val Leu Asp Glu Phe Val Ser Glu Ser Val
35 40 45

Ser Ala Glu Thr Val Glu Lys Trp Leu Lys Arg Lys Asn Asn Lys Ser
50 55 60

Glu Asp Glu Ser Ala Pro Lys Glu Val
65 70

<210> 46
<211> 75
<212> PRT
<213> Homo sapiens

<400> 46
Met Glu Gly Ser Thr Ala Trp Ser Ser Glu Ala Glu Pro Gln Glu Gly
1 5 10 15

Arg Ser Gln Met Leu Gln Arg Ala Gly Leu Thr Asp Glu Lys Val Lys
20 25 30

Ala Tyr Leu Ser Leu His Pro Gln Val Leu Asp Glu Phe Val Ser Glu
35 40 45

Ser Val Ser Ala Glu Thr Val Glu Lys Trp Leu Lys Arg Lys Asn Asn
50 55 60

Lys Ser Glu Asp Glu Ser Ala Pro Lys Glu Val
65 70 75

<210> 47
<211> 300
<212> PRT
<213> Homo sapiens

<400> 47
Phe Leu Gly Glu Cys Asn Asn Ser Leu Cys Ile Phe Thr Pro Pro Gly
1 5 10 15

Ile Lys Glu Gly Lys Pro Arg Leu Ile Pro Ala Gly Pro Ile Thr Gln
20 25 30

Gly Thr Thr Val Ser Ala Tyr Val Ala Lys Ser Arg Lys Thr Leu Leu
35 40 45

Val Glu Asp Ile Leu Gly Asp Glu Arg Phe Pro Arg Gly Thr Gly Leu
50 55 60

Glu Ser Gly Thr Arg Ile Gln Ser Val Leu Cys Leu Pro Ile Val Thr
65 70 75 80

Ala Ile Gly Asp Leu Ile Gly Ile Leu Glu Leu Tyr Arg His Trp Gly
85 90 95

```
Lys Glu Ala Phe Cys Leu Ser His Gln Glu Val Ala Thr Ala Asn Leu
            100                 105                 110

Ala Trp Ala Ser Val Ala Ile His Gln Val Gln Val Cys Arg Gly Leu
        115                 120                 125

Ala Lys Gln Thr Glu Leu Asn Asp Phe Leu Leu Asp Val Ser Lys Thr
    130                 135                 140

Tyr Phe Asp Asn Ile Val Ala Ile Asp Ser Leu Leu Glu His Ile Met
145                 150                 155                 160

Ile Tyr Ala Lys Asn Leu Val Asn Ala Asp Arg Cys Ala Leu Phe Gln
                165                 170                 175

Val Asp His Lys Asn Lys Glu Leu Tyr Ser Asp Leu Phe Asp Ile Gly
            180                 185                 190

Glu Glu Lys Glu Gly Lys Pro Val Phe Lys Lys Thr Lys Glu Ile Arg
        195                 200                 205

Phe Ser Ile Glu Lys Gly Ile Ala Gly Gln Val Ala Arg Thr Gly Glu
    210                 215                 220

Val Leu Asn Ile Pro Asp Ala Tyr Ala Asp Pro Arg Phe Asn Arg Glu
225                 230                 235                 240

Val Asp Leu Tyr Thr Gly Tyr Thr Thr Arg Asn Ile Leu Cys Met Pro
                245                 250                 255

Ile Val Ser Arg Gly Ser Val Ile Gly Val Val Gln Met Val Asn Lys
            260                 265                 270

Ile Ser Gly Ser Ala Phe Ser Lys Thr Asp Glu Asn Asn Phe Lys Met
        275                 280                 285

Phe Ala Val Phe Cys Ala Leu Ala Leu His Cys Ala
    290                 295                 300
```